(19) Europäisches Patentamt
European Patent Office
Office européen des brevets

(11) EP 4 707 301 A1

(12) **EUROPEAN PATENT APPLICATION**
published in accordance with Art. 153(4) EPC

(43) Date of publication:
**11.03.2026 Bulletin 2026/11**

(21) Application number: **24800249.5**

(22) Date of filing: **03.05.2024**

(51) International Patent Classification (IPC):
***C07K 16/28** (2006.01)* ***A61P 37/00** (2006.01)*
***A61K 39/00** (2006.01)*

(52) Cooperative Patent Classification (CPC):
**A61K 39/00; A61P 37/00; C07K 16/28**

(86) International application number:
**PCT/KR2024/005975**

(87) International publication number:
**WO 2024/228568 (07.11.2024 Gazette 2024/45)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **03.05.2023 KR 20230057864**

(71) Applicant: **AJOU UNIVERSITY INDUSTRY-ACADEMIC COOPERATION FOUNDATION**
**Gyeonggi-do 16499 (KR)**

(72) Inventors:
- **KIM, Yong Sung**
  **Suwon-si, Gyeonggi-do 16517 (KR)**
- **KIM, Jun-Ho**
  **Hwaseong-si, Gyeonggi-do 18429 (KR)**

(74) Representative: **Mewburn Ellis LLP**
**Aurora Building**
**Counterslip**
**Bristol BS1 6BX (GB)**

Remarks:
The complete document including Reference Table(s) and the Sequence Listing(s) can be downloaded from the EPO website

(54) **IL-5RA×CD3E T CELL ENGAGING BISPECIFIC ANTIBODY AND USES THEREOF**

(57) The present invention relates to a T cell engaging bispecific antibody. More particularly, the present invention relates to: a bispecific antibody that simultaneously binds to human interleukin-5 (IL-5) receptor alpha subunit (IL-5Rα), which is a receptor of human IL-5, and CD3ε, which is a human T cell marker; a nucleic acid encoding same; a vector comprising the nucleic acid; a cell transformed with the vector; a method for producing the bispecific antibody or; a composition comprising same for the prevention or treatment of allergic diseases, inflammatory diseases, and/or diseases caused by an increase in eosinophils; and a composition comprising same for the diagnosis of allergic diseases, inflammatory diseases, and/or diseases caused by an increase in eosinophils.

【Fig. 16】

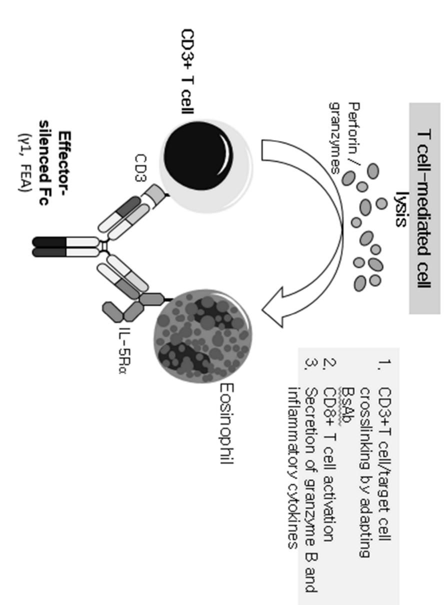


EP 4 707 301 A1

Processed by Luminess, 75001 PARIS (FR)

## Description

[Technical Field]

The present invention relates to a T cell engaging bispecific antibody. More specifically, the present invention relates to a humanized antibody that simultaneously binds to a human T cell marker (CD3) and human IL-5 receptor alpha subunit (IL-5Ra), which is a receptor for human interleukin-5 (IL-5), a nucleic acid encoding the same, a vector including the nucleic acid, cells transformed with the vector, a method of producing the antibody, a composition for preventing or treating allergic diseases, inflammatory diseases, and/or diseases caused by an increase in eosinophils containing the same, a composition for diagnosing the allergic diseases, inflammatory diseases, and/or diseases caused by an increase in eosinophils containing the same, and a composition for removing eosinophils by engaging T cells.

[Background Art]

Human interleukin 5 (hereinafter referred to as "IL-5") is a type of cytokine secreted by T-cells or mast cells. IL-5 in mice is known to act as a differentiation and proliferation factor for B-cells and eosinophils. It is known that the IL-receptor acts mainly as a differentiation and growth factor of eosinophils in the human body. The IL-5 receptor is composed of an $\alpha$ chain (hereinafter referred to as IL-5R$\alpha$) and a $\beta$ chain (hereinafter referred to as IL-5R$\beta$ or $\beta$c receptor)]. In addition, IL-5R$\alpha$ is involved in direct IL-5 binding, and the $\beta$c receptor itself does not exhibit IL-5 binding ability, but is responsible for intracellular signaling. In addition, the $\beta$c receptor is known to act as a common receptor with receptors such as interleukin-3 (hereinafter referred to as IL-3) and a granulocyte-macrophage colony-stimulating factor (hereinafter referred to as GM-CSF).

The IL-5 receptor is known to be expressed in eosinophils, basophils and mast cells in the human body. It is particularly overexpressed in eosinophils. IL-5 is essential for eosinophil maturation, proliferation and activation and can enhance basophil differentiation. IL-5 promotes tissue accumulation of eosinophils through the ability thereof to upregulate the expression of vestibular adhesion molecules upon migration of eosinophils to other tissues. IL-5 also prolongs eosinophil survival by blocking eosinophil apoptosis. The main cellular sources of IL-5 are Th2 cells (Type 2 helper T cells), ILC2 (type 2 innate lymphoid cells), mast cells and natural killer T cells.

Eosinophils are circulating granulocytes produced in the bone marrow and are a type of major cells recruited to sites of inflammatory responses. It is known that the number of eosinophils is increased in the blood of patients with allergic diseases such as chronic bronchial asthma. Eosinophils function to release toxic granule proteins, reactive oxygen species (ROS) cytokines, and lipid mediators. Therefore, an increase in eosinophils is known to be involved in the development of various inflammatory diseases including allergic diseases related to hypersensitivity reactions in lung tissue (Possa et al., 2013). The close association between eosinophils and IL-5 leads to the development of novel drugs that are capable of inhibiting the activity of eosinophils by directly acting on IL-5 and IL-5R$\alpha$ and treating allergic diseases such as chronic bronchial asthma.

Mepolizumab is an N-glycosylated humanized IgG1 antibody that binds to IL-5 and inhibits the binding to IL-5R$\alpha$. Mepolizumab has been tested in several diseases including severe asthma, atopic dermatitis and nasal polyposis. Mepolizumab was approved by the FDA in 2015 based on excellent results in asthma patients. The amount of eosinophils decreased in subjects treated with mepolizumab, but serum IL-5 levels were found to increase over time (Pouliquen et al., 2015; Tsukamoto et al., 2016). One group hypothesizes that most IL-5 detected during treatment is part of a complex bound to immunoglobulins (mostly mepolizumab) and prolongs the half-life of complexed IL-5. The biological significance and fate of this complex are unknown.

Reslizumab is a humanized IgG4 antibody that binds to IL-5 with high affinity to block the biological functions thereof. It was approved for use as an add-on maintenance therapy in patients with severe eosinophilic asthma in the United States and Europe. Like mepolizumab, reslizumab increased serum IL-5 levels after 1-month treatment of patients with hypereosinophilic syndrome (HES). However, it is still unknown whether or not this is due to free or complex IL-5. Culture medium-containing serum from reslizumab-treated patients has been shown to prolong eosinophil survival *in vitro,* and researchers have hypothesized that anti-IL-5 not only prolongs half-life, but actually improves IL-5 activity under certain conditions (Roufosse, 2018).

Benralizumab is an afucosylated humanized IgG1 antibody and has two mechanisms different from anti-IL-5 antibodies because it targets IL-5R$\alpha$. Benralizumab 1) inhibits cell growth by blocking the action of IL-5, and 2) induces antibody-dependent cellular cytotoxicity (ADCC) by natural killer cells and macrophages. In addition, the afucosylated Fc improves the affinity for Fc$\gamma$RIIIa expressed on the surface of natural killer (NK) cells and macrophages, resulting in an approximately 1,000-times stronger ADCC effect. This dual mechanism of action of benralizumab results in a much faster and higher level of depletion of eosinophils than that induced by other monoclonal antibodies targeting the IL-5 pathway, such as mepolizumab and reslizumab (Davila Gonzalez et al., 2019). Therefore, benralizumab is proposed as a therapeutic agent for not only eosinophilic asthma but also chronic obstructive pulmonary disease (COPD), eosinophilic syndrome, and chronic rhinitis.

According to research reported to date, targeting IL-5Rα rather than IL-5 has the advantage of directly lowering the number of eosinophils without increasing the half-life of IL-5. However, no therapeutic anti-IL-5Rα antibody other than benralizumab has yet been approved, and benralizumab did not show significant effects in some patients. In addition, the continuous and repeated administration of one therapeutic agent may disadvantageously cause resistance to the therapeutic agent, such as the generation of anti-drug antibodies (ADA). An anti-IL-5Rα antibody, 5R65.7 that targets an epitope in the membrane-proximal domain of IL-5Rα and has higher affinity has been developed (Korean Patent Application No. 10-2020-0117668). However, the result of evaluation of ADCC efficacy using eosinophils and PBMCs from patient blood samples showed the cytotoxicity was limited to only about 30% of total eosinophils. Furthermore, previous studies have shown that eosinophil depletion does not increase the risk of parasitic infection or reduced vaccine response (Manetz et al., 2021). Considering these findings, there remains a high demand for pharmaceuticals that can induce more potent eosinophil reduction.

The mechanism of action of benralizumab and 5R65.7 is to selectively induce an immune response against the target, which triggers the attack and subsequent destruction of the target cells by natural killer (NK) cells which are immune effector cells. Meanwhile, regarding T cells, although there are several subsets of T cells having distinct functions, cytotoxic T lymphocytes (CTLs) constitute the most potent effector cells of the immune system, but cannot be activated by effector mechanisms mediated by the Fc domain of conventional therapeutic antibodies. T cells may be distinguished from other lymphocytes, such as NK cells and B cells, based on the presence of the T cell receptor (TCR) on the cell surface thereof. The T cell receptor (TCR) is bound to major histocompatibility complex (MHC) molecules and antigen recognition is mediated by two distinct protein chains. In 95% of T cells, the TCR consists of an alpha (α) chain and a beta (β) chain. When the TCR engages an antigenic peptide and MHC (the peptide/MHC complex), the T lymphocyte is activated through a series of biochemical events mediated by associated enzymes, co-receptors, specialized adaptor molecules, and activated or released transcription factors.

The CD3 receptor complex is a protein complex composed of four chains. In mammals, the complex includes a CD3γ (gamma) chain, a CD3δ (delta) chain, and two CD3ε (epsilon) chains. These chains are linked to the T cell receptor (TCR) and the so-called ζ (zeta) chain to form the T cell receptor-CD3 complex and generate an activation signal within the T lymphocyte. The CD3γ (gamma), CD3δ (delta), and CD3ε (epsilon) chains are highly related cell surface proteins of the immunoglobulin superfamily that contain a single extracellular immunoglobulin domain. The intracellular tail of the CD3 molecule contains a single conserved motif, known as the immunoreceptor tyrosine-based motif for short, or ITAM, which is essential for the signaling capacity of the TCR. The CD3ε molecule is a polypeptide encoded by the human CD3E gene on chromosome 11. The most preferred epitope of CD3ε is incorporated in amino acid residues 1 to 27 of the human CD3 epsilon extracellular domain.

Therefore, in this regard, the present invention provides a T cell-engaging bispecific antibody that induces a reduction of target cells *in vivo* by recruiting T cells to the target cells, thereby forming a cytolytic synapse between the T cells and the target cells, and enabling the T cells to kill the target cells without requiring activation mediated by major histocompatibility complex (MHC) proteins.

Research on bispecific antibodies that induce cytotoxicity by targeting only cells expressing cancer-specific biomarkers or overexpressing specific biomarkers among various cell types has been conducted using T cells, the most potent immune system in the body. Several T cell-engaging bispecific antibody formats have been developed, primarily in the field of cancer immunotherapy, and the suitability thereof for T cell-mediated cancer immunotherapy has been studied. Among these, BiTE (bispecific T cell engager) molecules are very well characterized and have already shown some promising results in clinical settings (Nagorsen, D. and P. A. Baeuerle (2011). "Immunomodulatory therapy of cancer with T cell-engaging BiTE antibody blinatumomab." Exp Cell Res 317 (9) : 1255-1260.). BiTEs are tandem scFv molecules, consisting of two scFv molecules fused together by a flexible linker. A representative example is blinatumomab, a CD19×CD3 BiTE used for the treatment of refractory acute lymphoblastic leukemia. In clinical trials, blinatumomab exhibits higher cytotoxicity at much lower concentrations compared to conventional anti-CD19 monoclonal antibodies, leading to FDA approval in 2014. Additional bispecific formats that have been evaluated for T cell engagement include diabodies (Diabody, Holliger, P., et al. (1996). "Specific killing of lymphoma cells by cytotoxic T-cells mediated by a bispecific diabody." Protein Engineering, Design and Selection 9(3): 299-305.) and derivatives thereof, such as tandem diabodies (Tandem diabody, Kipriyanov, S. M., et al. (1999). "Bispecific tandem diabody for tumor therapy with improved antigen binding and pharmacokinetics." J Mol Biol 293(1): 41-56.). There is a more recent development of "DART" (dual affinity retargeting) molecules, which are based on the diabody format but feature a C-terminal disulfide (S-S) bond for additional stabilization (Moore, P. A., et al. (2011). "Application of dual affinity retargeting molecules to achieve optimal redirected T-cell killing of B-cell lymphoma." Blood 117(17): 4542-4551.). The so-called triomabs, which are full hybrid mouse/rat IgG molecules and are currently being evaluated in clinical trials, represent a larger format (Seimetz, D., et al. (2010). "Development and approval of the trifunctional antibody catumaxomab (anti-EpCAM×anti-CD3) as a targeted cancer immunotherapy." Cancer Treatment Reviews 36(6): 458-467.). In addition, there are development and clinical trials of several bispecific antibodies that target cancer cells, such as CD20, BCMA, CEA, mesothelin, and ROR1, or target biomarkers overexpressed in normal cells, and CD3 simultaneously, to induce a mechanism for eliminating cancer cells using T cells (Qi, J., et al. (2018). "Potent and selective antitumor activity of a T cell-engaging bispecific antibody targeting a membrane-proximal epitope of ROR1." Proc Natl Acad Sci U S A 115(24): E5467-e5476).

However, known bispecific antibodies, such as BiTEs, diabodies, and DARTs, have major drawbacks in the efficacy, toxicity, and availability thereof. Molecules that do not have an Fc domain having less than 70 kDa such as BiTEs, diabodies, and DARTs, may efficiently cross-link effectors with target cells, but have very short serum half-lives, requiring continuous infusion into patients. On the other hand, T-cell-engaging bispecific antibodies have Fc with larger molecular weights (>100 kDa), which make cross-linking effectors and target cells more difficult than in bispecific antibodies lacking the Fc domain, thus resulting in reduced T-cell-mediated cytotoxicity induction. However, the T-cell-engaging bispecific antibodies have the advantages of simpler protein production and purification processes and a longer half-life than bispecific antibodies lacking Fc. Meanwhile, there is currently no progress in developing bispecific antibodies that remove, from the body, cells other than cancer cells that are involved in immune responses and cause inflammatory and allergic reactions.

Under this technical background, the present inventors tried to develop a T cell-engaging bispecific antibody that simultaneously targets IL-5Rα, overexpressed in eosinophils of patients with eosinophilic asthma, and the ε domain of CD3, a T cell marker, to induce T cell-mediated eosinophilia reduction. In the present invention, eight formats of IL-5Rα×CD3ε T cell-engaging bispecific antibodies were constructed using the 5R65.7 antibody, which targets an epitope in the membrane-proximal domain of IL-5Rα having higher affinity than the conventional benralizumab antibody, and the humanized SP34 antibody, which is a mouse antibody targeting the ε domain of CD3, and were then screened to determine which format exhibited the highest T cell-mediated cytotoxicity induction capacity.

Meanwhile, heterodimeric Fc technology was used to produce T cell-engaging bispecific antibodies containing Fc. Heterodimeric Fc technology involves engineering the interaction surface between the last domains of the constant regions of naturally occurring antibodies (IgG, IgM, IgA, IgD, and IgE) to favor heterodimerization, and disfavor or repel homodimerization, thereby forming a heterodimer-like heavy chain constant region. More specifically, mutations are induced in the CH3 domains of two different antibody heavy chains through genetic manipulation, resulting in two heavy chains that are structurally very similar to naturally occurring antibodies and possess minimal sequence variation, yet form heterodimers. Among them, the EW-RVT heterodimeric Fc mutant used in the present invention is a mutant that promotes the formation of a heterodimer by forming a selective electrostatic bond at the CH3 domain interaction surface (K360ECH3A-Q347RCH3B) and forming a complementary hydrophobic bond instead of the conventional electrostatic bond (K409WCH3A-D399VCH3B/F405TCH3B) through a new mutation strategy by analyzing the interaction of the existing mutants in terms of sequence and structure (US Patent No. 9,951,145; Republic of Korea Patent No. 1,522,954; Choi HJ et al. Molecular Cancer Therapeutics, 2013;12(12): 2748-2759). It was found that the IL-5Rα×CD3ε T cell-engaging bispecific antibody thus manufactured exhibited superior T cell activation ability and T cell-mediated cytotoxicity in the same format, compared to the negative control and the benralizumab control, which is a T cell-engaging bispecific antibody manufactured using benralizumab.

[Disclosure]

Therefore, the present invention has been made in view of the above problems, and it is one object of the present invention to provide a bispecific antibody that targets IL-5Rα to bind to IL-5Rα-expressing eosinophils and basophils and at the same time, targets CD3 to bind to CD3-expressing T cells, thereby inducing T cell-mediated cytotoxicity.

It is another object of the present invention to provide a bispecific antibody using a humanized antibody fragment that strongly induces T cell-mediated cytotoxicity against IL-5Rα-expressing eosinophils and basophils, using a 5R65.7 antibody fragment that binds to IL-5Rα with high affinity and has a membrane-proximal domain as an epitope.

It is another object of the present invention to provide a bispecific antibody constructed using heterodimeric Fc technology, wherein the bispecific antibody is generated from an anti-IL-5Rα human antibody (5R65.7) and an anti-CD3ε mouse antibody (SP34), by preparing a partially humanized SP34 in which the amino acid sequences of the variable regions of the heavy and light chains of SP34 are replaced with the corresponding CDR amino acid sequences of SP34 in the most homologous human germline antibody sequences according to prior literature (Korean Patent No. 10-1973060) and a nucleic acid encoding the bispecific antibody.

It is another object of the present invention to provide a vector including the nucleic acid, cells transformed with the vector, and a method of producing the same.

It is another object of the present invention to provide a pharmaceutical composition containing the bispecific antibody for preventing or treating allergic diseases, inflammatory diseases, and/or diseases caused by an increase in eosinophils.

It is another object of the present invention to provide a composition for removing eosinophils containing the bispecific antibody or an antigen-binding fragment thereof.

It is another object of the present invention to provide a composition containing the bispecific antibody for diagnosing allergic diseases, inflammatory diseases, and/or diseases caused by an increase in eosinophils.

In accordance with one aspect of the present invention, the above and other objects can be accomplished by the provision of eight formats of T cell-engaging bispecific antibodies using an antibody or antigen-binding fragment thereof binding to human IL-5Rα recognizing, as an epitope, at least one amino acid residue selected from the group consisting of amino acid residues 221 to 322 corresponding to domain 3 (D3) of a sequence of human IL-5 receptor alpha subunit (IL-5Rα).

In accordance with another aspect of the present invention, provided are eight formats of T cell engaging bispecific antibodies using an SP34 antibody or antigen binding fragment thereof that binds to human CD3ε, recognizing, as an epitope, at least one amino acid residue selected from the group consisting of amino acid residues 23 to 126 corresponding to the extracellular domain of the sequence of human CD3ε as previously disclosed.

In accordance with another aspect of the present invention, provided is a bispecific antibody or antigen-binding fragment thereof that binds to interleukin (IL)-5 receptor α (IL-5Rα) and CD3α, including an antibody or antigen-binding fragment thereof that binds to IL-5Rα and an antibody or antigen-binding fragment thereof that binds to CD3α.

In accordance with another aspect of the present invention, provided is a bispecific antibody constructed using a previously disclosed CD3-activating agonist antibody.

In accordance with another aspect of the present invention, provided is a bispecific antibody including a previously disclosed CD3-activating agonist antibody and an IL-5Rα-targeting antibody 5R65.7 paired therewith.

In accordance with another aspect of the present invention, provided is a method of inducing T cell-mediated cytotoxicity against IL-5Rα-expressing cells.

In accordance with another aspect of the present invention, provided is a method of inducing T cell-mediated cytotoxicity using a bispecific antibody including a CD3-activating agonist antibody and an antibody targeting IL-5Rα paired therewith.

In accordance with another aspect of the present invention, provided is a method of treating eosinophilic asthma using a bispecific antibody including a CD3-activating agonist antibody and an antibody targeting IL-5Rα paired therewith.

In accordance with another aspect of the present invention, provided is a molecule in which each domain is linked to another domain via a peptide linker.

In accordance with another aspect of the present invention, provided are a nucleic acid encoding the bispecific antibody or antigen-binding fragment thereof and an expression vector containing the nucleic acid.

In accordance with another aspect of the present invention, provided is a cell transformed with the expression vector.

In accordance with another aspect of the present invention, provided is a method of producing a bispecific antibody binding to both human IL-5Rα and human CD3ε.

In accordance with another aspect of the present invention, provided is a conjugate including the bispecific antibody and a drug.

In accordance with another aspect of the present invention, provided is a composition for preventing or treating allergic diseases, inflammatory diseases, and/or diseases caused by an increase in eosinophils containing the bispecific antibody or antigen-binding fragment thereof. In accordance with another aspect of the present invention, provided is a method of preventing or treating allergic diseases, inflammatory diseases, and/or diseases caused by an increase in eosinophils including administering the bispecific antibody or antigen-binding fragment thereof to a patient. In accordance with another aspect of the present invention, provided is the use of the bispecific antibody or antigen-binding fragment thereof for the prevention or treatment of allergic diseases, inflammatory diseases, and/or diseases caused by an increase in eosinophils.

In accordance with another aspect of the present invention, provided is a composition for removing eosinophils containing the bispecific antibody or antigen-binding fragment thereof. In accordance with another aspect of the present invention, provided is a method of removing eosinophils including administering the bispecific antibody or antigen-binding fragment thereof to a patient. In accordance with another aspect of the present invention, provided is the use of the bispecific antibody or antigen-binding fragment thereof for the preparation of the composition for removing eosinophils.

In accordance with another aspect of the present invention, provided is a composition for diagnosing allergic diseases, inflammatory diseases, and/or diseases caused by an increase in eosinophils containing the bispecific antibody or antigen-binding fragment thereof.

[Description of Drawings]

FIG. 1 is a schematic diagram illustrating eight different formats of IL-5Rα×CD3ε T cell-engaging bispecific antibodies and the nomenclature of each format, predicted molecular weights, and the expression level of each bispecific antibody produced using the method described in Example 2. Hereinafter, "$C_H3$ (EW)" in the drawing will be referred to as "CH3A (EW)" and "$C_H3$ (RVT)" will be referred to as "CH3B (RVT)".

FIG. 1A is a schematic diagram illustrating recombinant expression vectors for expressing IL-5Rα×CD3ε-Diabody-Fc. L represents a peptide linker with a GGGSGGGG sequence consisting of glycine and serine, and *H represents a hinge sequence consisting of glycine, cysteine, and proline, which is different from the typical hinge sequence of the IgG1 subclass.

FIG. 2B is a schematic diagram illustrating recombinant expression vectors for expressing IL-5Rα×CD3ε-scFv(L)/scFv-Fc. L represents a peptide linker consisting of the amino acids GGGGSGGGGSGGGGS (hereinafter referred to as $(G_4S)_3$) composed of glycine and serine, and H represents a hinge sequence of a typical IgG1 subclass.

FIG. 2C is a schematic diagram illustrating recombinant expression vectors for expressing IL-5Rα×CD3ε-scFv(H)/scFv-Fc. L represents a peptide linker consisting of the amino acids $(G_4S)_3$ composed of glycine and serine, and H represents a hinge sequence of a typical IgG1 subclass.

FIG. 2D is a schematic diagram illustrating recombinant expression vectors for expressing IL-5Rα×CD3ε-BiTE Fc. The bolded L represents a $(G_4S)_3$ peptide linker composed of the amino acids, glycine and serine, and the thin L represents a GGGGS (hereafter referred to as "$G_4S$") peptide linker composed of the amino acids, glycine and serine. H represents the hinge sequence of a typical IgG1 subclass.

FIG. 2E is a schematic diagram illustrating recombinant expression vectors for expressing IL-5Rα×CD3ε-scFv/Fab-Fc. L represents a $(G_4S)_3$ peptide linker composed of the amino acids, glycine and serine. H represents the hinge sequence of a typical IgG1 subclass.

FIG. 2F is a schematic diagram illustrating recombinant expression vectors for expressing IL-5Rα×CD3ε-IgG analogs. H represents the hinge sequence of a typical IgG1 subclass. The SP34 heavy and light chains, and the 5R65.7 heavy and light chains, include complementary mutations in the CH1 and CL regions to prevent cross-linking.

FIG. 2G is a schematic diagram illustrating recombinant expression vectors for expressing IL-5Rα×CD3ε-BiTE(L)/scFv-Fc. Bold L represents a $(G_4S)_3$ peptide linker composed of glycine and serine amino acids, and thin L represents a $G_4S$ peptide linker composed of glycine and serine amino acids. H represents the hinge sequence of a typical IgG1 subclass.

FIG. 2H is a schematic diagram illustrating recombinant expression vectors for expressing IL-5Rα×CD3ε-BiTE(H)/scFv-Fc. The bolded L represents a $(G_4S)_3$ peptide linker composed of the amino acids, glycine and serine, and the thin L represents a $G_4S$ peptide linker composed of the amino acids, glycine and serine. H represents the hinge of the GCPPCP sequence.

FIG. 2I is a schematic illustrating the recombinant expression vector for expressing HER2×CD3ε-IgG analogs. The SP34 heavy and light chains, and the Trastuzumab heavy and light chains, include complementary mutations in the CH1 and CL regions to prevent cross-linking.

FIG. 3A shows the results of transient expression and purification of eight formats of IL-5Rα×CD3ε T cell-engaging bispecific antibodies in HEK293F cells by co-transfection, separation of 3 μg of protein on 12% SDS-PAGE under non-reducing and reducing conditions, and analysis of size and combinations using Coomassie Blue staining. "Non-reducing" represents non-reducing conditions, "reducing" represents reducing conditions, and "M" represents a protein marker. The red areas in results of the non-reducing conditions represent the bands corresponding to the sizes of the IL-5Rα×CD3ε T cell-engaging bispecific antibodies in Lanes 1 to 8.

FIG. 3B shows the results of size exclusion chromatography (SEC) analysis of the specified eight formats of IL-5Rα×CD3ε T cell-engaging bispecific antibodies. In SEC analysis, the running buffer was 1× PBS (pH 7.4, 12 mM phosphate, 137 mM NaCl, 2.7 mM KCl), the flow rate was 0.75 ml/min, and the protein input was 20 μg (20 μl injection at 1 mg/ml).

FIG. 4 shows results of treatment of four formats of IL-5Rα×CD3ε T cell-engaging bispecific antibodies (diabody-Fc, scFv(L)/scFv-Fc, IgG analog, BiTE(L)/scFv-Fc) selected after evaluation of physical properties in K562/IL-5Rα cell line expressing human IL-5Rα, activated PBMC expressing human CD3ε, and wild-type K562 (WT K562) cell line expressing neither IL-5Rα nor CD3ε, at concentrations of 50 nM (for K562/IL-5Rα, WT K562) and 5 nM (for activated PBMC), followed by fluorescence labeling using Alexa Flour 647 AFFINIPURE Goat Anti-human IgG, Fcγ fragment specific 2nd antibody (Jacksonimmunology, 109-605-098) and measurement of fluorescence intensity using a FACSCalibur.

FIG. 5A shows binding isotherms obtained by analyzing the affinity of four formats (diabody-Fc, scFv(L)/scFv-Fc, IgG analog, and BiTE(L)/scFv-Fc) of IL-5Rα×CD3ε T cell-engaging bispecific antibodies selected after physical property evaluation.

FIG. 5B shows binding isotherms obtained by analyzing the simultaneous binding of four formats (diabody-Fc, scFv(L)/scFv-Fc, IgG analog, and BiTE(L)/scFv-Fc) of IL-5Rα×CD3ε T cell-engaging bispecific antibodies selected after physical property evaluation.

FIG. 6A shows the results of evaluation of the T cell-mediated cytotoxicity-inducing ability of four different formats of IL-5Rα×CD3ε T cell-engaging bispecific antibodies using K562/IL-5Rα, which is a human IL-5Rα-expressing cell line and activated T cells at an effector cell-to-target cell ratio of 5:1 and 24-hour incubation.

FIG. 6B shows the results of evaluating the T cell-mediated cytotoxicity-inducing ability of four different formats of IL-5Rα×CD3ε T cell-engaging bispecific antibodies using wild-type K562 cells and activated T cells.

FIG. 7 shows the result of ELISA of IFN-γ present in the culture medium after the T cell-mediated cytotoxicity-inducing ability test.

FIG. 8 shows the result of FACSCalibur analysis of IL-5Rα expression in eosinophils (Siglec-8-expressing cells) and neutrophils (Siglec-8-non-expressing cells) isolated from the peripheral blood of healthy controls.

FIG. 9 shows the results of FACSCalibur analysis of IL-5Rα expression in eosinophils (Siglec-8-expressing cells) and neutrophils (Siglec-8-non-expressing cells) in the granulocyte layer (containing eosinophils and neutrophils) isolated from the peripheral blood of patients with severe eosinophilic asthma (SEA).

FIG. 10 shows the percentage of eosinophils in the granulocyte layer (containing eosinophils and neutrophils) isolated from the peripheral blood of healthy controls and patients with severe asthma.

FIG. 11A is a schematic diagram illustrating recombinant expression vectors for expressing IL-5Rα(ben) × CD3ε-scFv(L)/scFv-Fc. L represents a peptide linker consisting of the amino acid sequence GGGGSGGGGSGGGGS (hereinafter referred to as "$(G_4S)_3$") composed of glycine and serine, and H represents the hinge sequence of a typical IgG1 subclass.

FIG. 11B is a schematic diagram illustrating recombinant expression vectors for expressing IL-5Rα(5R65) × CD3ε-scFv(L)/scFv-Fc. L represents a peptide linker consisting of the amino acids GGGGSGGGGSGGGGS (hereinafter referred to as "$(G_4S)_3$)" composed of glycine and serine, and H represents a hinge sequence of a typical IgG1 subclass.

FIG. 11C is a schematic diagram illustrating recombinant expression vectors for expressing HER2×CD3ε-scFv(L)/scFv-Fc. L represents a peptide linker consisting of the amino acids GGGGSGGGGSGGGGS (hereinafter referred to as "$(G_4S)_3$)" composed of glycine and serine, and H represents a hinge sequence of a typical IgG1 subclass.

FIG. 12A is a schematic diagram illustrating three control T cell-engaging bispecific antibodies in the IL-5Rα×CD3ε-scFv(L)/scFv-Fc format, the nomenclature of each format, the predicted molecular weight, and the expression level of each bispecific antibody produced by the method described in Example 2.

FIG. 12B shows the results of transient expression and purification of three control T cell-engaging bispecific antibodies in the IL-5Rα×CD3ε-scFv(L)/scFv-Fc format in HEK293F cells by co-transfection, separation of 3 μg of protein on 12% SDS-PAGE under non-reducing and reducing conditions, and analysis of size and combinations using Coomasie Blue staining. "Non-reducing" represents non-reducing conditions, "reducing" represents reducing conditions, and "M" represents a protein marker.

FIG. 12C shows the results of size exclusion chromatography (SEC) analysis of three control T cell-engaging bispecific antibodies in the IL-5Rα×CD3ε-scFv(L)/scFv-Fc format. In SEC analysis, the running buffer was 1×PBS (pH 7.4, 12 mM phosphate, 137 mM NaCl, 2.7 mM KCl), the flow rate was 0.75 ml/min, and the protein input was 20 μg (20 μl injection at 1 mg/ml).

FIG. 13 shows results of treatment of K562/IL-5Rα cell line expressing human IL-5Rα, activated PBMC expressing human CD3ε, and wild-type K562 (WT K562) cell line expressing neither IL-5Rα nor CD3ε with IL-5Rα×CD3ε-scFv(L)/scFv-Fc and three control T cell-engaging bispecific antibodies at concentrations of 50 nM (for K562/IL-5Rα, WT K562) and 5 nM (for activated PBMC) , followed by fluorescence labeling using Alexa Flour 647 AFFINIPURE Goat Anti-human IgG, Fcγ fragment specific 2nd antibody (Jacksonimmunology, 109-605-098) and measurement of fluorescence intensity using a FACSCalibur.

FIG. 14A shows the results of evaluation of the T cell-mediated cytotoxicity-inducing ability of IL-5Rα×CD3ε-scFv(L)/scFv-Fc and three control T cell-engaging bispecific antibodies using K562/IL-5Rα, which is a human IL-5Rα-expressing cell line and activated T cells at an effector cell-to-target cell ratio of 5:1 and 24-hour incubation.

FIG. 14B shows the results of evaluation of the T cell-mediated cytotoxicity-inducing ability of IL-5Rα×CD3ε-scFv(L)/scFv-Fc and three control T cell-engaging bispecific antibodies using K562/IL-5Rα, which is a human IL-5Rα-expressing cell line and activated T cells at an effector cell-to-target cell ratio of 10:1 and 24-hour incubation.

FIG. 14C shows the results of evaluation of the T cell-mediated cytotoxicity-inducing ability of IL-5Rα×CD3ε-scFv(L)/scFv-Fc and three control T cell-engaging bispecific antibodies using K562/IL-5Rα, which is a human IL-5Rα-expressing cell line and activated T cells at an effector cell-to-target cell ratio of 10:1 and 12-hour incubation.

FIG. 15 shows the results of evaluation of the T cell-mediated cytotoxicity-inducing ability of IL-5Rα×CD3ε-scFv(L)/scFv-Fc format of T cell-engaging bispecific antibodies and three control T cell-engaging bispecific antibodies using eosinophils and autologous activated T cells isolated from blood samples of five healthy donors, at an effector cell-to-target cell ratio of 5:1 and under 24-hour incubation conditions.

FIG. 16 illustrates the use of the IL-5Rα×CD3ε bispecific antibodies according to the present invention.

[Best Mode]

Unless defined otherwise, all technical and scientific terms used herein have the same meanings as appreciated by those skilled in the field to which the present invention pertains. In general, the nomenclature used herein is well-known in the art and is ordinarily used.

In an embodiment of the present invention, a K562/IL-5Rα cell line stably expressing IL-5Rα was constructed using a lentiviral system and the biological activity of the humanized anti-IL-5Rα antibody was evaluated.

To further increase eosinophil reduction, T cells, which are the most potent effector cells in the body, were used, T cell-engaging bispecific antibodies were produced and eight IL-5Rα×CD3ε T cell-engaging bispecific antibodies were provided.

The T cell-mediated cytotoxicity of the eight IL-5Rα×CD3ε T cell-engaging bispecific antibodies was evaluated using the K562/IL-5Rα cell line and eosinophils derived from healthy donors and asthma patients.

Based on this, the present inventors provide eight IL-5Rα×CD3ε T cell-engaging bispecific antibodies that induce eosinophil reduction *in vivo* using T cells, the most potent effector cells in the body.

Accordingly, the present invention is directed to a bispecific antibody that recognizes, as an epitope, at least one amino acid residue selected from the group consisting of amino acid residues 221 to 322 corresponding to domain 3 (D3) of the human IL-5 receptor alpha subunit (IL-5Rα) sequence and simultaneously recognizes, as an epitope, at least one amino acid residue selected from the group consisting of amino acid residues 23 to 126 corresponding to the extracellular domain of the human CD3ε sequence.

The present invention relates to a bispecific antibody or antigen-binding fragment thereof that binds to interleukin (IL)-5 receptor α (IL-5Rα) and CD3α, including an antibody or antigen-binding fragment thereof that binds to IL-5Rα and an antibody or antigen-binding fragment thereof that binds to CD3α.

In an embodiment of the present invention, the bispecific antibody competitively binds to human interleukin-5 (IL-5) and IL-5Rα, and can simultaneously bind to an epitope including at least one amino acid residue selected from the group consisting of amino acid residues 221 to 322 corresponding to domain 3 (D3) of the human IL-5 receptor alpha subunit (IL-5Rα) and an epitope including at least one amino acid residue selected from the group consisting of amino acid residues 23 to 126 corresponding to the extracellular domain of human CD3ε.

As used herein, the term "patient" refers to any single animal, preferably a mammal (including non-human animals such as dogs, cats, horses, rabbits, zoo animals, cows, pigs, sheep, and non-human primates) in need of treatment. Most preferably, the patient herein is a human. In one embodiment, the term "subject" in need of diagnosis or treatment is a prokaryotic or eukaryotic cell, tissue culture, tissue, or animal, such as a mammal, including a human.

As used herein, the term "recombinant" refers to a polypeptide or polynucleotide that may be produced by combining polynucleotides or polypeptides in a configuration that does not occur naturally and does not normally occur together. The term may refer to a polypeptide produced through a biological host selected from a mammal expression system, an insect cell expression system, a yeast expression system, and a bacteria expression system.

As used herein, the term "epitope" refers to a protein determinant to which an antibody can specifically bind. Epitopes usually consist of a group of chemically active surface molecules, such as amino acid or sugar side chains, and generally have not only specific three-dimensional structural characteristics but also specific charge characteristics. Three-dimensional epitopes are distinguished from non-three-dimensional epitopes in that a bond to the former is broken in the presence of a denatured solvent, while a bond to the latter is not broken.

The eight IL-5Rα×CD3ε T cell-engaging bispecific antibodies described herein have T cell-mediated cytotoxicity and thus mediate the death of cells expressing IL-5Rα.

The antibody according to the present invention provides compositions and methods for treating diseases associated with IL-5 activity, such as allergic diseases. The composition contains a bispecific antibody recognizing an epitope on the extracellular domain of the human IL-5 receptor (IL-5Ra). The antibody has the ability to inhibit the biological activity of IL-5 and induces the death of cells (eosinophils, etc.) expressing IL-5Rα. Therefore, the present invention may be used for diagnosis and treatment of allergic diseases such as chronic bronchial asthma and atopic dermatitis.

The present invention relates to a bispecific antibody that simultaneously binds to human IL-5 receptor alpha subunit (IL-5Rα) and human CD3ε, produced by co-transfection with a nucleic acid encoding at least two sequences from SEQ ID NO: 33 to SEQ ID NO: 51.

The antibody or antigen-binding fragment thereof that binds to IL-5Rα includes a heavy chain CDR1 of SEQ ID NO: 1, a heavy chain CDR2 of SEQ ID NO: 2, a heavy chain CDR3 of SEQ ID NO: 3, and a light chain CDR1 of SEQ ID NO: 4; a light chain CDR2 of SEQ ID NO: 5; and a light chain CDR3 of SEQ ID NO: 6, and

the antibody or antigen-binding fragment thereof that binds to CD3α includes a heavy chain CDR1 of SEQ ID NO: 7, a heavy chain CDR2 of SEQ ID NO: 8, a heavy chain CDR3 of SEQ ID NO: 9, and a light chain CDR1 of SEQ ID NO: 10; a light chain CDR2 of SEQ ID NO: 11; and a light chain CDR3 of SEQ ID NO: 12.

As used herein, the term "IL-5Rα×CD3ε T cell-engaging bispecific antibody" refers to an antibody that specifically binds simultaneously to human IL-5Rα and CD3ε. Specifically, the bispecific antibody is preferably an antibody that does not exist naturally and is produced by genetic engineering or any other method. The term "bispecific antibody" may be used interchangeably with "bitargeting antibody" or "bispecific antibody protein". The bispecific antibody refers to a molecule whose antigen-binding sites are linked directly or via a linker, or which can form a heterodimer through electrostatic interactions. The scope of the present invention includes all formats of bispecific antibodies that may be constructed, including the complementarity-determining regions (CDRs) of the anti-IL-5Rα antibody 5R65.7 and the anti-CD3ε antibody SP34, in addition to the eight formats mentioned above.

The bispecific antibodies of the present invention are structures including proteins including variable and constant domains of two to four antibodies, each linked by a disulfide (disulfide, S-S) bond.

As used herein, the term "heavy chain" encompasses both a full-length heavy chain, which includes a variable domain (VH) containing an amino acid sequence having a sufficient variable region sequence for imparting specificity to an antigen and three constant domains (CH1, CH2 and CH3), and a fragment thereof. As used herein, the term "light chain" encompasses both a full-length light chain, which includes a variable domain (VL) containing an amino acid sequence having a sufficient variable region sequence for imparting specificity to an antigen and a constant domain (CL), and a fragment thereof.

The antibody or antigen-binding fragment thereof that binds to IL-5Rα may include a heavy chain variable region of SEQ ID NO: 20 and a light chain variable region of SEQ ID NO: 21, a heavy chain variable region of SEQ ID NO: 22 and a light chain variable region of SEQ ID NO: 23, or a heavy chain variable region of SEQ ID NO: 24 and a light chain variable region of SEQ ID NO: 21, and

the antibody or antigen-binding fragment thereof that binds to CD3α may include a heavy chain variable region of SEQ ID NO: 25 and a light chain variable region of SEQ ID NO: 26.

| SEQ ID NO: | Designation | Amino acid sequence |
|---|---|---|
| 20 | 5R65.7 VH | QVQLVQSGAEVKKPGSSVKVSCKASGYTFTSYWINWVRQAPGQGLEWIGHIYPNKNENYYNHKFKDKATLTVDKSTNTAYMELSSLRSEDTAVYYCTREFYGRQYYQAMDYWGQGTTLTVSS |
| 21 | 5R65.7 VL | DIQMTQSPSTLSASVGDRVTITCKASQNVGTAVAWYQQKPGKAPKLLIYWASTRHTGVPDRFSGSGSGTDFTLTISSLQPDDFATYYCQQFGRYPYTFGSGTKVEVK |
| 22 | Benralizumab VH | EVQLVQSGAEVKKPGASVKVSCKASGYTFTSYVIHWVRQRPGQGLAWMGYINPYNDGTKYNERFKGKVTITSDRSTSTVYMELSSLRSEDTAVYLCGREGIRYYGLLGDYWGQGTLVTVSS |
| 23 | Benralizumab VL | DIQMTQSPSSLSASVGDRVTITCGTSEDIINYLNWYQQKPGKAPKLLIYHTSRLQSGVPSRFSGSGSGTDFTLTISSLQPEDFATYYCQQGYTLPYTFGQGTKVEIK |
| 24 | 5R65 VH | QVQLVQSGAEVKKPGSSVKVSCKASGYTFTSYWINWVRQAPGQGLEWIGHIYPNKNENYYNHKFKDKATLTVDKSTNTAYMELSSLRSEDTAVYYCTRDYYGRSYYYAMDYWGQGTTLTVSS |
| 25 | SP34 VH | EVQLVESGGGLVQPKGSLKLSCAASGFTFNTYAMNWVRQAPGKGLEWVARIRSKYNNYATYYADSVKDRFTISRDDSQSILYLQMNNLKTEDTAMYYCVRHGNFGNSYVSWFAYWGQGTLVTVSS |
| 26 | SP34 VL | QAVVTQESALTTSPGETVTLTCRSSTGAVTTSNYANWVQEKPDHLFTGLIGGTNKRAPGVPARFSGSLIGDKAALTITGAQTEDEAIYFCALWYSNLWVFGGGTKLTVL |

The whole bispecific antibody includes subtypes of IgA, IgD, IgE, IgM and IgG, and in particular, IgG includes IgG1, IgG2, IgG3 and IgG4. The heavy chain constant region has gamma ($\gamma$), mu ($\mu$), alpha ($\alpha$), delta ($\delta$) and epsilon ($\varepsilon$) types, and is subclassified into gamma 1 ($\gamma$1), gamma 2 ($\gamma$2), gamma 3 ($\gamma$3), gamma 4 ($\gamma$4), alpha 1 ($\alpha$1), and alpha 2 ($\alpha$2). The light chain constant region has kappa ($\kappa$) and lambda ($\lambda$) types.

The antigen-binding fragment of an antibody or antibody fragment refers to a fragment that has antigen-binding function and includes Fab, F(ab'), F(ab')2, Fv and the like. Among the antibody fragments, Fab refers to a structure including a variable region of each of the heavy chain and the light chain, the constant region of the light chain, and the first constant domain (CH1) of the heavy chain, each having one antigen-binding site. Fab' is different from Fab in that it further includes a hinge region including at least one cysteine residue at the C-terminus of the CH1 domain of the heavy chain.

F(ab')2 is created by a disulfide bond between cysteine residues in the hinge region of Fab'.

Fv is the minimal antibody fragment having only a heavy chain variable region and a light chain variable region. Two-chain Fv is a fragment in which the variable region of the heavy chain and the variable region of the light chain are linked by a non-covalent bond, and single-chain Fv (scFv) is a fragment in which the variable region of the heavy chain and the variable region of the light chain are generally linked by a covalent bond via a peptide linker therebetween, or are directly linked at the C-terminal, forming a dimer-shaped structure, like the two-chain Fv. Such antibody fragments may be obtained using proteases (e.g., Fab can be obtained by restriction-cleaving the complete antibody with papain, and the F(ab')2 fragment may be obtained by restriction-cleaving the complete antibody with pepsin), and may be produced using genetic recombination techniques.

An "Fv" fragment is an antibody fragment containing complete antibody recognition and binding sites. Such a region includes a dimer that consists of one heavy chain variable domain.

A "Fab" fragment contains a variable domain and a constant domain of the light chain and a variable domain and a first constant domain (CH1) of the heavy chain. A $F(ab')_2$ antibody fragment generally includes a pair of Fab fragments covalently linked near the carboxyl terminal thereof via a hinge cysteine therebetween.

The "single chain Fv" or "scFv" antibody fragment includes VH and VL domains of the antibody, wherein these domains are present in a single polypeptide chain. The Fv polypeptide may further include a polypeptide linker between the VH domain and the VL domain in order for the scFv to form a target structure for antigen binding.

In an embodiment, the bispecific antibody according to the present invention includes, but is not limited to multispecific antibodies, human antibodies, humanized antibodies, chimeric antibodies, scFVs, Fab fragments, F(ab') fragments, disulfide-bond Fvs (sdFVs), anti-idiotypic (anti-Id) antibodies, epitope-binding fragments of such antibodies, and the like.

The heavy chain constant region may be selected from gamma ($\gamma$), mu (u), alpha ($\alpha$), delta ($\delta$) and epsilon (c) isotypes. For example, the constant region may be gamma 1 (IgG1), gamma 3 (IgG3), or gamma 4 (IgG4). The light chain constant region may be kappa or lambda.

The bispecific antibody may include, but is not limited to, one or more selected from the group consisting of:

- a diabody including an antibody binding to IL-5R$\alpha$ and an antibody binding to CD3$\alpha$;
- an scFv antibody binding to IL-5R$\alpha$ and an scFv antibody binding to CD3$\alpha$;
- an antibody including a Fab binding to IL-5R$\alpha$ and a Fab binding to CD3$\alpha$; and
- a bispecific T-cell engager (BiTE) binding to IL-5R$\alpha$ and CD3$\alpha$, and an scFv antibody binding to CD3$\alpha$.

The term "bispecific antibody" refers to an identical antibody, which is obtained from a population of substantially homogeneous antibodies, that is, each antibody constituting the population, excluding possible naturally occurring mutations that may be present in trivial amounts. Bispecific antibodies are highly specific and are thus induced against a single antigenic site of each antigen.

For example, bispecific antibodies useful in the present invention may be produced by quadroma methods, or may be produced in bacterial, eukaryotic or plant cells using recombinant DNA methods.

The term "affinity" refers to the ability to specifically recognize a specific site of an antigen and to bind thereto, and specificity and high-affinity of an antibody for an antigen are important factors in the immune response. The affinity constant ($K_D$) can be determined using surface plasmon resonance (SPR), for example, a BIAcore system. An affinity constant ($K_D$) calculated from a 1:1 Langmuir binding model (simultaneously $k_{on}$ and $k_{off}$) and the ratio of the rate constant $k_{off}/k_{on}$ was obtained based on surface plasmon resonance data.

The binding affinity of the anti-IL-5R$\alpha$ antibody, 5R65.7, to IL-5R$\alpha$ ranges from $10^{-5}$ M to $10^{-12}$ M. For example, the binding affinity is $10^{-6}$ M to $10^{-12}$ M, $10^{-7}$ M to $10^{-12}$ M, $10^{-8}$ M to $10^{-12}$ M, $10^{-9}$ M to $10^{-12}$ M, $10^{-5}$ M to $10^{-11}$ M, $10^{-6}$ M to $10^{-11}$ M, $10^{-7}$ M to $10^{-11}$ M, $10^{-8}$ M to $10^{-11}$ M, $10^{-9}$ M to $10^{-11}$ M, $10^{-10}$ M to $10^{-11}$ M, $10^{-5}$ M to $10^{-10}$ M, $10^{-6}$ M to $10^{-10}$ M, $10^{-7}$ M to $10^{-10}$ M, $10^{-8}$ M to $10^{-10}$ M, $10^{-9}$ M to $10^{-10}$ M, $10^{-5}$ M to $10^{-9}$ M, $10^{-6}$ M to $10^{-9}$ M, $10^{-7}$ M to $10^{-9}$ M, $10^{-8}$ M to $10^{-9}$ M, $10^{-5}$ M to $10^{-8}$ M, $10^{-6}$ M to $10^{-8}$ M, $10^{-7}$ M to $10^{-8}$ M, $10^{-5}$ M to $10^{-7}$ M, $10^{-6}$ M to $10^{-7}$ M or $10^{-5}$ M to $10^{-6}$ M.

The binding affinity of anti-CD3$\varepsilon$ antibody SP34 to CD3$\varepsilon$ ranges from $10^{-5}$ M to $10^{-12}$ M. For example, the binding affinity may be $10^{-6}$ M to $10^{-12}$ M, $10^{-7}$ M to $10^{-12}$ M, $10^{-8}$ M to $10^{-12}$ M, $10^{-9}$ M to $10^{-12}$ M, $10^{-5}$ M to $10^{-11}$ M, $10^{-6}$ M to $10^{-11}$ M, $10^{-7}$ M to $10^{-11}$ M, $10^{-8}$ M to $10^{-11}$ M, $10^{-9}$ M to $10^{-11}$ M, $10^{-10}$ M to $10^{-11}$ M, $10^{-5}$ M to $10^{-10}$ M, $10^{-6}$ M to $10^{-10}$ M, $10^{-7}$ M to $10^{-10}$ M, $10^{-8}$ M to $10^{-10}$ M, $10^{-9}$ M to $10^{-10}$ M, $10^{-5}$ M to $10^{-9}$ M, $10^{-6}$ M to $10^{-9}$ M, $10^{-7}$ M to $10^{-9}$ M, $10^{-8}$ M to $10^{-9}$ M, $10^{-5}$ M to $10^{-8}$ M, $10^{-6}$ M to $10^{-8}$ M, $10^{-7}$ M to $10^{-8}$ M, $10^{-5}$ M to $10^{-7}$ M, $10^{-6}$ M to $10^{-7}$ M or $10^{-5}$ M to $10^{-6}$ M.

The non-human (e.g., murine) antibody of the "humanized" form is a chimeric antibody containing a minimal sequence derived from non-human immunoglobulin. In most cases, the humanized antibody is a human immunoglobulin (receptor antibody) in which a residue from the hypervariable region of a receptor is replaced with a residue from the hypervariable region of a non-human species (donor antibody) such as a mouse, rat, rabbit or non-human primate having the desired specificity, affinity and ability.

The term "human antibody" means a molecule derived from human immunoglobulin, wherein the entire amino acid sequence constituting the antibody including a complementarity-determining region and a structural region are composed of human immunoglobulin.

A part of the heavy chain and/or light chain is identical to or homologous with the corresponding sequence in an antibody derived from a particular species or belonging to a particular antibody class or subclass, while the other chain(s) include "chimeric" antibodies (immunoglobulins) which are identical to or homologous with corresponding sequences in an antibody derived from another species or belonging to another antibody class or subclass, as well as fragments of such antibody exhibiting the desired biological activity.

As used herein, the term "variable region" of the bispecific antibody refers to the light- and heavy chain regions of an antibody molecule including the amino acid sequences of a complementarity-determining region (CDR; i.e., CDR1, CDR2, and CDR3) and a framework region (FR). VH refers to a variable domain of the heavy chain. VL refers to a variable domain of the light chain.

The term "complementarity-determining region" (CDR; i.e., CDR1, CDR2, and CDR3) refers to an amino acid residue of the antibody variable domain that is necessary for antigen binding. Each variable domain typically has three CDR regions, identified as CDR1, CDR2, and CDR3.

The bispecific antibody according to the present invention may include an antibody (immunoglobulin) heavy chain constant region (Fc) pair including a first Fc region and a second Fc region, and the Fc pair may be a heterodimer including a mutation in the CH3 domain of the first Fc region or the second Fc region.

The bispecific antibody may bind to the C-terminus or N-terminus of the first Fc region or the second Fc region, but is not limited thereto.

The CH3 domain mutation of the first Fc region or the second Fc region may include at least one mutation selected from the following group and the position of the mutation may be determined in accordance with the EU index:

(1) K360E substitution at position K360 in the CH3 domain of the first Fc region;
(2) K409W substitution at position K409 in the CH3 domain of the first Fc region;
(3) E347R substitution at position E347 with in the CH3 domain of the second Fc region; and
(4) F405T substitution at position F405 and D399V substitution at position D399 in the CH3 domain of the second Fc region.

Specifically, the bispecific antibody according to the present invention may include the amino acid sequences set forth in Tables 1 and 5. Specifically, the bispecific antibody may include the sequences of SEQ ID NOs: 33 to 51.

In another aspect, the present invention is directed to a nucleic acid encoding the bispecific antibody. Various bispecific antibodies may be produced by isolating the nucleic acid encoding the bispecific antibody of the present invention.

As used herein, the term "amino acid" or "amino acid residue" refers to modified, synthetic, or rare amino acids if desired, but typically refers to an amino acid as defined in the art, for example, an amino acid selected from alanine (Ala or A); arginine (Arg or R); asparagine (Asn or N); aspartic acid (Asp or D); cysteine (Cys or C); glutamine (Gln or Q); glutamic acid (Glu or E); glycine (Gly or G); histidine (His or H); isoleucine (He or I); leucine (Leu or L); lysine (Lys or K); methionine (Met or M); phenylalanine (Phe or F); proline (Pro or P); serine (Ser or S); threonine (Thr or T); tryptophan (Trp or W); tyrosine (Tyr or Y); and valine (Val or V). Generally, the amino acid may be grouped into those having nonpolar side chains (e.g., Ala, Cys, He, Leu, Met, Phe, Pro, Val); negatively charged side chains (e.g., Asp, Glu); positively charged side chains (e.g., Arg, His, Lys); or uncharged polar side chains (e.g., Asn, Cys, Gln, Gly, His, Met, Phe, Ser, Thr, Trp, and Tyr).

As used herein, the term "fluorescent label" refers to any molecule that may be detected through its intrinsic fluorescent properties. Suitable fluorescent labels include, but are not limited to, fluorescein, rhodamine, tetramethylrhodamine, eosin, erythrosine, coumarin, methyl-coumarin, pyrene, malacite green, stilbene, Lucifer Yellow, Cascade Blue J, Texas Red, IAEDANS, EDANS, BODIPY FL, LC Red 640, Cy 5, Cy 5.5, LC Red 705, Oregon Green, Alexa-Fluor dyes (Alexa Fluor 350, Alexa Fluor 430, Alexa Fluor 488, Alexa Fluor 546, Alexa Fluor 568, Alexa Fluor 594, Alexa Fluor 633, Alexa Fluor 660, Alexa Fluor 680), Cascade Blue, Cascade Yellow, and R-phycoerythrin (PE) (Molecular Probes, Eugene, OR), FITC, rhodamine, and Texas Red (Pierce, Rockford, IL), Cy5, Cy5.5, Cy7 (Amersham Life Science, Pittsburgh, PA). Suitable optical dyes containing fluorophores are disclosed in the Molecular Probes Handbook by Richard P. Haugland.

The term "nucleic acid" is intended to encompass both DNA (gDNA and cDNA) and RNA molecules, and a nucleotide, which is a basic constituent unit of a nucleic acid, includes naturally derived nucleotides as well as analogs, wherein sugar or base moieties are modified. The sequence of the nucleic acid encoding heavy- and light chain variable regions of the present invention can vary. Such variation includes addition, deletion, or non-conservative or conservative substitution of nucleotides.

The DNA encoding the antibody can be easily separated or synthesized using conventional molecular biological techniques (for example, using an oligonucleotide probe capable of specifically binding to DNA encoding heavy and light chains of the antibody). Nucleic acids are isolated and inserted into replicable vectors for further cloning (amplification of DNA) or further expression. Based on this, in another aspect, the present invention is directed to a recombinant expression vector including the nucleic acid.

As used herein, the term "vector" refers to a means for expressing target genes in host cells, and includes plasmid vectors, cosmid vectors, and viral vectors such as bacteriophage vectors, adenovirus vectors, retroviral vectors and adeno-associated viral vectors. Vector components generally include, but are not limited to, one or more of the following components: signal sequences, replication origins, one or more antibiotic resistance marker genes, enhancer elements, promoters, and transcription termination sequences. The nucleic acid encoding the antibody is operably linked to promoters, transcription termination sequences or the like.

The term "operably linked" means a functional linkage between a nucleic acid expression regulation sequence *(e.g.,* an array of promoter, signal sequence or transcription regulator binding sites) and another nucleic acid sequence, and enables the regulation sequence to regulate the transcription and/or translation of the other nucleic acid sequence.

When a prokaryotic cell is used as a host, it generally includes a potent promoter capable of conducting transcription (such as a tac promoter, a lac promoter, a lacUV5 promoter, a lpp promoter, a pLλ promoter, a pRλ promoter, a rac5 promoter, an amp promoter, a recA promoter, SP6 promoter, a trp promoter, or a T7 promoter), a ribosome-binding site for initiation of translation, and a transcription/translation termination sequence. In addition, for example, when a eukaryotic cell is used as a host, it includes a promoter *(e.g.,* a metallothionein promoter, a β-actin promoter, a human hemoglobin promoter and a human muscle creatine promoter) derived from the genome of mammalian cells, or a promoter derived from a mammalian virus such as an adenovirus late promoter, vaccinia virus 7.5K promoter, SV40 promoter, cytomegalovirus (CMV) promoter, HSV tk promoter, mouse mammary tumor virus (MMTV) promoter, HIV LTR promoter, Moloney virus promoter, Epstein-Barr virus (EBV) promoter, or Rous sarcoma virus (RSV) promoter, and generally has a polyadenylation sequence as a transcription termination sequence.

Optionally, the vector may be fused with another sequence in order to facilitate purification of the antibody expressed therefrom. The sequence to be fused therewith includes, for example, glutathione S-transferase (Pharmacia, USA), maltose-binding protein (NEB, USA), FLAG (IBI, USA), 6x His (hexahistidine; Qiagen, USA) and the like.

The vector includes antibiotic-resistance genes commonly used in the art as selectable markers, and examples thereof include genes conferring resistance to ampicillin, gentamycin, carbenicillin, chloramphenicol, streptomycin, kanamycin, geneticin, neomycin and tetracycline.

In another aspect, the present invention is directed to a cell transfected with the recombinant expression vector. The host cell used to produce the antibody of the present invention may be a prokaryote, yeast or higher eukaryotic cell, but is not limited thereto.

Prokaryotic host cells such as *Escherichia coli,* the genus *Bacillus,* such as *Bacillus subtilis* and *Bacillus thuringiensis, Streptomyces* spp., *Pseudomonas* spp. (for example, *Pseudomonas putida), Proteus mirabilis* and *Staphylococcus* spp. (for example, *Staphylococcus carnosus)* can be used.

Interest in animal cells is the greatest, and examples of useful host cell lines include, but are not limited to, COS-7, BHK, CHO, CHOK1, DXB-11, DG-44, CHO/-DHFR, CV1, COS-7, HEK293, BHK, TM4, VERO, HELA, MDCK, BRL 3A, W138, Hep G2, SK-Hep, MMT, TRI, MRC 5, FS4, 3T3, RIN, A549, PC12, K562, PER.C6, SP2/0, NS-0, U20S, and HT1080.

In another aspect, the present invention is directed to a method of producing a bispecific antibody including culturing the host cells to produce a bispecific antibody, and isolating the produced bispecific antibody from the cultured cells, followed by purification.

Specifically, the method of producing a bispecific antibody binding to human IL-5Rα and CD3ε includes, but is not limited to:

(a) culturing a host cell expressing an antibody that binds to human IL-5Rα and an antibody that binds to human CD3ε according to the present invention to produce an antibody that binds to human IL-5Rα and human CD3ε according to the present invention; and
(b) recovering the produced bispecific antibody.

The host cells can be cultured in various media. Any commercially available medium can be used as a culture medium without limitation. All other essential supplements well-known to those skilled in the art may be included in appropriate concentrations. Culture conditions such as temperature and pH are those that are conventionally used with the host cells selected for expression, which will be apparent to those skilled in the art.

The recovery of the bispecific antibody can be carried out, for example, by centrifugation or ultrafiltration to remove impurities and further purification of the resulting product using, for example, affinity chromatography. Other additional purification techniques such as anion or cation exchange chromatography, hydrophobic interaction chromatography and hydroxyapatite (HA) chromatography may be used.

In another aspect, the present invention is directed to a conjugate in which the bispecific antibody is fused with a bioactive molecule selected from the group consisting of peptides, proteins, small-molecule drugs, nucleic acids, nanoparticles and liposomes.

The proteins include bispecific antibodies, fragments of antibodies, immunoglobulins, peptides, enzymes, growth factors, cytokines, transcription factors, toxins, antigenic peptides, hormones, transport proteins, motor function proteins, receptors, signaling proteins, storage proteins, membrane proteins, transmembrane proteins, internal proteins, external proteins, secreted proteins, viral proteins, sugar proteins, truncated proteins, protein complexes, chemically modified proteins and the like.

The term "small-molecule drugs" refers to an organic compound, an inorganic compound or an organometallic compound that has a molecular weight of less than about 1,000 Da and has activity as a therapeutic agent for diseases, which is widely used herein. The small-molecule drug used herein includes oligopeptides and other biomolecules having a molecular weight of less than about 1,000 Da.

As used herein, the term "nanoparticle" refers to a particle including a material having a diameter of 1 to 1,000 nm, and the nanoparticle may be a metal/metal core-shell complex including a metal nanoparticle, a metal nanoparticle core and a metal shell including the core, a metal/non-metal core-shell complex including a metal nanoparticle core and a non-metal shell surrounding the core, or a nonmetal/metal core-shell complex including a nonmetal nanoparticle core and a metal shell surrounding the core. According to one embodiment, the metal may be selected from gold, silver, copper, aluminum, nickel, palladium, platinum, magnetic iron, and oxides thereof, but is not limited thereto, and the nonmetal may be selected from silica, polystyrene, latex and acrylic substances, but is not limited thereto.

The liposome consists of one or more lipid bilayer membranes surrounding an aqueous internal compartment that can self-associate. Liposomes can be specified based on the type and size of the membrane thereof. Small unilamellar vesicles (SUVs) have a single membrane, and may have a diameter of 20 nm to 50 nm. Large unilamellar vesicles (LUV) may have a diameter of 50 nm or more. Oligolamellar large vesicles and multilamellar large vesicles have multiple, generally concentric, membrane layers, and may be 100 nm or more in diameter. Liposomes having a plurality of non-concentric membranes, that is, several small vesicles contained within larger vesicles, are called "multivesicular vesicles".

As used herein, the term "fusion" refers to the integration of two molecules having different or identical functions or structures, and includes fusion through any physical, chemical or biological method capable of binding the bispecific antibody to the protein, small-molecule drug, nanoparticle, or liposome. The fusion may preferably be carried out using a linker peptide, and the linker peptide may mediate the fusion with the bioactive molecule at various positions of the antibody light chain variable region, antibody, or fragment thereof according to the present invention.

The term "T cell-mediated cytotoxicity" refers to a reaction in which T-cells lyse target cells labeled by a specific bispecific antibody. Antibody-dependent cellular cytotoxicity (ADCC) also lyses the target, but involves actions of effector cells rather than T cells, and the immune complement system does not require other cells. Therefore, T cell-mediated cytotoxicity is distinguished from these two terms. ADCC is also independent of the immune complement system, which lyses the target, but does not require other cells. ADCC typically requires effector cells known to be natural killer (NK) cells that interact with immunoglobulin G (IgG) antibodies. However, macrophages, neutrophils and eosinophils can also mediate ADCC, for example, eosinophils that kill certain parasitic worms known as parasites via IgE antibodies.

The bioactive molecule that can be conjugated to the antibody according to the present invention is a small molecule drug, particularly preferably a drug that is effective in the treatment of allergic diseases, inflammatory diseases, and/or diseases caused by an increase in eosinophils, for example, hypereosinophilic syndrome (HES), hypereosinophilia, asthma including eosinophilic asthma, eosinophilic bronchial asthma (ABA) and severe eosinophilic bronchial asthma (ABA), chronic obstructive pulmonary disease (COPD), Churg-Strauss syndrome, eosinophilic esophagitis, eosinophilic gastroenteritis, eosinophilic gastrointestinal disease (EGID), atopic diseases such as atopic dermatitis, allergic diseases such as allergic rhinitis, immunoglobulin (IgE)-mediated food allergy, inflammatory bowel disease, allergic colitis, gastroesophageal reflux, endocardial myocardial fibrosis, Loeffler endocarditis, Davis disease, intermittent angioedema associated with eosinophilia, eosinophilia-myalgia syndrome/Spanish toxic oil syndrome, liver cirrhosis, dermatitis impetigo, bullous pemphigoid, Churg-Strauss syndrome, acute myelogenous eosinophilic leukemia, acute lymphocytic eosinophilic leukemia, systemic mast cell disease with eosinophilia, eczema, Wegner's granulomatosis, polyarteritis nodosa, eosinophilic vasculitis, rheumatoid arthritis, and the like.

More preferably, examples of the bioactive molecule include, but are not limited to, beta agonists such as indacaterol, formoterol, vilanterol, albuterol, levalbuterol, and theophylline, anticholinergic agents such as ipratropium, tiotropium, and glycopyrrolate, and leukotriene modifiers such as montelukast, zafirlukast, and zileuton.

In another aspect, the present invention is directed to a multispecific antibody including the bispecific antibody.

The term "binding valency" means the presence of a specified number of binding sites specific to an antigen in the molecule. As such, the terms "monovalent" and "bivalent", refer to the presence of each of 1 and 2 binding sites specific for an antigen in a molecule.

The term "multispecific antibody" refers to an antibody that has binding specificity for at least three different antigens. The multispecific antibody includes a tri- or higher antibody, for example, a trispecific antibody, a tetraspecific antibody, or an antibody that targets more targets.

Any antibody may be used without limitation as the antibody capable of forming a multispecific antibody using the antibody according to the present invention as long as it is used in combination with the IL-5Rα×CD3ε T cell-engaging bispecific antibody according to the present invention to provide synergistically therapeutic or prophylactic effects for diseases caused by an increase in eosinophils, for example, hypereosinophilic syndrome (HES), hypereosinophilia, asthma, including eosinophilic asthma, eosinophilic bronchial asthma (ABA) and severe eosinophilic bronchial asthma (ABA), chronic obstructive pulmonary disease (COPD), Churg-Strauss syndrome, eosinophilic esophagitis, eosinophilic gastroenteritis, eosinophilic gastrointestinal disease (EGID), atopic diseases such as atopic dermatitis, allergic diseases such as allergic rhinitis, immunoglobulin (IgE)-mediated food allergy, inflammatory bowel disease, allergic colitis, gastroesophageal reflux, endocardial myocardial fibrosis, Loeffler endocarditis, Davis disease, intermittent angioedema associated with eosinophilia, eosinophilia-myalgia syndrome/Spanish toxic oil syndrome, liver cirrhosis, dermatitis impetigo, bullous pemphigoid, Churg-Strauss syndrome, acute myelogenous eosinophilic leukemia, acute lymphocytic eosinophilic leukemia, systemic mast cell disease with eosinophilia, eczema, Wegner's granulomatosis, polyarteritis nodosa, eosinophilic vasculitis, and rheumatoid arthritis.

For example, suitable antibodies include, but are not limited to, anti-IgE antibodies such as omalizumab and ligelizumab, anti-interleukin or anti-interleukin receptor antibodies, such as anti-IL4R antibodies.

In another aspect, the present invention is directed to a composition for removing eosinophils containing the bispecific antibody or an antigen-binding fragment thereof as an active ingredient.

Conventional methods for suppressing eosinophils have involved inducing ADCC via natural killer cells (NK cells) using antibodies against IL5Ra. However, according to the present invention, eosinophils, a major cause of asthma and allergies, may be removed by inducing cytolysis of eosinophils by T cells using a bispecific antibody targeting an antigen specifically expressed on eosinophils.

In another aspect, the present invention is directed to a composition for preventing or treating allergic diseases, inflammatory diseases, and/or diseases caused by an increase in eosinophils, especially, diseases caused by an increase in eosinophils, containing the bispecific antibody or antigen-binding fragment thereof as an active ingredient.

Examples of diseases that can be treated using the bispecific antibody according to the present invention include, but are not limited to, hypereosinophilic syndrome (HES), hypereosinophilia, asthma, including eosinophilic asthma, eosinophilic bronchial asthma (ABA) and severe eosinophilic bronchial asthma (ABA), chronic obstructive pulmonary disease (COPD), Churg-Strauss syndrome, eosinophilic esophagitis, eosinophilic gastroenteritis, eosinophilic gastrointestinal disease (EGID), atopic diseases such as atopic dermatitis, allergic diseases such as allergic rhinitis, immunoglobulin (IgE)-mediated food allergy, inflammatory bowel disease, allergic colitis, gastroesophageal reflux, endocardial myocardial fibrosis, Loeffler endocarditis, Davis disease, intermittent angioedema associated with eosinophilia, eosinophilia-myalgia syndrome/Spanish toxic oil syndrome, liver cirrhosis, dermatitis impetigo, bullous pemphigoid, Churg-Strauss syndrome, acute myelogenous eosinophilic leukemia, acute lymphocytic eosinophilic leukemia, systemic mast cell disease with eosinophilia, eczema, Wegner's granulomatosis, polyarteritis nodosa, eosinophilic vasculitis, and rheumatoid arthritis.

In another aspect, the present invention is directed to a pharmaceutical composition for preventing or treating allergic diseases, inflammatory diseases, and/or diseases caused by an increase in eosinophils, containing (a) a pharmaceutically effective amount of the bispecific antibody according to the present invention, and (b) a pharmaceutically acceptable carrier.

The present invention is also directed to a method for preventing or treating allergic diseases, inflammatory diseases and/or diseases caused by an increase in eosinophils, including administering the bispecific antibody according to the present invention in an effective amount required for a patient.

The bispecific antibody according to the present invention is useful for the prevention or treatment of IL-5-mediated diseases by removing, inhibiting, or reducing IL-5 activity. The bispecific antibody according to the present invention binds to IL-5Rα and is used for the treatment of diseases associated with IL-5 activity. Examples of the diseases include eosinophilic asthma, chronic obstructive pulmonary disease (COPD), Churg-Strauss syndrome, eosinophilic esophagitis, eosinophilic gastroenteritis or heavy chain diseases.

In order to treat the autoimmune disease or related autoimmune condition, the IL-5Rα×CD3ε T cell-engaging bispecific antibody of the present invention can be administered to a patient in combination with other therapeutic agents using multi-drug therapy. The IL-5Rα×CD3ε T cell-engaging bispecific antibody may be administered simultaneously with, sequentially or alternately with immunosuppressive agents, or after resistance to other therapies appears. Immunosuppressive agents may be administered in an amount identical to or lower than that used in the art. The selection of preferred immunosuppressive agent may depend on many factors, including the type of disease to be treated and the patient's medical history.

As used herein, the term "prevention" refers to any action causing the suppression of growth of allergic diseases, inflammatory diseases, and/or diseases caused by an increase in eosinophils or the delay of progression of such diseases by administration of the composition according to the present invention. The term "treatment" means suppression of the progression of allergic diseases, inflammatory diseases, and/or diseases caused by an increase in eosinophils or alleviation or elimination of such diseases. The antibodies of the present invention can be useful both *in vitro* and *in*

*vivo* for applications involving cells expressing IL-5Rα.

The pharmaceutical composition of the present invention contains the bispecific antibody or the conjugate according to the present invention, and the pharmaceutical composition may further contain a pharmaceutically acceptable carrier, in addition to the component for administration of the pharmaceutical composition of the present invention. The term "pharmaceutically acceptable carrier" as used herein refers to a carrier or diluent that does not impair the biological activities or properties of the administered compound and does not stimulate an organism. Pharmaceutically acceptable carriers for compositions that are formulated into liquid solutions are sterilized and biocompatible, and examples thereof include saline, sterile water, buffered saline, albumin injection solutions, dextrose solutions, maltodextrin solutions, glycerol, and mixtures of one or more thereof. If necessary, other conventional additives such as antioxidants, buffers and bacteriostatic agents may be added. In addition, diluents, dispersants, surfactants, binders and lubricants can be additionally added to formulate injectable solutions such as aqueous solutions, suspensions and emulsions, pills, capsules, granules, or tablets.

The pharmaceutical composition according to the present invention may be any one of various oral or parenteral formulations. In this regard, the pharmaceutical composition may be formulated using an ordinary diluent or excipient such as a filler, a thickener, a binder, a wetting agent, a disintegrant, a surfactant, or the like. Solid formulations for oral administration may include tablets, pills, powders, granules, capsules and the like. Such a solid formulation is prepared by mixing at least one compound with at least one excipient such as starch, calcium carbonate, sucrose, lactose or gelatin. In addition to a simple excipient, a lubricant such as magnesium stearate or talc may be further used. Liquid formulations for oral administration may include suspensions, solutions for internal use, emulsions, syrups, and the like. In addition to a simple diluent such as water or liquid paraffin, various excipients such as wetting agents, sweeteners, aromatics and preservatives may be incorporated in the liquid formulations. In addition, formulations for parenteral administration include sterile aqueous solutions, non-aqueous solvents, suspensions, emulsions, lyophilizates, suppositories and the like. Useful non-aqueous solvents and suspensions include propylene glycol, polyethylene glycol, vegetable oils such as olive oil and injectable esters such as ethyl oleate. The base ingredients of suppositories include Witepsol, macrogol, Tween 61, cacao butter, laurin butter and glycerogelatin.

The method for treating inflammatory diseases using the bispecific antibody or the conjugate according to the present invention includes administering, to a subject, a pharmaceutically effective amount of the bispecific antibody or the conjugate. It will be apparent to those skilled in the art that an appropriate total daily dose can be determined based on the judgment of a medical specialist. In addition, the antibody, antigen-binding fragment thereof, or the conjugate may be administered in a single dose, or may be divided into multiple doses. However, in consideration of the objects of the present invention, the specific therapeutically effective amount for a certain patient is preferably determined depending upon a variety of factors, including the type and extent of the response to be achieved, as well as the presence of other agents used, the specific composition, the age, body weight, general state of health, gender, and diet of the patient, the administration time, the administration route, the treatment period, and drugs used in conjunction with or concurrently with the specific composition, and other similar factors well-known in the field of pharmaceuticals.

The subject to which the composition of the present invention is administered includes mammals including humans, without limitation thereto.

As used herein, the term "administration" refers to an action of supplying the pharmaceutical composition according to the present invention to a patient by any appropriate method, and the composition according to the present invention may be orally or parenterally administered through any one of various routes enabling the composition to be delivered to a target tissue.

The bispecific antibody according to the present invention may be used as a single drug or in combination with a conventional therapeutic agent.

Any drug may be used without limitation as the drug that can be used in combination therapy with the bispecific antibody according to the present invention so long as it can be used to treat diseases caused by an increase in eosinophils, for example, hypereosinophilic syndrome (HES), hypereosinophilia, asthma, including eosinophilic asthma, eosinophilic bronchial asthma (ABA) and severe eosinophilic bronchial asthma (ABA), chronic obstructive pulmonary disease (COPD), Churg-Strauss syndrome, eosinophilic esophagitis, eosinophilic gastroenteritis, eosinophilic gastrointestinal disease (EGID), atopic diseases such as atopic dermatitis, allergic diseases such as allergic rhinitis, immunoglobulin (IgE)-mediated food allergy, inflammatory bowel disease, allergic colitis, gastroesophageal reflux, endocardial myocardial fibrosis, Loeffler endocarditis, Davis disease, intermittent angioedema associated with eosinophilia, eosinophilia-myalgia syndrome/Spanish toxic oil syndrome, liver cirrhosis, dermatitis impetigo, bullous pemphigoid, Churg-Strauss syndrome, acute myelogenous eosinophilic leukemia, acute lymphocytic eosinophilic leukemia, systemic mast cell disease with eosinophilia, eczema, Wegner's granulomatosis, polyarteritis nodosa, eosinophilic vasculitis, and rheumatoid arthritis.

Preferably, examples of the drug include, but are not limited to, beta agonists such as indacaterol, formoterol, vilanterol, albuterol, levalbuterol, and theophylline, anticholinergic agents such as ipratropium, tiotropium, and glycopyrrolate, leukotriene modifiers such as montelukast, zafirlukast, and zileuton, inhaled corticosteroids such as fluticasone propionate, budesonide, ciclesonide, beclomethasone, and mometasone, and anti-IgE antibodies such as omalizumab and ligelizumab.

In addition, the present invention is directed to a method of treating a disease including administering the bispecific antibody according to the present invention to a patient in need of treatment.

In another aspect, the present invention is directed to a composition for diagnosing allergic diseases, inflammatory diseases, and/or diseases caused by an increase in eosinophils including the bispecific antibody or antigen-binding fragment thereof. In another aspect, the present invention is directed to a kit for diagnosing allergic diseases, inflammatory diseases, and/or diseases caused by an increase in eosinophils containing the composition for diagnosing the diseases.

As used herein, the term "diagnosis" means determining the presence or features of pathophysiology. In the present invention, diagnosis serves to determine the onset or progress of diagnosing allergic diseases, inflammatory diseases, and/or diseases caused by an increase in eosinophils.

For diagnostic methods using the bispecific antibody according to the present invention, the drug may include a detectable label used to detect the presence of IL-4Rα antigen-expressing cells *in vitro* or *in vivo.* Radioisotopes that are detectable *in vivo* such as labels that can be detected using scintillation, magnetic resonance imaging or ultrasound can be used for clinical diagnostic applications. Useful scintillation labels include positron emitters and γ-emitters. Representative contrast agents as magnetic sources for imaging include paramagnetic or superparamagnetic ions (e.g., iron, copper, manganese, chromium, erbium, europium, dysprosium, holmium and gadolinium), iron oxide particles, and water-soluble contrast agents. For ultrasonic detection, a gas or liquid can be trapped in the porous inorganic particles released as a microbubble contrast agent. Detectable labels useful for *in-vitro* detection include fluorophores, detectable epitopes or binders and radiolabels.

The kit for diagnosing allergic diseases, inflammatory diseases, and/or diseases caused by an increase in eosinophils may further include a composition, solution or device having one or more other components suitable for the analysis method.

In one embodiment, the kit may include a bottle, vial, bag, needle, or syringe. The container may be made from various materials, such as glass, plastic, or metal. The label on the container may provide instructions for use. The kit may further include other materials desirable from commercial and usage perspectives, such as other buffers, diluents, filters, needles and syringes.

Hereinafter, the present invention will be described in more detail with reference to examples. However, it will be obvious to those skilled in the art that these examples are provided only for illustration of the present invention and should not be construed as limiting the scope of the present invention.

Example 1: Construction of eight formats of IL-5Rα×CD3ε T-cell-engaging bispecific antibodies

The structures of target cells and target antigens expressed on target cells, and the epitopes targeted by T-cell-mediated bispecific antibodies differ. The bispecific antibody formats have different affinities for each antigen, including avidity effects due to differences in binding valency. Furthermore, the degree of T-cell-mediated cytotoxicity varies depending on the proximity of the cytolytic synapse between T cells and eosinophils. Therefore, to select the T-cell-engaging bispecific antibody format that can most effectively eliminate target cells, T cell-engaging bispecific antibodies capable of specifically and simultaneously binding to human interleukin-5 receptor α and (IL-5Rα) and CD3ε were constructed in eight formats (diabody-Fc, scFv(L)/scFv-Fc, scFV(H)/scFv-Fc, BiTE-Fc, scFv/Fab-Fc, IgG analog, BiTE(L)/scFv-Fc, BiTE(H)/scFv-Fc; FIG. 1) having different antibody sizes based on different binding valencies and different binding sites and constant and variable regions of the antibody. The Fc mutants for the construction of fusion proteins were obtained using heterodimeric heavy chain constant region Fc (γ1, FEA, EW-RVT) which included the EW-RVT mutation to form a heterodimeric heavy chain constant region (heterodimeric Fc) based on IgG1 and L234F/L235E/-D265A mutations (hereinafter referred to as "FEA mutations") to prevent unnecessary *in vivo* clearance due to unintended ADCC/ADCP/CDC functions (Liu et al, 2020). The names, structures, predicted molecular weights, and expression levels of the eight bispecific antibody formats are shown in FIG. 1 (FIG. 1).

FIGS. 2A to 2H are schematic diagrams illustrating nucleic acids for expressing each format of IL-5Rα×CD3ε T cell-engaging bispecific antibodies. DNA encoding proteins into which heterodimeric heavy chain constant region Fc mutant pairs were introduced was cloned in-frame into pcDNA3.4 (+) (Invitrogen, USA), an animal cell expression vector having a CMV promoter, using NotI/HindIII, to form a signal sequence-nucleic acid sequence encoding SEQ ID NOS: 27 to 42-stop codon. The VH CDR1, VH CDR2, VH CDR3, VL CDR1, VL CDR2, and VL CDR3 sequences of 5R65.7 and SP34 used herein are as follows:

| SEQ ID NO: | Designation | CDR sequence |
|---|---|---|
| 1 | 5R65.7 VH CDR1 | SYWIN |
| 2 | 5R65.7 VH CDR2 | HIYPNKNENYYNHKFKD |
| 3 | 5R65.7 VH CDR3 | EFYGRQYYQAMDY |
| 4 | 5R65.7 VL CDR1 | KASQNVGTAVA |
| 5 | 5R65.7 VL CDR2 | WASTRHT |
| 6 | 5R65.7 VL CDR3 | QQFGRYPYT |
| 7 | SP34 VH CDR1 | TYAMN |
| 8 | SP34 VH CDR2 | RIRSKYNNYATYYADSVKD |
| 9 | SP34 VH CDR3 | HGNFGNSYVSWFAY |
| 10 | SP34 VL CDR1 | RSSTGAVTTSNYAN |
| 11 | SP34 VL CDR2 | GTNKRAP |
| 12 | SP34 VL CDR3 | ALWYSNLWV |

In addition, as a negative control for subsequent biological activity experiments, HER2×CD3ε IgG analogs were constructed by replacing the VH and VL CDR regions of the anti-IL-5Rα antibody 5R65.7 with trastuzumab, a HER2-targeting antibody. FIG. 2I is a schematic diagram illustrating four nucleic acids used to express HER2×CD3ε IgG analogs (FIG. 2I).

Table 1 below shows the amino acid sequences encoded by the nucleic acids used to construct the eight IL-5Rα×CD3ε T cell-engaging bispecific antibodies and the control bispecific antibodies used in the examples described later.

[Table 1]

| SEQ ID NO | Bispecific antibody containing proteins having amino acid sequence | Amino acid sequence |
|---|---|---|
| 27 | IL-5Rα×CD3ε-Diabody-Fc (including CH3A) | MGWSCIILFLVATATGVHSDIQMTQSPSTLSASVGDRVTITCKASQN<br>VGTAVAWYQQKPGKAPKLLIYWASTRHTGVPDRFSGSGSGTDFTLTI<br>SSLQPDDFATYYCQQFGRYPYTFGSGTKVEVKGGGSGGGGEVQLVES<br>GGGLVQPKGSLKLSCAASGFTFNTYAMNWVRQAPGKGLEWVARIRSK<br>YNNYATYYADSVKDRFTISRDDSQSILYLQMNNLKTEDTAMYYCVRH<br>GNFGNSYVSWFAYWGQGTLVTVSSGCPPCPAPEFEGGPSVFLFPPKP<br>KDTLMISRTPEVTCVVVAVSHEDPEVKFNWYVDGVEVHNAKTKPREE<br>QYNSTYRVVSVLTVLHQDWLNGKEYKCKVSNKALPAPIEKTISKAKG<br>QPREPQVYTLPPSRDELTENQVSLTCLVKGFYPSDIAVEWESNGQPE<br>NNYKTTPPVLDSDGSFFLYSWLTVDKSRWQQGNVFSCSVMHEALHNH<br>YTQKSLSLSPGK- |

(continued)

| SEQ ID NO | Bispecific antibody containing proteins having amino acid sequence | Amino acid sequence |
|---|---|---|
| 28 | IL-5Rα×CD3ε-Diabody-Fc (including CH3B) | MGWSCIILFLVATATGVHSQAVVTQESALTTSPGETVTLTCRSSTGA VTTSNYANWVQEKPDHLFTGLIGGTNKRAPGVPARFSGSLIGDKAAL TITGAQTEDEAIYFCALWYSNLWVFGGGTKLTVLGGGSGGGGQVQLV QSGAEVKKPGSSVKVSCKASGYTFTSYWINWVRQAPGQGLEWIGHIY PNKNENYYNHKFKDKATLTVDKSTNTAYMELSSLRSEDTAVYYCTRE FYGRQYYQAMDYWGQGTTLTVSSGCPPCPAPEFEGGPSVFLFPPKPK DTLMISRTPEVTCVVVAVSHEDPEVKFNWYVDGVEVHNAKTKPREEQ YNSTYRVVSVLTVLHQDWLNGKEYKCKVSNKALPAPIEKTISKAKGQ PREPRVYTLPPSRDELTKNQVSLTCLVKGFYPSDIAVEWESNGQPEN NYKTTPPVLVSDGSFTLYSKLTVDKSRWQQGNVFSCSVMHEALHNHY TQKSLSLSPGK- |
| 29 | IL-5Rα×CD3ε-scFv (L) /scFv-Fc, IL-5Rα×CD3ε-scFv(H)/scFv-Fc, IL-5Rα×CD3ε-scFv/Fab-Fc (INCLUDING CH3A) | MGWSCIILFLVATATGVHSQAVVTQESALTTSPGETVTLTCRSSTGA VTTSNYANWVQEKPDHLFTGLIGGTNKRAPGVPARFSGSLIGDKAAL TITGAQTEDEAIYFCALWYSNLWVFGGGTKLTVLGGGGSGGGGSGGG GSEVQLVESGGGLVQPKGSLKLSCAASGFTFNTYAMNWVRQAPGKGL EWVARIRSKYNNYATYYADSVKDRFTISRDDSQSILYLQMNNLKTED TAMYYCVRHGNFGNSYVSWFAYWGQGTLVTVSSEPKSCDKTHTCPPC PAPEFEGGPSVFLFPPKPKDTLMISRTPEVTCVVVAVSHEDPEVKFN WYVDGVEVHNAKTKPREEQYNSTYRVVSVLTVLHQDWLNGKEYKCKV SNKALPAPIEKTISKAKGQPREPQVYTLPPSRDELTENQVSLTCLVK GFYPSDIAVEWESNGQPENNYKTTPPVLDSDGSFFLYSWLTVDKSRW QQGNVFSCSVMHEALHNHYTQKSLSLSPGK- |
| 30 | IL-5Rα×CD3ε-scFv(L)/scFv-Fc (INCLUDING CH3B) | MGWSCIILFLVATATGVHSDIQMTQSPSTLSASVGDRVTITCKASQN VGTAVAWYQQKPGKAPKLLIYWASTRHTGVPDRFSGSGSGTDFTLTI SSLQPDDFATYYCQQFGRYPYTFGSGTKVEVKGGGGSGGGGSGGGGS QVQLVQSGAEVKKPGSSVKVSCKASGYTFTSYWINWVRQAPGQGLEW IGHIYPNKNENYYNHKFKDKATLTVDKSTNTAYMELSSLRSEDTAVY YCTREFYGRQYYQAMDYWGQGTTLTVSSEPKSCDKTHTCPPCPAPEF EGGPSVFLFPPKPKDTLMISRTPEVTCVVVAVSHEDPEVKFNWYVDG VEVHNAKTKPREEQYNSTYRVVSVLTVLHQDWLNGKEYKCKVSNKAL PAPIEKTISKAKGQPREPRVYTLPPSRDELTKNQVSLTCLVKGFYPS DIAVEWESNGQPENNYKTTPPVLVSDGSFTLYSKLTVDKSRWQQGNV FSCSVMHEALHNHYTQKSLSLSPGK- |
| 31 | IL-5Rα×CD3ε-scFv(H)/scFv-Fc, IL-5Rα×CD3ε-BiTE(L) /scFv-Fc (INCLUDING CH3B) | MGWSCIILFLVATATGVHSQVQLVQSGAEVKKPGSSVKVSCKASGYT FTSYWINWVRQAPGQGLEWIGHIYPNKNENYYNHKFKDKATLTVDKS TNTAYMELSSLRSEDTAVYYCTREFYGRQYYQAMDYWGQGTTLTVSS GGGGSGGGGSGGGGSDIQMTQSPSTLSASVGDRVTITCKASQNVGTA VAWYQQKPGKAPKLLIYWASTRHTGVPDRFSGSGSGTDFTLTISSLQ PDDFATYYCQQFGRYPYTFGSGTKVEVKEPKSCDKTHTCPPCPAPEF EGGPSVFLFPPKPKDTLMISRTPEVTCVVVAVSHEDPEVKFNWYVDG VEVHNAKTKPREEQYNSTYRVVSVLTVLHQDWLNGKEYKCKVSNKAL PAPIEKTISKAKGQPREPRVYTLPPSRDELTKNQVSLTCLVKGFYPS DIAVEWESNGQPENNYKTTPPVLVSDGSFTLYSKLTVDKSRWQQGNV FSCSVMHEALHNHYTQKSLSLSPGK- |

(continued)

| SEQ ID NO | Bispecific antibody containing proteins having amino acid sequence | Amino acid sequence |
|---|---|---|
| 32 | IL-5Rα×CD3ε-BiTE-Fc (INCLUDING CH3A) | MGWSCIILFLVATATGVHSDIQMTQSPSTLSASVGDRVTITCKASQN<br>VGTAVAWYQQKPGKAPKLLIYWASTRHTGVPDRFSGSGSGTDFTLTI<br>SSLQPDDFATYYCQQFGRYPYTFGSGTKVEVKGGGGSGGGGSGGGGS<br>QVQLVQSGAEVKKPGSSVKVSCKASGYTFTSYWINWVRQAPGQGLEW<br>IGHIYPNKNENYYNHKFKDKATLTVDKSTNTAYMELSSLRSEDTAVY<br>YCTREFYGRQYYQAMDYWGQGTTLTVSSGGGGSEVQLVESGGGLVQP<br>KGSLKLSCAASGFTFNTYAMNWVRQAPGKGLEWVARIRSKYNNYATY<br>YADSVKDRFTISRDDSQSILYLQMNNLKTEDTAMYYCVRHGNFGNSY<br>VSWFAYWGQGTLVTVSSGGGGSGGGGSGGGGSQAVVTQESALTTSPG<br>ETVTLTCRSSTGAVTTSNYANWVQEKPDHLFTGLIGGTNKRAPGVPA<br>RFSGSLIGDKAALTITGAQTEDEAIYFCALWYSNLWVFGGGTKLTVL<br>EPKSCDKTHTCPPCPAPEFEGGPSVFLFPPKPKDTLMISRTPEVTCV<br>VVAVSHEDPEVKFNWYVDGVEVHNAKTKPREEQYNSTYRVVSVLTVL<br>HQDWLNGKEYKCKVSNKALPAPIEKTISKAKGQPREPQVYTLPPSRD<br>ELTENQVSLTCLVKGFYPSDIAVEWESNGQPENNYKTTPPVLDSDGS<br>FFLYSWLTVDKSRWQQGNVFSCSVMHEALHNHYTQKSLSLSPGK- |
| 33 | IL-5Rα×CD3ε-BiTE-Fc (INCLUDING CH3B) | MGWSCIILFLVATATGVHSEPKSCDKTHTCPPCPAPEFEGGPSVFLF<br>PPKPKDTLMISRTPEVTCVVVAVSHEDPEVKFNWYVDGVEVHNAKTK<br>PREEQYNSTYRVVSVLTVLHQDWLNGKEYKCKVSNKALPAPIEKTIS<br>KAKGQPREPRVYTLPPSRDELTKNQVSLTCLVKGFYPSDIAVEWESN<br>GQPENNYKTTPPVLVSDGSFTLYSKLTVDKSRWQQGNVFSCSVMHEA<br>LHNHYTQKSLSLSPGK- |
| 34 | IL-5Rα×CD3ε-scFv/Fab-Fc, IgG analog (INCLUDING CH3A) | MGWSCIILFLVATATGVHSQVQLVQSGAEVKKPGSSVKVSCKASGYT<br>FTSYWINWVRQAPGQGLEWIGHIYPNKNENYYNHKFKDKATLTVDKS<br>TNTAYMELSSLRSEDTAVYYCTREFYGRQYYQAMDYWGQGTTLTVSS<br>ASTKGPSVFPLAPSSKSTSGGTAALGCLVKDYFPEPVTVSWNSGALT<br>SGVHTFPAVLQSSGLYSLSSVVTVPSSSLGTQTYICNVNHKPSNTKV<br>DKKVEPKSCDKTHTCPPCPAPEFEGGPSVFLFPPKPKDTLMISRTPE<br>VTCVVVAVSHEDPEVKFNWYVDGVEVHNAKTKPREEQYNSTYRVVSV<br>LTVLHQDWLNGKEYKCKVSNKALPAPIEKTISKAKGQPREPRVYTLP<br>PSRDELTKNQVSLTCLVKGFYPSDIAVEWESNGQPENNYKTTPPVLV<br>SDGSFTLYSKLTVDKSRWQQGNVFSCSVMHEALHNHYTQKSLSLSPG<br>K- |
| 35 | IL-5Rα×CD3ε-scFv/Fab-Fc, IgG analog (Light chain binding to CH3A) | MGWSCIILFLVATATGVHSDIQMTQSPSTLSASVGDRVTITCKASQN<br>VGTAVAWYQQKPGKAPKLLIYWASTRHTGVPDRFSGSGSGTDFTLTI<br>SSLQPDDFATYYCQQFGRYPYTFGSGTKVEVKRTVAAPSVFIFPPSD<br>EQLKSGTASVKCLLNNFYPREAKVQWKVDNALQSGNSQESVTEQDSK<br>DSTYSLSSTLTLSKADYEKHKVYACEVTHQGLSSPVTKSFNRGEC- |
| 36 | IL-5Rα×CD3ε-IgG analog (INCLUDING CH3A) | MGWSCIILFLVATATGVHSEVQLVESGGGLVQPKGSLKLSCAASGFT<br>FNTYAMNWVRQAPGKGLEWVARIRSKYNNYATYYADSVKDRFTISRD<br>DSQSILYLQMNNLKTEDTAMYYCVRHGNFGNSYVSWFAYWGQGTLVT<br>VSSASTKGPSVFPLAPSSKSTSGGTAALGCLVKDYFPEPVTVSWNSG<br>ALTSGVHTFPAVLQSSGLYSLKSVVTVPSSSLGTQTYICNVNHKPSN<br>TKVDKKVEPKSCDKTHTCPPCPAPELLGGPSVFLFPPKPKDTLMISR<br>TPEVTCVVVDVSHEDPEVKFNWYVDGVEVHNAKTKPREEQYNSTYRV<br>VSVLTVLHQDWLNGKEYKCKVSNKALPAPIEKTISKAKGQPREPQVY<br>TLPPSRDELTENQVSLTCLVKGFYPSDIAVEWESNGQPENNYKTTPP<br>VLDSDGSFFLYSWLTVDKSRWQQGNVFSCSVMHEALHNHYTQKSLSL<br>SPGK- |
| 37 | IL-5Rα×CD3ε-IgG analog (INCLUDING CH3B) | QAVVTQESALTTSPGETVTLTCRSSTGAVTTSNYANWVQEKPDHLFT<br>GLIGGTNKRAPGVPARFSGSLIGDKAALTITGAQTEDEAIYFCALWY<br>SNLWVFGGGTKLTVLRTVAAPSVFIFPPSDEQLKSGTASVECLLNNF<br>YPREAKVQWKVDNALQSGNSQESVTEQDSKDSTYSLSSTLTLSKADY<br>EKHKVYACEVTHQGLSSPVTKSFNRGEC- |

(continued)

| SEQ ID NO | Bispecific antibody containing proteins having amino acid sequence | Amino acid sequence |
|---|---|---|
| 38 | IL-5Rα×CD3ε-BiTE (L) /scFv-Fc (INCLUDING CH3A) | MGWSCIILFLVATATGVHSDIQMTQSPSTLSASVGDRVTITCKASQN<br>VGTAVAWYQQKPGKAPKLLIYWASTRHTGVPDRFSGSGSGTDFTLTI<br>SSLQPDDFATYYCQQFGRYPYTFGSGTKVEVKGGGGSGGGGSGGGGS<br>QVQLVQSGAEVKKPGSSVKVSCKASGYTFTSYWINWVRQAPGQGLEW<br>IGHIYPNKNENYYNHKFKDKATLTVDKSTNTAYMELSSLRSEDTAVY<br>YCTREFYGRQYYQAMDYWGQGTTLTVSSGGGGSQAVVTQESALTTSP<br>GETVTLTCRSSTGAVTTSNYANWVQEKPDHLFTGLIGGTNKRAPGVP<br>ARFSGSLIGDKAALTITGAQTEDEAIYFCALWYSNLWVFGGGTKLTV<br>LGGGGSGGGGSGGGGSEVQLVESGGGLVQPKGSLKLSCAASGFTFNT<br>YAMNWVRQAPGKGLEWVARIRSKYNNYATYYADSVKDRFTISRDDSQ<br>SILYLQMNNLKTEDTAMYYCVRHGNFGNSYVSWFAYWGQGTLVTVSS<br>EPKSCDKTHTCPPCPAPEFEGGPSVFLFPPKPKDTLMISRTPEVTCV<br>VVAVSHEDPEVKFNWYVDGVEVHNAKTKPREEQYNSTYRVVSVLTVL<br>HQDWLNGKEYKCKVSNKALPAPIEKTISKAKGQPREPQVYTLPPSRD<br>ELTENQVSLTCLVKGFYPSDIAVEWESNGQPENNYKTTPPVLDSDGS<br>FFLYSWLTVDKSRWQQGNVFSCSVMHEALHNHYTQKSLSLSPGK- |
| 39 | IL-5Rα×CD3ε-BiTE(H)/scFv-Fc (INCLUDING CH3A) | MGWSCIILFLVATATGVHSDIQMTQSPSTLSASVGDRVTITCKASQN<br>VGTAVAWYQQKPGKAPKLLIYWASTRHTGVPDRFSGSGSGTDFTLTI<br>SSLQPDDFATYYCQQFGRYPYTFGSGTKVEVKGGGGSGGGGSGGGGS<br>QVQLVQSGAEVKKPGSSVKVSCKASGYTFTSYWINWVRQAPGQGLEW<br>IGHIYPNKNENYYNHKFKDKATLTVDKSTNTAYMELSSLRSEDTAVY<br>YCTREFYGRQYYQAMDYWGQGTTLTVSSGGGGSEVQLVESGGGLVQP<br>KGSLKLSCAASGFTFNTYAMNWVRQAPGKGLEWVARIRSKYNNYATY<br>YADSVKDRFTISRDDSQSILYLQMNNLKTEDTAMYYCVRHGNFGNSY<br>VSWFAYWGQGTLVTVSSGGGGSGGGGSGGGGSQAVVTQESALTTSPG<br>ETVTLTCRSSTGAVTTSNYANWVQEKPDHLFTGLIGGTNKRAPGVPA<br>RFSGSLIGDKAALTITGAQTEDEAIYFCALWYSNLWVFGGGTKLTVL<br>GCPPCPAPEFEGGPSVFLFPPKPKDTLMISRTPEVTCVVVAVSHEDP<br>EVKFNWYVDGVEVHNAKTKPREEQYNSTYRVVSVLTVLHQDWLNGKE<br>YKCKVSNKALPAPIEKTISKAKGQPREPQVYTLPPSRDELTENQVSL<br>TCLVKGFYPSDIAVEWESNGQPENNYKTTPPVLDSDGSFFLYSWLTV<br>DKSRWQQGNVFSCSVMHEALHNHYTQKSLSLSPGK- |
| 40 | IL-5Rα×CD3ε-BiTE(H)/scFv-Fc (INCLUDING CH3B) | MGWSCIILFLVATATGVHSDIQMTQSPSTLSASVGDRVTITCKASQN<br>VGTAVAWYQQKPGKAPKLLIYWASTRHTGVPDRFSGSGSGTDFTLTI<br>SSLQPDDFATYYCQQFGRYPYTFGSGTKVEVKGGGGSGGGGSGGGGS<br>QVQLVQSGAEVKKPGSSVKVSCKASGYTFTSYWINWVRQAPGQGLEW<br>IGHIYPNKNENYYNHKFKDKATLTVDKSTNTAYMELSSLRSEDTAVY<br>YCTREFYGRQYYQAMDYWGQGTTLTVSSGCPPCPAPEFEGGPSVFLF<br>PPKPKDTLMISRTPEVTCVVVAVSHEDPEVKFNWYVDGVEVHNAKTK<br>PREEQYNSTYRVVSVLTVLHQDWLNGKEYKCKVSNKALPAPIEKTIS<br>KAKGQPREPRVYTLPPSRDELTKNQVSLTCLVKGFYPSDIAVEWESN<br>GQPENNYKTTPPVLVSDGSFTLYSKLTVDKSRWQQGNVFSCSVMHEA<br>LHNHYTQKSLSLSPGK- |
| 41 | HER2×CD3ε-IgG analog (INCLUDING CH3B) | MGWSCIILFLVATATGVHSDIQMTQSPSSLSASVGDRVTITCRASQD<br>VNTAVAWYQQKPGKAPKLLIYSASFLYSGVPSRFSGSRSGTDFTLTI<br>SSLQPEDFATYYCQQHYTTPPTFGQGTKVEIKGGGSGGGGEVQLVES<br>GGGLVQPKGSLKLSCAASGFTFNTYAMNWVRQAPGKGLEWVARIRSK<br>YNNYATYYADSVKDRFTISRDDSQSILYLQMNNLKTEDTAMYYCVRH<br>GNFGNSYVSWFAYWGQGTLVTVSSGCPPCPAPEFEGGPSVFLFPPKP<br>KDTLMISRTPEVTCVVVAVSHEDPEVKFNWYVDGVEVHNAKTKPREE<br>QYNSTYRVVSVLTVLHQDWLNGKEYKCKVSNKALPAPIEKTISKAKG<br>QPREPQVYTLPPSRDELTENQVSLTCLVKGFYPSDIAVEWESNGQPE<br>NNYKTTPPVLDSDGSFFLYSWLTVDKSRWQQGNVFSCSVMHEALHNH<br>YTQKSLSLSPGK- |

(continued)

| SEQ ID NO | Bispecific antibody containing proteins having amino acid sequence | Amino acid sequence |
|---|---|---|
| 42 | HER2×CD3ε -IgG analog (Light chain binding to CH3B) | DIQMTQSPSSLSASVGDRVTITCRASQDVNTAVAWYQQKPGKAPKLL IYSASFLYSGVPSRFSGSRSGTDFTLTISSLQPEDFATYYCQQHYTT PPTFGQGTKVEIKRTVAAPSVFIFPPSDEQLKSGTASVKCLLNNFYP REAKVQWKVDNALQSGNSQESVTEQDSKDSTYSLSSTLTLSKADYEK HKVYACEVTHQGLSSPVTKSFNRGEC- |

Example 2: Expression/purification of 8 formats of IL-5Rα×CD3ε T-cell-engaging bispecific antibodies

To express and purify 8 formats of IL-5Rα×CD3ε T-cell-engaging bispecific antibodies, HEK293F (Invitrogen) cells were transiently transfected. For 100 mL transfection, HEK293F cells were seeded in 90 mL of medium at a density of $1.0\times10^6$ cells/mL in a shaker flask (Corning) and incubated at 120 rpm, 8% $CO_2$, and 37°C. For Diabody-Fc, scFv(L)/scFv-Fc, scFv(H)/scFv-Fc, BiTE-Fc, BiTE(L)/scFv-Fc, and BiTE(H)/scFv-Fc, CH3A (EW) 62.5 μg, CH3B(RVT) 62.5 μg (CH3A(EW) :CH3B(RVT)=1:1); for scFv/Fab-Fc, CH3A (EW) 41.7 μg, CH3B (RVT) 41.7 μg, light chain binding to CH3B heavy chain 41.7 μg (CH3A(EW):CH3B(RVT):light chain binding to CH3B heavy chain=1:1:1); and for IgG analogs, CH3A (EW) 31.25 μg, CH3B (RVT) 31.25 μg, 31.25 μg of light chain binding to CH3A heavy chain, 31.25 μg of light chain binding to CH3B heavy chain (CH3A (EW): CH3B (RVT): light chain binding to CH3A heavy chain: light chain binding to CH3B heavy chain = 1:1:1:1) . Therefore, a total of 125 μg of the constructed plasmid was diluted in 4 ml of serum-free Freestyle 293 expression medium (Invitrogen), filtered, mixed with 6 ml of medium in which 375 μg of polyethylenimine (PEI) was diluted, and reacted at room temperature for 10 minutes. Then, the reacted mixed medium was added to the cells previously seeded in 90 ml, and incubated at 135 rpm, 8% $CO_2$, and cultured for 6 days. As a result, the produced protein was secreted outside the cells and accumulated in the medium. Therefore, the protein was purified from the cell culture supernatant collected by centrifugation at 3,800 rpm for 25 minutes after cell culture using a Protein A Sepharose column (GE Healthcare). The purification was performed in accordance with the standard protocol provided by the Protein A column manufacturer. The eluted protein was concentrated using a Vivaspin 30,000 MWCO (Satorius) centrifugal concentrator after buffer exchange with 1X PBS. The purified protein was measured for absorbance at 562 nm using the solution in the BCA protein assay kit (Thermo) and quantified according to the standard curve. FIG. 1 shows the purification yield for each format. The results were expressed as the standard error of the mean (mean ± S.D.) for three independent experiments.

Example 3: Purity and physical properties of eight IL-5Rα × CD3ε T cell-engaging bispecific antibodies

The purity of 3 μg of the purified bispecific antibody proteins in the eight formats described above was confirmed by SDS-PAGE analysis (FIG. 3A). For each format, the protein bands corresponding to the predicted molecular weights under non-reducing conditions are marked with red boxes. Among the purified proteins, scFv(H)/scFv-Fc, BiTE-Fc, scFv/Fab-Fc, and BiTE(H)/scFv-Fc did not have the desired antibody purity, and aggregates and unassembled bands corresponding to unbound two complementary heavy chains CH3A and CH3B were observed. Furthermore, the results of analysis of the reducing (RE) conditions for these formats showed that the expression levels of the complementary heavy chains CH3A and CH3B varied despite transfection using the same amount of nucleic acid.

FIG. 3B is a chromatogram showing the mAU at 280 nm as a function of time (min) for the quantitative physical properties of eight formats of IL-5Rα×CD3ε T cell-engaging bispecific antibodies, obtained by SEC (size exclusion chromatography). Measurements were performed using an HPLC (Agilent Technologies, USA) instrument and a Superdex200 10/300 GL column (Cytiva, USA) according to the following specific protocol.

Filtered and degassed triple-distilled water and 1×PBS (pH 7.4, 12 mM phosphate, 137 mM NaCl, 2.7 mM KCl) were prepared. Eight formats of IL-5Rα × CD3ε T cell-engaging bispecific antibodies were diluted to a concentration of 1 μg/μl and 30 μl of each antibody was added to 250 μl inserts with Polymer Feet, 100/PK (Agilent Technologies, USA) and placed in an autosampler vial to prepare a final analytic sample. In addition, markers to distinguish the size of unknown protein samples were also prepared under the same concentration conditions. The marker corresponding to 150 kDa is alcohol dehydrogenase, and the marker corresponding to 66 kDa is BSA.

First, triple-distilled water was supplied in an amount of 2-3 times the column volume at 0.5 ml/min to remove the initial storage buffer (20% ethanol) in the column and perform the standardization process. After this process was completed, 1×PBS (pH 7.4, 137 mM NaCl), a physiological condition running buffer, was supplied in an amount of 2-3 times the column volume at 0.75 ml/min to complete the standardization process before starting the analysis. After the prepared sample for analysis was loaded in the device, the analysis was performed at a flow rate of 0.75 ml/min and an injection amount of 30 μg (only 30 μl of the prepared 45 μl sample was injected) for 40 minutes. Then, analysis was performed for a post time of 10 mins. When the sample was injected in a predetermined amount by a sample injector, it flowed into the buffer-flowing tube and was injected into the column. When a buffer containing a sample is passed through a column filled with a porous resin, the time taken for the protein in the sample to elute out of the column varied depending on the molecular weight or size thereof. The physical properties were analyzed through a chromatogram obtained by measuring the change in absorbance at 280 nm. At this time, the chromatogram was analyzed under the principle that, as the size of the protein decreases, the protein passes through the inner pores of the porous resin and is eluted later, while, as the size of the protein increases, the protein passes through the column along the outer portion of the porous resin and is eluted earlier.

The purity and properties of eight formats of IL-5Rα×CD3ε T cell-engaging bispecific antibodies were analyzed using SDS-PAGE and SEC analysis (FIG. 3B). Diabody-Fc, scFv(L)/scFv-Fc, IgG analog, and BiTE(L)/scFv-Fc were found to have desirable properties based on the homogeneous protein composition thereof. However, scFv(H)/scFv-Fc, BiTE-Fc, scFv/Fab-Fc, and BiTE (H) /scFv-Fc were found to have undesirable physical properties, such as heterogeneous protein compositions, where purified proteins exist in undesirable aggregates or unassembled proteins with unbound CH3A and CH3B chains. Therefore, scFv(H)/scFv-Fc, BiTE-Fc, scFv/Fab-Fc, and BiTE(H)/scFv-Fc were excluded from the *in vitro* and ex *vivo* experiments using cell lines transfected to express IL-5Rα, activated PBMCs, and eosinophils described in Example 5 and thereafter.

Example 4: Construction of K562 cell line expressing IL-5Rα (K562/IL-5Rα)

To compare the biological activity of four IL-5Rα×CD3ε T cell-engaging bispecific antibodies, cell lines were first constructed. Specifically, the DNA encoding IL-5Rα was cloned into the pLJM1 (Addgene) lentiviral vector using SalI/EcoRI. 3 x $10^6$ HEK293T cells were cultured in 10 ml of medium containing 10% FBS in a cell culture plate for 12 hours at 5% $CO_2$ and 37°C. Once the cells were stabilized, the medium was removed and the plates were washed with DPBS (Welgene, LB001-04). 40 μl of Lipofectamine 3000 (Invitrogen, USA) was added to 600 μl of Opti-MEM media (Gibco), the constructed lentiviral vector and the viral packaging vectors pMDL, pRSV, and pVSV-G (Addgene) were carefully added thereto, allowed to react at room temperature for 20 minutes and then the result was added to the dish. In addition, 9 ml of DMEM containing no antibiotics was added thereto, and incubated in the presence of 5% $CO_2$ at 37°C for 6 hours, the medium was replaced with 10 ml of DMEM containing 10% FBS, and incubated for 48 hours. The medium obtained after transient transfection of the lentiviral vector was filtered and the viral particles in the medium were added to the cell culture dish containing the K562 cells prepared in advance. The puromycin resistance gene was used as a selection marker for antibiotic resistance.

Example 5: Isolation of human PBMCs and production of activated PBMCs

In the T cell-mediated cytotoxicity induction evaluation experiment performed in the example herein, T cells and PBMCs (activated PBMCs) produced from human PBMCs were used as effector cells. First, to isolate human PBMCs from human blood, blood from a healthy donor was diluted 1:1 with DPBS. 20 mL of a Ficoll-Paque solution (GE Healthcare, 17-5442-03) was aliquoted into 50 mL polypropylene centrifuge tubes to allow 20 mL of heparinized patient blood to be layered onto each tube. The tubes were centrifuged at 750 × g at room temperature for 20 minutes to separate the buffy coat layer for PBMC isolation. The isolated buffy coat layer was transferred to a 50 mL polypropylene centrifuge tube, the tube was filled with 50 mL of DPBS and centrifuged at 25°C and 1,500 rpm for 5 minutes. The supernatant was discarded and the tube was filled to 50 mL with DPBS again. This was centrifuged at 25°C and 1,500 rpm for 5 minutes and the PBMC layer was washed. PBMCs were then aliquoted at a density of 1 × $10^7$ cells/tube in a stock solution containing FBS and DMSO in 9:1 and stored in liquid nitrogen until use.

Human PBMC activation was performed using Dynabeads human T-activator CD3/CD28 (Gibco™, Cat#:11161D) in accordance with the manufacturer's protocol. After completion of the experiment, 1×$10^7$ cells were aliquoted into 1 ml of a stock solution containing FBS and DMSO in 9:1 and stored in liquid nitrogen until use.

Example 6: Comparison in binding ability to receptors expressed on the surface of cell lines expressing IL-5Rα and CD3ε between four formats of IL-5Rα×CD3ε T cell-engaging bispecific antibodies.

To evaluate the binding ability of four formats (Diabody-Fc, scFv(L)/scFv-Fc, IgG analog, BiTE(L)/scFv-Fc) of IL-5Rα×CD3ε T cell-engaging bispecific antibodies to target receptors (IL-5Rα, CD3ε) expressed on the cell surface, binding to cell surface antigens was analyzed using a FACS Calibur flow cytometer (Flow Cytometry, BD bioscience). In the experiment of receptor binding ability for K562/IL-5Rα, the positive control for the IL-5Rα×CD3ε T cell-engaging bispecific antibody was 5R65.7, which is an anti-IL-5Rα monoclonal antibody developed in a previous study, and the negative control was a HER2×CD3ε IgG analog constructed in the same manner as in IgG analog format using the anti-HER2 monoclonal antibody Trastuzumab and the humanized anti-CD3ε monoclonal antibody SP34. Human activated PBMC was used as the cell line expressing CD3ε. At this time, 5R65.7, a monoclonal antibody targeting IL-5Rα, was used as the negative control for the IL-5Rα×CD3ε T cell-engaging bispecific antibody. The wild-type K562 (WT K562) cell line that expresses neither IL-5Rα nor CD3ε was used as a negative control for the cell line. Specifically, K562/IL-5Rα, activated PBMC, and K562 cells seeded at 1 × $10^5$ cells/90 μl for each sample were mixed with primary antibodies at 50 nM (K562/IL-5Rα, K562) and 5 nM (activated PBMC), respectively, and incubated at 4°C for 60 minutes. The result was washed with PBSM (Miltenyi biotec; 130-091-221), mixed with Alexa Flour 647 AFFINIPURE Goat Anti-human IgG, Fcγ fragment specific 2nd antibody (Jacksonimmunology, 109-605-098) at a dilution of 500:1, incubated at 4°C for 30 minutes, and analyzed using a flow cytometer, FACS Calibur (BD Bioscience) . After analysis, a histogram for each sample was obtained (FIG. 4). All bispecific antibody formats exhibited receptor binding abilities for the target receptors similar to those of the positive control (IL-5Rα, CD3ε) and had no significant differences in receptor binding abilities between formats. All bispecific antibody formats did not bind to the wild-type K562 cell line, which indicates that non-specific binding to the cell surface where receptors are not expressed was not observed.

Example 7: Binding affinity analysis of four formats of IL-5Rα×CD3ε T cell-engaging bispecific antibodies (Diabody-Fc, scFv(L)/scFv-Fc, IgG Analog, BiTE(L)/scFv-Fc).

The binding affinities of four IL-5Rα×CD3ε T cell engaging bispecific antibodies (Diabody-Fc, scFv(L)/scFv-Fc, IgG Analog, BiTE(L)/scFv-Fc) to IL-5Rα and CD3ε were quantitatively analyzed using an Octet $QK^e$ (ForteBio, USA) instrument in accordance with the manufacturer's protocol.

Specifically, each bispecific antibody was diluted to a concentration of 5 μg/ml in DPBS (WELGENE). IL-5Rα, expressed and purified according to the method described in a previous study (Kim et al., 2021), and commercially available CD3ε (sinobiological, 10977-H08H) were sequentially diluted in DPBS to concentrations of 4,000, 2,000, 1,000, 500, 250, 125, and 0 nM. 200 μl of each solution was added to an opaque 96-well plate. The antigen affinity of the antibody was analyzed by measuring the change in refractive index that occurred during the binding of the antigen to the antibody and detachment of the antigen from the antibody while the AHC (anti-human IgG Fc capture) sensor chip was moved in the order of DPBS, antibody dilution solution, DPBS, antigen dilution solution, and DPBS. Affinities were calculated using octet data analysis software 11.0 using a 1:1 binding model. The results are shown in FIG. 5A. FIG. 5A shows the results of analysis of affinity for IL-5Rα and CD3ε of four different formats of IL-5Rα×CD3ε T cell-engaging bispecific antibodies using Octet $QK^e$.

[Table 2] below shows the numerical results of analysis of the affinity for each single antigen.

[Table 2]

| Antigen | Format | $K_D$ (nM) | $K_{on}$ ($M^{-1}s^{-1}$) | $K_{off}$ ($s^{-1}$) | $R^2$ |
|---|---|---|---|---|---|
| hIL-5Rα | Diabody-Fc | **123** | $2.5\times10^3$ | $3.06\times10^{-4}$ | 0.99 |
| | scFv(L)/scFv-Fc | **59.8** | $2.85\times10^3$ | $1.71\times10^{-4}$ | 0.99 |
| | IgG analog | **30.5** | $3.61\times10^3$ | $1.10\times10^{-4}$ | 0.99 |
| | BiTE(L) /scFv-Fc | **22.9** | $3.09\times10^4$ | $7.08\times10^{-4}$ | 0.96 |
| CD3ε | Diabody-Fc | **8.41** | $1.67\times10^5$ | $1.41\times10^{-3}$ | 0.95 |
| | scFv (L) /scFv-Fc | **3.61** | $1.76\times10^5$ | $6.34\times10^{-4}$ | 0.97 |
| | IgG analog | **1.71** | $2.78\times10^5$ | $4.74\times10^{-4}$ | 0.97 |
| | BiTE(L) /scFv-Fc | **4.62** | $1.79\times10^5$ | $8.27\times10^{-4}$ | 0.96 |

Meanwhile, to demonstrate that the four IL-5Rα×CD3ε T cell-engaging bispecific antibodies constructed above can simultaneously bind to IL-5Rα and CD3ε, the following protocol was used using an Octet $QK^e$ (ForteBio, USA) device. IL-5Rα and CD3ε were diluted to 4,000 nM in DPBS and 200 μl each was placed in an opaque 96-well plate that did not allow light to pass through.

Specifically, as the AR2G (amine reactive second-generation) sensor chip was moved in the following order: nuclease-free pure water (UPW), EDC/s-NHS diluted solution, 1 μM CD3ε diluted solution, 1 M ethanolamine solution, DPBS, and T cell-engaging bispecific antibody/diluted solution, the sequential binding of T cell-engaging bispecific antibody and IL-5Rα antigen to the CD3ε antigen immobilized on the AR2G sensor was analyzed qualitatively through two changes in refractive index that occurred simultaneously for one antibody. As a negative control, a solution diluted with HER2×CD3ε-IgG analog was added to the antibody dilution solution and the same experimental conditions were performed. The experimental results show that, for the four formats of IL-5Rα×CD3ε T cell-engaging bispecific antibodies, the refractive index increases as the sensor moves to the wells containing the T cell engaging bispecific antibodies and IL-5Rα, which indicates that both antigens can bind to the antibodies simultaneously, but the refractive index for the HER2×CD3ε-IgG analog decreases because it cannot target IL-5Rα. Therefore, this demonstrates that the IL-5Rα×CD3ε T cell-engaging bispecific antibody can specifically and simultaneously bind to IL-5Rε and CD3ε (FIG. 5B).

Example 8: Evaluation of T cell-dependent cytotoxicity-inducing abilities of four different formats of IL-5Rα×CD3ε T cell-engaging bispecific antibodies (Diabody-Fc, scFv(L)/scFv-Fc, IgG analog, BiTE(L)/scFv-Fc) against IL-5Rα-expressing cell lines and activated T cells (activated PBMCs)

The four different IL-5Rα×CD3α bispecific antibodies exhibited different receptor binding abilities due to the different formats thereof, and had differences in toxicity due to the distance of the cytolytic synapse between target cells and effector cells (i.e., T cells) caused by different antibody sizes. Therefore, to screen bispecific antibody formats that can most effectively reduce *in vivo* eosinophils, the present inventors evaluated the ability to induce T cell-mediated cytotoxicity in the K562/IL-5Rα cell line, which expresses IL-5Rα. To determine nonspecific T cell-mediated cytotoxicity in a cell line that does not express IL-5Rα, experiments were also conducted on wild-type K562 cells. The effector cells used herein were activated PBMCs, which have more T cells and are more activated than naive PBMCs. The activated PBMCs were used to compare the effectiveness of four formats in an environment where the activated PBMCs can bind to T cell-engaging bispecific antibodies to induce strong and rapid cytotoxicity.

Specifically, activated PBMCs and target cells (K562/IL-5Rα, wild-type K562) were used as effector and target cells, respectively, at a ratio of 5:1. In a U-shaped 96-well cell culture plate, 1 x $10^4$/well of target cells and four types of IL-5Rα×CD3ε T cell-engaging bispecific antibodies and a negative control HER2×CD3ε IgG analog were diluted to 10 nM, 1 nM, 100 pM, 10 pM, 100 fM, 10 fM, and 1 fM, aliquoted, and then incubated at 37°C in a $CO_2$ incubator for 1 hour to facilitate the binding of the bispecific antibodies and target cells to induce a T cell response. Then, 5 x $10^4$/well of human PBMCs were aliquoted. Each antibody was cultured on 2 wells and the final volume of each well was adjusted to 150 μl. The cells were cultured in a $CO_2$ incubator at 37°C for 24 hours. Two hours before harvesting the cell suspension, 15 μl of 10x lysis buffer was added to the sample to maximize lactate dehydrogenase (LDH) release. After incubation, 50 μl of the cell suspension was collected from each well. 50 μl of CytoTox96® reagent (Promega) was added and incubated at room temperature for 30 minutes. 50 μl of stop solution was added and absorbance was measured using a microplate reader to analyze T cell-mediated cytotoxicity induction. The Y and X axes represent the percent maximum cytotoxicity and antibody concentration, respectively.

$$\text{Cytotoxicity } (\%) = (A-B)/(C-B) \times 100$$

A: Release value upon antibody treatment

B: Spontaneous cell release value

C: Maximum cell release value

Analysis of the experimental results showed that the IL-5Rα × CD3ε T cell-engaging bispecific antibody did not induce significant cytotoxicity against cells that did not express IL-5Rα (FIG. 6B). It was found that scFv(L)/scFv-Fc, which can keep the two cells closest together and has a binding affinity for IL-5Rα that is approximately 2.1 times higher than the Diabody-Fc format, exhibited the lowest $EC_{50}$ and induced the highest cytotoxicity. Then, $EC_{50}$ increased in the order: Diabody-Fc, which is similar in size to scFv(L)/scFv-Fc but has a shorter hinge region; BiTE(L)/scFv-Fc, which has a binding valency of 2 for IL-5Rα; and the IgG analog format, which is the largest in size and has a form similar to a monoclonal antibody (Table 3). Therefore, the cytotoxicity induction ability increased in the order of scFv(L)/scFv-Fc, Diabody-Fc, BiTE(L)/scFv-Fc, and IgG analog.

[Table 3]

| IL-5Rα×CD3ε Format name | 24hr $EC_{50}$ |
|---|---|
| Diabody-Fc | 4.974±0.2460 pM |
| scFv(L)/scFv-Fc | 0.8571±0.5640 pM |
| IgG analog | 23.10±6.895 pM |
| BiTE (L) /scFv-Fc | 14.69±2.76 pM |

Example 9: Evaluation of the IFN-γ release-inducing ability of IL-5Rα×CD3ε T cell-engaging bispecific antibodies (Diabody-Fc, scFv(L)/scFv-Fc, IgG analog, BiTE(L)/scFv-Fc) from activated human PBMCs.

To determine whether or not the T cell-mediated cytotoxicity induction ability results obtained in Example 8 were due to T cells within activated PBMCs and to quantitatively assess the T cell activation-inducing ability of each format, interferon-γ in the supernatant was quantified using an enzyme-linked immunosorbent assay (ELISA). This is because interferon-γ release increases proportionally with the degree of T cell activation due to the nature of bispecific antibodies that induce T cell activation and T cell-mediated cytotoxicity. The IL-5Rα×CD3ε T cell-engaging bispecific antibody is identical to the previously tested bispecific antibody except that the target cells are *in vivo* eosinophils rather than cancer cells. Therefore, interferon-γ present in the supernatant was quantified to assess the degree of T cell activation.

The IFN gamma Human Uncoated ELISA (Invitrogen, Cat#: 88-7316) was used to quantify interferon-γ. For the experiment, supernatants were collected from each well treated with the four bispecific antibodies in the plate incubated for 24 hours in Example 8 and diluted 20-fold for use. After sample preparation, the experiment was conducted in accordance with the manufacturer's protocol. As with the results of Example 8, as cytotoxicity increased, the amount of Interferon-γ released by T cells also increased proportionally, and $EC_{50}$ also increased in the order of scFv (L) /scFv-Fc, Diabody-Fc, BiTE(L)/scFv-Fc, and IgG analog (FIG. 7, Table 4). When the results of Examples 8 and 9 are summarized, among clones, scFv(L)/scFv-Fc exhibited the highest cytotoxicity-inducing ability and cytokine release-inducing ability, and the IgG analog exhibited the lowest cytotoxicity-inducing ability and cytokine release-inducing ability. Considering all of the expression amount, physical properties, T cell-mediated cytotoxicity-inducing ability, and cytokine release-inducing ability, the format that was predicted to have the best eosinophil-reducing effect was scFv(L)/scFv-Fc. The subsequent *ex vivo* experiments using eosinophils were conducted using the scFv(L)/scFv-Fc format and a control group.

[Table 4]

| IL-5Rα×CD3ε format | IFN-γ $EC_{50}$ (pM) |
|---|---|
| Diabody-Fc | 2338 |
| scFv(L)/scFv-Fc | 769.5 |
| IgG analog | 2208 |
| BiTE(L)/scFv-Fc | 30971 |

Example 10: Confirmation of IL-5Rα expression in human granulocytes derived from healthy donors and patients with severe asthma

Prior to evaluation of the biological activity of the IL-5Rα×CD3ε-scFv(L)/scFv-Fc T cell-engaging bispecific antibody using eosinophils, IL-5Rα expression in eosinophils and neutrophils in the granulocyte layer of peripheral blood from healthy donors was determined

Specifically, 15 mL of Ficoll-Paque solution (GE Healthcare, 17-5442-03) was aliquoted into 50 mL polypropylene centrifuge tubes, and 20 mL of heparinized healthy donor blood was layered onto each tube. The tubes were centrifuged at 750 × g for 20 minutes at room temperature to isolate and recover the bottom layer. To remove mixed red blood cells, 10 x RBC lysis buffer (Invitrogen, 00-4300-54), diluted 10-fold, was aliquoted into 50 ml. The solution was then centrifuged at 300×g at 25°C for 10 minutes to separate and recover the enriched granulocyte layer (eosinophils and neutrophils) from which red blood cells were removed.

Enriched granulocytes may be classified into eosinophils and neutrophils based on the expression of Siglec-8 (sialic acid-binding immunoglobulin-like lectin 8). Eosinophils express Siglec-8, while neutrophils do not. Specifically, 1 × $10^6$ enriched granulocytes per donor sample were mixed with 5 μl of APC anti-human Siglec-8 Ab (Biolegend; 347105) and 10 μl of PE anti-human IL-5Rα Ab (BD Pharmingen; 555902), allowed to react at 4°C for 30 minutes, washed with PBSM (Miltenyi biotec; 130-091-221), and analyzed using a flow cytometer, FACS Calibur (BD Bioscience) . After analysis, dot graphs for each sample were obtained.

FIGS. 8 and 9 show the gating strategy for distinguishing eosinophils and neutrophils based on Siglec-8 expression in enriched granulocytes from healthy donors and patients with severe asthma, and the results of confirmation of IL-5Rα expression in eosinophils and neutrophils. FIG. 10 shows the results of confirmation of the expression of IL-5Rα in eosinophils and neutrophils. The proportion of eosinophils in patients with severe asthma was found to be significantly higher than that in healthy donors.

Example 11: Expression and physical properties of IL-5Rα(ben)×CD3ε-scFv(L)/scFv-Fc, IL-5Rα (5R65) ×CD3ε-scFv(L)/scFv-Fc, and HER2×CD3ε-scFv(L)/scFv-Fc.

The present inventors constructed a scFv(L)/scFv-Fc format using Benralizumab, a conventional FDA-approved anti-IL-5Rα monoclonal antibody, and compared *in vivo* eosinophil-reduction induction ability thereof between scFv(L)/scFv-Fc format and IL-5Rα×CD3ε-scFv(L)/scFv-Fc constructed with 5R65.7. The differences between benralizumab and 5R65.7 lie in the epitopes and affinities for the antigen. Benralizumab has an epitope in the D1 domain (membrane distal domain) far from the cell membrane and has an affinity for IL-5Rα of 26.8 nM, while 5R65.7 has an epitope in the D3 domain (membrane proximal domain) close to the cell membrane and has an affinity for IL-5Rα of 4.64 nM. Therefore, when scFv(L)/scFv-Fc was constructed using benralizumab (referred to as "IL-5Rα(ben) ×CD3ε-scFv(L)/scFv-Fc") and the cytotoxicity induction ability thereof was compared with that of IL-5Rα×CD3α scFv(L)/scFv-Fc. At this time, a problem arose in which it was unclear whether the difference was due to a difference in affinity or a difference in epitope. Therefore, in an attempt to precisely identify the cause of T cell-dependent cytotoxicity induction, scFv(L)/scFv-Fc was constructed using 5R65, the parent antibody of 5R65.7, that has a different epitope only (referred to as "IL-5Rα(5R65) ×CD3ε-scFv(L)/scFv-Fc") from 5R65.7, and has a lower binding affinity to the antigen and thus less difference in affinity with benralizumab.

FIGS. 11A to 11C are schematic diagrams illustrating nucleic acids for expressing each format of the IL-5Rα (ben)×CD3ε-scFv(L)/scFv-Fc, IL-5Rα(5R65)×CD3ε-scFv(L)/scFv-Fc, and HER2×CD3ε-scFv(L)/scFv-Fc T cell-engaging bispecific antibodies. DNA encoding proteins with heterodimeric heavy chain constant region Fc mutant pairs was cloned in-frame into pcDNA3.4 (+) (Invitrogen, USA), which is an animal cell expression vector having a CMV promoter, using NotI/HindIII, to form signal sequence-nucleic acid sequence encoding SEQ ID NOs: 43-45-stop codon. The VH CDR1, VH CDR2, VH CDR3, VL CDR1, VL CDR2, and VL CDR3 sequences of benralizumab and 5R65 used herein are disclosed in the following papers and patent (Kim et al, 2021) (Korean Patent No. 10-1973060).

| SEQ ID NO | Name | CDR sequence |
|---|---|---|
| 13 | Benralizumab VH CDR1 | SYVIH |
| 14 | Benralizumab VH CDR2 | YINPYNDGTKYNERFKG |
| 15 | Benralizumab VH CDR3 | EGIRYYGLLGDY |
| 16 | Benralizumab VL CDR1 | GTSEDIINYLN |
| 17 | Benralizumab VL CDR2 | HTSRLQS |
| 18 | Benralizumab VL CDR3 | QQGYTLPYT |

| SEQ ID NO | Name | CDR sequence |
|---|---|---|
| 1 | 5R65 VH CDR1 | SYWIN |
| 2 | 5R65 VH CDR2 | HIYPNKNENYYNHKFKD |
| 19 | 5R65 VH CDR3 | DYYGRSYYYAMDY |
| 4 | 5R65 VL CDR1 | KASQNVGTAVA |
| 5 | 5R65 VL CDR2 | WASTRHT |
| 6 | 5R65 VL CDR3 | QQFGRYPYT |

For the expression and purification of IL-5Rα(ben)×CD3ε-scFv(L)/scFv-Fc, IL-5Rα(5R65)×CD3ε-scFv(L)/scFv-Fc, and HER2×CD3ε-scFv(L)/scFv-Fc T cell-engaging bispecific antibodies, transient transfection was performed on HEK293F (Invitrogen) cells. When transfected in 100 mL of a shaker flask, HEK293F cells were seeded in 90 mL of medium at a density of $1.0\times10^6$ cells/mL and incubated at 120 rpm, 8% $CO_2$, and 37°C. The DNA ratio was CH3A (EW) 62.5 μg, CH3B (RVT) 62.5 μg (CH3A (EW): CH3B (RVT) = 1:1), and therefore, a total of 125 μg of the constructed plasmid was diluted in 5 ml of serum-free Freestyle 293 expression medium (Invitrogen), filtered, and mixed with 5 ml of medium in which 375 μg of polyethylenimine (PEI) was diluted, and reacted at room temperature for 10 minutes. Then, the reacted mixed medium was added to the cells seeded in 90 ml previously, and incubated at 120 rpm, 8% $CO_2$, and cultured for 6 days. The resulting produced protein was secreted outside the cells and accumulated in the medium. Therefore, the protein was purified using a protein A Sepharose column (GE healthcare) from the cell culture supernatant collected by centrifugation at 3,800 rpm for 25 minutes after cell culture. The purification method was based on the standard protocol provided by the protein A column company, and the eluted protein was concentrated after exchanging the buffer with 1× PBS using a Vivaspin 30,000 MWCO (Satorius) centrifugal concentrator. The absorbance of the purified protein was measured at a wavelength of 562 nm using a solution in the BCA protein assay kit (Thermo) and the amount thereof was assayed depending on the drawn standard curve. Table 4 shows the purification yield for each format. The resulting values were expressed as the standard errors of the mean (mean±S.D) from triplicate independent experiments.

The physical property measurement was performed in the same manner as in Example 3 and the results of SDS-PAGE and SEC are shown in FIGS. 12B and 12C. It can be seen from the results that there were no problems with the physical properties of the three IL-5Rα×CD3ε-scFv(L)/scFv-Fc control T cell-engaging bispecific antibodies.

The following [Table 5] shows the amino acid sequences encoded by the nucleic acid sequences used to construct the IL-5Rα(ben)×CD3ε-scFv(L)/scFv-Fc and IL-5Rα(5R65)×CD3ε-scFv(L)/scFv-Fc T cell-engaging bispecific antibodies.

[Table 5]

| SEQ ID NO | T cell-engaging bispecific antibody containing proteins including amino acid sequences | Amino acid sequence |
|---|---|---|
| 43 | IL-5Rα(ben)×CD3ε-scFv (L) / scFv-Fc (including CH3B) | DIQMTQSPSSLSASVGDRVTITCGTSEDIINYLNWYQQKPGKAPKLLIYHTSRLQSGVPSRFSGSGSGTDFTLTISSLQPEDFATYYCQQGYTLPYTFGQGTKVEIKGGGGSGGGGSGGGGSEVQLVQSGAEVKKPGASVKVSCKASGYTFTSYVIHWVRQRPGQGLAWMGYINPYNDGTKYNERFKGKVTITSDRSTSTVYMELSSLRSEDTAVYLCGREGIRYYGLLGDYWGQGTLVTVSSEPKSCDKTHTCPPCPAPEFEGGPSVFLFPPKPKDTLMISRTPEVTCVVVAVSHEDPEVKFNWYVDGVEVHNAKTKPREEQYNSTYRVVSVLTVLHQDWLNGKEYKCKVSNKALPAPIEKTISKAKGQPREPRVYTLPPSRDELTKNQVSLTCLVKGFYPSDIAVEWESNGQPENNYKTTPPVLVSDGSFTLYSKLTVDKSRWQQGNVFSCSVMHEALHNHYTQKSLSLSPGK- |
| 44 | IL-5Rα (5R65) ×CD3ε-scFv (L) /scFv-Fc (including CH3B) | DIQMTQSPSTLSASVGDRVTITCKASQNVGTAVAWYQQKPGKAPKLLIYWASTRHTGVPDRFSGSGSGTDFTLTISSLQPDDFATYYCQQFGRYPYTFGSGTKVEVKGGGGSGGGGSGGGGSQVQLVQSGAEVKKPGSSVKVSCKASGYTFTSYWINWVRQAPGQGLEWIGHIYPNKNENYYNHKFKDKATLTVDKSTNTAYMELSSLRSEDTAVYYCTRDYYGRSYYYAMDYWGQGTTLTVSSEPKSCDKTHTCPPCPAPEFEGGPSVFLFPPKPKDTLMISRTPEVTCVVVAVSHEDPEVKFNWYVDGVEVHNAKTKPREEQYNSTYRVVSVLTVLHQDWLNGKEYKCKVSNKALPAPIEKTISKAKGQPREPRVYTLPPSRDELTKNQVSLTCLVKGFYPSDIAVEWESNGQPENNYKTTPPVLVSDGSFTLYSKLTVDKSRWQQGNVFSCSVMHEALHNHYTQKSLSLSPGK- |
| 45 | HER2×CD3ε-scFv (L) /scFv-Fc (including CH3B) | DIQMTQSPSSLSASVGDRVTITCRASQDVNTAVAWYQQKPGKAPKLLIYSASFLYSGVPSRFSGSRSGTDFTLTISSLQPEDFATYYCQQHYTTPPTFGQGTKVEIKGGGGSGGGGSGGGGEVQLVESGGGLVQPGGSLRLSCAASGFNIKDTYIHWVRQAPGKGLEWVARIYPTNGYTRYADSVKGRFTISADTSKNTAYLQMNSLRAEDTAVYYCSRWGGDGFYAMDYWGQGTLVTVSSEPKSCDKTHTCPPCPAPEFEGGPSVFLFPPKPKDTLMISRTPEVTCVVVAVSHEDPEVKFNWYVDGVEVHNAKTKPREEQYNSTYRVVSVLTVLHQDWLNGKEYKCKVSNKALPAPIEKTISKAKGQPREPRVYTLPPSRDELTKNQVSLTCLVKGFYPSDIAVEWESNGQPENNYKTTPPVLVSDGSFTLYSKLTVDKSRWQQGNVFSCSVMHEALHNHYTQKSLSLSPGK- |

Example 12: Comparison of receptor binding abilities for IL-5Rα and CD3ε-expressing cell lines of IL-5Rα (ben) ×CD3α-Diabody Fc and IL-5Rα(5R65) ×CD3ε-Diabody Fc bispecific antibodies.

To determine the binding ability of IL-5Rα(ben)×CD3α-scFv(L)/scFv-Fc, IL-5Rα(5R65)×CD3ε-scFv(L)/scFv-Fc, and HER2×CD3ε-scFv(L)/scFv-Fc, which were constructed as controls for IL-5Rα×CD3ε-scFv(L)/scFv-Fc, to cell surface-expressed receptors, K562/IL-5Rα expressing IL-5Rα and activated PBMC expressing CD3 were analyzed using a FACS Calibur flow cytometer (Flow Cytometry, BD bioscience) in the same test manner as in Example 7 (FIG. 13).

The binding affinities (KD) of 5R65.7, 5R65, and benralizumab to IL-5Rα, as previously reported, were 4.64 ±0.23 nM, 14.5±0.29 nM, and 26.8±0.52 nM, respectively (Kim et al., 2021). The binding patterns for the receptor expressed on the surface of K562/IL-5Rα cells were consistent with the binding affinity patterns reported in the literature. Therefore, in a subsequent comparative experiment on the T cell-mediated cytotoxicity induction ability of IL-5Rα×CD3ε-scFv(L)/scFv-Fc and three control antibodies, the differences in biological efficacy were described in terms of binding affinity to antigen and the epitope.

Example 13: Comparison of T cell-engaging bispecific antibodies (IL-5Rα×CD3α-scFv(L)/scFv-Fc, IL-5Rα(ben)×CD3α-scFv(L)/scFv-Fc, and IL-5Rα(5R65)×CD3ε-scFv(L)/scFv-Fc) inducing T cell-mediated cytotoxicity against K562/IL-5Rα.

To compare the T cell-mediated cytotoxicity induction capacity between the IL-5Rα×CD3α-scFv(L)/scFv-Fc format and the previously constructed controls, IL-5Rα(ben)×CD3α-scFv(L)/scFv-Fc and IL-5Rα(5R65) ×CD3ε-scFv(L)/scFv-Fc, the T cell-mediated cytotoxicity induction capacity for K562/IL-5Rα was compared.

Specifically, activated PBMCs and target cells (K562/IL-5Rα, wild-type K562) were used as effector and target cells, respectively. K562/IL-5Rα cells (1 x $10^4$ cells/well), IL-5Rα×CD3α-scFv(L)/scFv-Fc, IL-5Rα(ben) ×CD3α-scFv(L)/scFv-Fc, IL-5Rα(5R65)×CD3α-scFv(L)/scFv-Fc, and HER2×CD3α-scFv(L)/scFv-Fc were diluted to 10 nM, 1 nM, 100 pM, 10 pM, 1 pM, 100 fM, 10 fM, 1 fM, 0.1 fM, and 0.01 fM, and aliquoted into a U-shaped 96-well cell culture plate and the cells were incubated in a $CO_2$ incubator at 37°C for 1 hour to facilitate the T cell response by binding the bispecific antibody to the target cells. Then, 5 x $10^4$ cells/well of human PBMCs were aliquoted. Each antibody was cultured on 2 wells and the final volume of each well was adjusted to 150 μl. The cells were cultured in a $CO_2$ incubator at 37°C for 24 hours. To compare the results under various experimental conditions, the process was performed under the following conditions: i. Effector cell:target cell ratio (E:T ratio) = 5:1, 24-hour culture, ii. E:T ratio = 10:1, 24-hour culture, iii. E:T ratio = 5:1, 12-hour culture. Two hours before harvesting the cell suspension, 15 μl of 10xlysis buffer was added to the sample to maximize lactate dehydrogenase (LDH) release. After incubation, 50 μl of the cell suspension was collected from each well. 50 μl of CytoTox96® reagent (Promega) was added and incubated at room temperature for 30 minutes. 50 μl of stop solution was added and absorbance was measured using a microplate reader to analyze T cell-mediated cytotoxicity induction. The Y and X axes represent the percent maximum cytotoxicity and antibody concentration, respectively.

$$\text{Cytotoxicity (\%)} = (A-B)/(C-B) \times 100$$

A: Release value upon antibody treatment

B: Spontaneous cell release value

C: Maximum cell release value

The T cell-mediated cytotoxicity induction ability of IL-5Rα×CD3ε-scFv(L)/scFv-Fc against K562/IL-5Rα was higher than that of IL-5Rα (ben) ×CD3ε-scFv(L)/scFv-Fc and IL-5Rα(5R65)×CD3ε-scFv(L)/scFv-Fc under i, ii, and iii conditions, and in particular, the difference in $EC_{50}$ between IL-5Rα(5R65)×CD3ε-scFv(L)/scFv-Fc and IL-5Rα (5R65)×CD3ε-scFv(L)/scFv-Fc was greater than that between IL-5Rα×CD3ε-scFv(L)/scFv-Fc and IL-5Rα (5R65)×CD3ε-scFv(L)/scFv-Fc. It was revealed that the difference in cytotoxicity induction between IL-5Rα×CD3ε-scFv(L)/scFv-Fc and IL-5Rα(ben)×CD3ε-scFv(L)/scFv-Fc was primarily due to differences in the epitopes of 5R65.7 and benralizumab. This can be seen from quantitative graphs (FIGS. 14A to 14C, Tables 6 to 8).

[Table 6. E:T=5:1, 24-hour culture]

| Clone name | 24hr $EC_{50}$ |
|---|---|
| IL-5Rα×CD3ε-scFv(L)/scFv-Fc | 0.7560 pM |
| IL-5Rα(5R65)×CD3ε-scFv(L)/scFv-Fc | 1.954 pM |
| IL-5Rα(ben)×CD3ε-scFv(L)/scFv-Fc | 22.55 pM |
| HER2×CD3ε-scFv(L)/scFv-Fc | 3175 pM |

[Table 7. E:T=10:1, 24-hour culture]

| Clone name | 24hr $EC_{50}$ |
|---|---|
| IL-5Rα×CD3ε-scFv(L)/scFv-Fc | 0.037 pM |
| IL-5Rα(5R65)×CD3ε-scFv(L)/scFv-Fc | 0.1289 pM |
| IL-5Rα(ben)×CD3ε-scFv(L)/scFv-Fc | 1.330 pM |
| HER2×CD3ε-scFv(L)/scFv-Fc | 10219 pM |

[Table 8. E:T=5:1, 12-hour culture]

| Clone name | 24hr $EC_{50}$ |
|---|---|
| IL-5Rα×CD3ε-scFv(L)/scFv-Fc | 0.039 pM |
| IL-5Rα(5R65)×CD3ε-scFv(L)/scFv-Fc | 0.1513 pM |
| IL-5Rα(ben)×CD3ε-scFv(L)/scFv-Fc | 0.5769pM |
| HER2×CD3ε-scFv(L)/scFv-Fc | 3751 pM |

Example 14: Comparison of T cell-mediated cytotoxicity induction ability against eosinophils from healthy donors between T cell-engaging bispecific antibodies (IL-5Rα×CD3α-scFv(L)/scFv-Fc, IL-5Rα(ben) ×CD3α-scFv(L)/scFv-Fc, and IL-5Rα(5R65) ×CD3ε-scFv(L)/scFv-Fc)

The result of evaluation of the T cell-mediated cytotoxicity induction ability against K562/IL-5Rα of the IL-5Rα×CD3ε-scFv(L)/scFv-Fc T cell-engaging bispecific antibody and the control groups, IL-5Rα(ben) ×CD3ε-scFv(L)/scFv-Fc and IL-5Rα(5R65)×CD3ε-scFv(L)/scFv-Fc, T cell-mediated cytotoxicity induction ability increased in the order of: IL-5Rα×CD3ε-scFv(L)/scFv-Fc that had the highest binding affinity and targeted the membrane proximal domain; IL-5Rα(5R65) ×CD3ε-scFv(L)/scFv-Fc that targeted the membrane proximal domain, but had a reduced binding affinity; and IL-5Rα(5R65)×CD3ε-scFv(L)/scFv-Fc that had the lowest antibody binding affinity and targeted the membrane distal domain. Therefore, to determine whether or not eosinophils exhibited similar efficacy differences, the present inventors evaluated T cell-mediated cytotoxicity induction ability using eosinophils as target cells present in the blood of healthy donors and patients with severe asthma.

Specifically, blood samples from healthy donors and patients with severe asthma were collected, and target and effector cells were isolated. 15 mL of Ficoll-Paque solution (GE Healthcare, 17-5442-03) was aliquoted into 50-mL polypropylene centrifuge tubes, and 20 mL of heparinized healthy donor blood was layered onto each tube. The results were centrifuged at 750 × g for 20 minutes at room temperature, and the bottom layer was isolated and recovered. To remove mixed red blood cells, 10x RBC lysis buffer (Invitrogen, 00-4300-54), which was diluted 10-fold, was aliquoted into 50 ml. Then, the supernatant was centrifuged at 300×g at 25°C for 10 minutes to separate and collect the layer enriched with granulocytes (eosinophils, neutrophils) from which red blood cells were removed. Finally, only eosinophils from granulocytes were purified using a commercially available eosinophil cell isolation kit (Miltenyi Biotec; 130-092-010) in accordance with the manufacturer's protocol.

Specifically, autologous activated PBMCs and target cells (eosinophils isolated from the blood of healthy donors and severe asthma patients) were used as effector cells and target cells, respectively, at a ratio of 5:1. K562/IL-5Rα cells (5 x $10^4$ cells/well) and IL-5Rα×CD3α-scFv(L)/scFv-Fc, IL-5Rα(ben)×CD3α-scFv(L)/scFv-Fc, IL-5Rα (5R65) ×CD3α-scFv(L)/scFv-Fc, and HER2×CD3α-scFv(L)/scFv-Fc were diluted to 10 nM, 1 nM, 100 pM, 10 pM, 1 pM, 100 fM, 10 fM, 1 fM, 0.1 fM, and 0.01 fM, and aliquoted onto a U-shaped 96-well cell culture plate, and the cells were incubated in a $CO_2$ incubator at 37°C for 1 hour, to facilitate the binding of the bispecific antibodies and target cells to induce a T cell response. Then, 2.5 x $10^5$ cells/well of autologous activated PBMCs were aliquoted. Human IL-5 was added at a final concentration of 100 pM to ensure minimal eosinophil survival during incubation. Each antibody was cultured on 2 wells and the final volume of each well was adjusted to 200 μl. 2 hours before harvesting the cell suspension, 15 μl of 10xlysis buffer was added to the sample to maximize lactate dehydrogenase (LDH) release. After the incubation was completed, 50 μl of cell suspension was harvested from each well, and 50 μl of CytoTox96® Reagent (Promega) sample was added and incubated at room temperature for 30 minutes. 50 μl of reaction stop solution was added and the absorbance was measured using a microplate reader to analyze the T cell-mediated cytotoxicity induction ability. The Y and X axes represent % maximum cytotoxicity and antibody concentration, respectively.

$$\text{Cytotoxicity (\%)} = (A-B)/(C-B) \times 100$$

A: Release value upon antibody treatment

B: Spontaneous cell release value

C: Maximum cell release value

The results of evaluation of the T cell-mediated cytotoxicity induction ability using a total of five healthy donors and five eosinophils are shown in FIG. 15. The result of evaluation of T cell-mediated cytotoxicity induction ability for K562/IL-5Rα cell line showed that the cytotoxicity induction ability increased in the order of IL-5Rα×CD3α-scFv(L)/scFv-Fc, IL-5Rα(5R65) ×CD3α-scFv(L)/scFv-Fc, and IL-5Rα(ben)×CD3α-scFv(L)/scFv-Fc, and the difference in T cell-mediated cytotoxicity induction ability between IL-5Rα×CD3α-scFv(L)/scFv-Fc and IL-5Rα(ben)×CD3α-scFv(L)/scFv-Fc was found to be due to the difference in the epitope targeted by 5R65.7 and benralizumab.

[Industrial applicability]

The present invention provides a bispecific antibody including an anti-IL-5Rα humanized antibody or anti-CD3ε humanized antibody, wherein the antibody inhibits the bioactivity of IL-5, suppresses the proliferation of patient-derived eosinophils, and eliminates eosinophils through T-cell mediated cytotoxicity.

Therefore, the present invention provides a bispecific antibody or antigen-binding fragment thereof that binds to interleukin (IL) -5 receptor α (IL-5Rα) and CD3α, including an antibody or antigen-binding fragment thereof that binds to IL-5Rα and an antibody or antigen-binding fragment thereof that binds to CD3α.

The bispecific antibody including an anti-IL-5Rα humanized antibody according to the present invention may be used for prevention or treatment of allergic diseases, inflammatory diseases, and/or diseases caused by an increase in eosinophils.

Examples of such diseases include, but are not limited to, hypereosinophilic syndrome (HES), hypereosinophilia, asthma including eosinophilic asthma, eosinophilic bronchial asthma (ABA) and severe eosinophilic bronchial asthma (ABA), chronic obstructive pulmonary disease (COPD), Churg-Strauss syndrome, eosinophilic esophagitis, eosinophilic gastroenteritis, eosinophilic gastrointestinal disease (EGID), atopic diseases such as atopic dermatitis, allergic diseases such as allergic rhinitis, immunoglobulin (IgE)-mediated food allergy, inflammatory bowel disease, allergic colitis, gastroesophageal reflux, endocardial myocardial fibrosis, Loeffler endocarditis, Davis disease, intermittent angioedema associated with eosinophilia, eosinophilia-myalgia syndrome/Spanish toxic oil syndrome, liver cirrhosis, dermatitis impetigo, bullous pemphigoid, Churg-Strauss syndrome, acute myelogenous eosinophilic leukemia, acute lymphocytic eosinophilic leukemia, systemic mast cell disease with eosinophilia, eczema, Wegner's granulomatosis, polyarteritis nodosa, eosinophilic vasculitis, rheumatoid arthritis, and the like.

Although specific configurations of the present invention have been described in detail, those skilled in the art will appreciate that this description is provided to set forth preferred embodiments for illustrative purposes and should not be construed as limiting the scope of the present invention. Therefore, the substantial scope of the present invention is defined by the accompanying claims and equivalents thereto.

[Sequence Listing Free Text]

An electronic file is attached.

## Claims

1. A bispecific antibody or antigen-binding fragment thereof binding to interleukin (IL)-5 receptor α (IL-5Rα) and CD3α, the bispecific antibody or antigen-binding fragment thereof comprising:

   an antibody or antigen-binding fragment thereof binding to IL-5Rα; and
   an antibody or antigen-binding fragment thereof binding to CD3α.

2. The bispecific antibody or antigen-binding fragment thereof according to claim 1, wherein the antibody or antigen-binding fragment thereof binding to IL-5Rα comprises:

   a heavy chain CDR1 of SEQ ID NO: 1, a heavy chain CDR2 of SEQ ID NO: 2, a heavy chain CDR3 of SEQ ID NO: 3, and a light chain CDR1 of SEQ ID NO: 4, a light chain CDR2 of SEQ ID NO: 5; a light chain CDR3 of SEQ ID NO: 6;
   a heavy chain CDR1 of SEQ ID NO: 13, a heavy chain CDR2 of SEQ ID NO: 14, a heavy chain CDR3 of SEQ ID NO: 15, and a light chain CDR1 of SEQ ID NO: 16; a light chain CDR2 of SEQ ID NO: 17; a light chain CDR3 of SEQ ID NO: 18; or
   a heavy chain CDR1 of SEQ ID NO: 1, a heavy chain CDR2 of SEQ ID NO: 2, a heavy chain CDR3 of SEQ ID NO: 19, and a light chain CDR1 of SEQ ID NO: 4; a light chain CDR2 of SEQ ID NO: 5, and
   the antibody or antigen-binding fragment thereof binding to CD3α comprises:
   a heavy chain CDR1 of SEQ ID NO: 7, a heavy chain CDR2 of SEQ ID NO: 8, a heavy chain CDR3 of SEQ ID NO: 9, and a light chain CDR1 of SEQ ID NO: 10; a light chain CDR2 of SEQ ID NO: 11; and a light chain CDR3 of SEQ ID NO: 12.

3. The bispecific antibody or antigen-binding fragment thereof according to claim 1, wherein the antibody or antigen-binding fragment thereof binding to IL-5Rα comprises:

   a heavy chain variable region of SEQ ID NO: 20 and a light chain variable region of SEQ ID NO: 21;
   a heavy chain variable region of SEQ ID NO: 22 and a light chain variable region of SEQ ID NO: 23; or
   a heavy chain variable region of SEQ ID NO: 24 and a light chain variable region of SEQ ID NO: 21, and
   the antibody or antigen-binding fragment thereof binding to CD3α comprises a heavy chain variable region of SEQ ID NO: 25 and a light chain variable region of SEQ ID NO: 26.

4. The bispecific antibody or antigen-binding fragment thereof according to claim 1, wherein the antibody or antigen-binding fragment thereof comprises at least one selected from the group consisting of:

a diabody including an antibody binding to IL-5Rα and an antibody binding to CD3α;
an scFv antibody binding to IL-5Rα and an scFv antibody binding to CD3α;
an antibody including a Fab binding to IL-5Rα and a Fab binding to CD3α; and
a bispecific T-cell engager (BiTE) binding to IL-5Rα and CD3α, and an scFv antibody binding to CD3α.

5. The bispecific antibody or antigen-binding fragment thereof according to claim 1, wherein the bispecific antibody or antigen-binding fragment thereof comprises an antibody (immunoglobulin) heavy chain constant region (Fc) pair including a first Fc region and a second Fc region and the Fc pair is a heterodimer including a mutation in the CH3 domain of the first Fc region or the second Fc region.

6. The bispecific antibody or antigen-binding fragment thereof according to claim 5, wherein the antibody binds to a C-terminus or N-terminus of the first Fc region or the second Fc region.

7. The bispecific antibody or antigen-binding fragment thereof according to claim 5, wherein the CH3 domain mutation of the first Fc region or the second Fc region comprises at least one mutation selected from the following group and a position of the mutation is determined in accordance with the EU index:

(1) K360E substitution at position K360 in the CH3 domain of the first Fc region;
(2) K409W substitution at position K409 in the CH3 domain of the first Fc region;
(3) E347R substitution at position E347 with in the CH3 domain of the second Fc region; and
(4) F405T substitution at position F405 and D399V substitution at position D399 in the CH3 domain of the second Fc region.

8. The bispecific antibody or antigen-binding fragment thereof according to claim 1, wherein the bispecific antibody or antigen-binding fragment thereof comprises sequences of SEQ ID NOS: 33 to 51.

9. A multi-specific antibody or antigen-binding fragment thereof comprising the bispecific antibody according to any one of claims 1 to 8.

10. A nucleic acid encoding the bispecific antibody according to any one of claims 1 to 8.

11. An expression vector comprising the nucleic acid according to claim 10.

12. A cell transformed with the expression vector according to claim 11.

13. A method of producing a bispecific antibody or antigen-binding fragment, the method comprising:

(a) culturing the cell according to claim 12 to produce a bispecific antibody or antigen-binding fragment thereof; and
(b) recovering the produced bispecific antibody or antigen-binding fragment thereof.

14. A composition for preventing or treating allergic diseases, inflammatory diseases, and/or diseases caused by an increase in eosinophils comprising the bispecific antibody or antigen-binding fragment thereof according to any one of claims 1 to 8.

15. The composition according to claim 14, wherein the allergic diseases, inflammatory diseases, and/or diseases caused by an increase in eosinophils are selected from the group consisting of hypereosinophilic syndrome (HES), hypereosinophilia, asthma, including eosinophilic asthma, eosinophilic bronchial asthma (ABA) and severe eosinophilic bronchial asthma (ABA), chronic obstructive pulmonary disease (COPD), Churg-Strauss syndrome, eosinophilic esophagitis, eosinophilic gastroenteritis, eosinophilic gastrointestinal disease (EGID), atopic diseases including atopic dermatitis, allergic diseases including allergic rhinitis, food allergic diseases including immunoglobulin (IgE)-mediated food allergy, inflammatory bowel disease, allergic colitis, gastroesophageal reflux, endocardial myocardial fibrosis, Loeffler endocarditis, Davis disease, intermittent angioedema associated with eosinophilia, eosinophilia-myalgia syndrome/Spanish toxic oil syndrome, liver cirrhosis, dermatitis impetigo, bullous pemphigoid, Churg-Strauss syndrome, acute myelogenous eosinophilic leukemia, acute lymphocytic eosinophilic leukemia, systemic mast cell disease with eosinophilia, eczema, Wegner's granulomatosis, polyarteritis nodosa, eosinophilic vasculitis, rheumatoid arthritis, Kawasaki disease, sickle cell disease, Churg-Strauss syndrome, Grave's disease, pre-eclampsia, Sjogren's syndrome, autoimmune lymphoproliferative syndrome, autoimmune hemolytic anemia, Barrett's esophagus, autoimmune uveitis, tuberculosis, nephropathy, herpes, chronic idiopathic urticaria, scleroderma, hypertrophic scarring, Whipple's disease, benign prostatic hyperplasia, mild or inflammatory bowel disease.

**16.** A composition for removing eosinophils comprising the bispecific antibody or antigen-binding fragment thereof according to any one of claims 1 to 8.

**17.** The composition according to claim 16, wherein the bispecific antibody engages T cells to induce cell lysis of eosinophils.

【Fig. 1】

| Valency (IL-5Rα:CD3ε) | 2:1 | | | | | | 1:1 | |
|---|---|---|---|---|---|---|---|---|
| Scheme (predicted MW) | 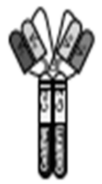 (102kDa) | 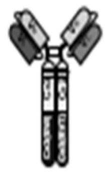 (105.3kDa) | 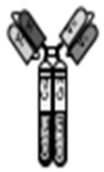 (105.3kDa) | 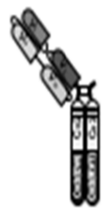 (105.6kDa) | 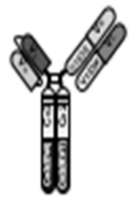 (126.2kDa) | 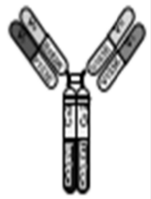 (147kDa) | 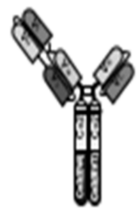 (132.1kDa) | 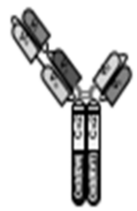 (130kDa) |
| Format name (IL-5Rα×CD3ε) | Diabody-Fc | scFv(L)/ scFv-Fc | scFv(H)/ scFv-Fc | BiTE-Fc | scFv/ Fab-Fc | IgG-like | BiTE(L)/ scFv-Fc | BiTE(H)/ scFv-Fc |
| Yield (mg/1L) | 23±5 | 7.8±1.3 | 3.1±1.7 | 32.3±8.1 | 29×4.4 | 32.5±5 | 6.2±2.1 | 7.2±2.7 |

【Fig. 2a】

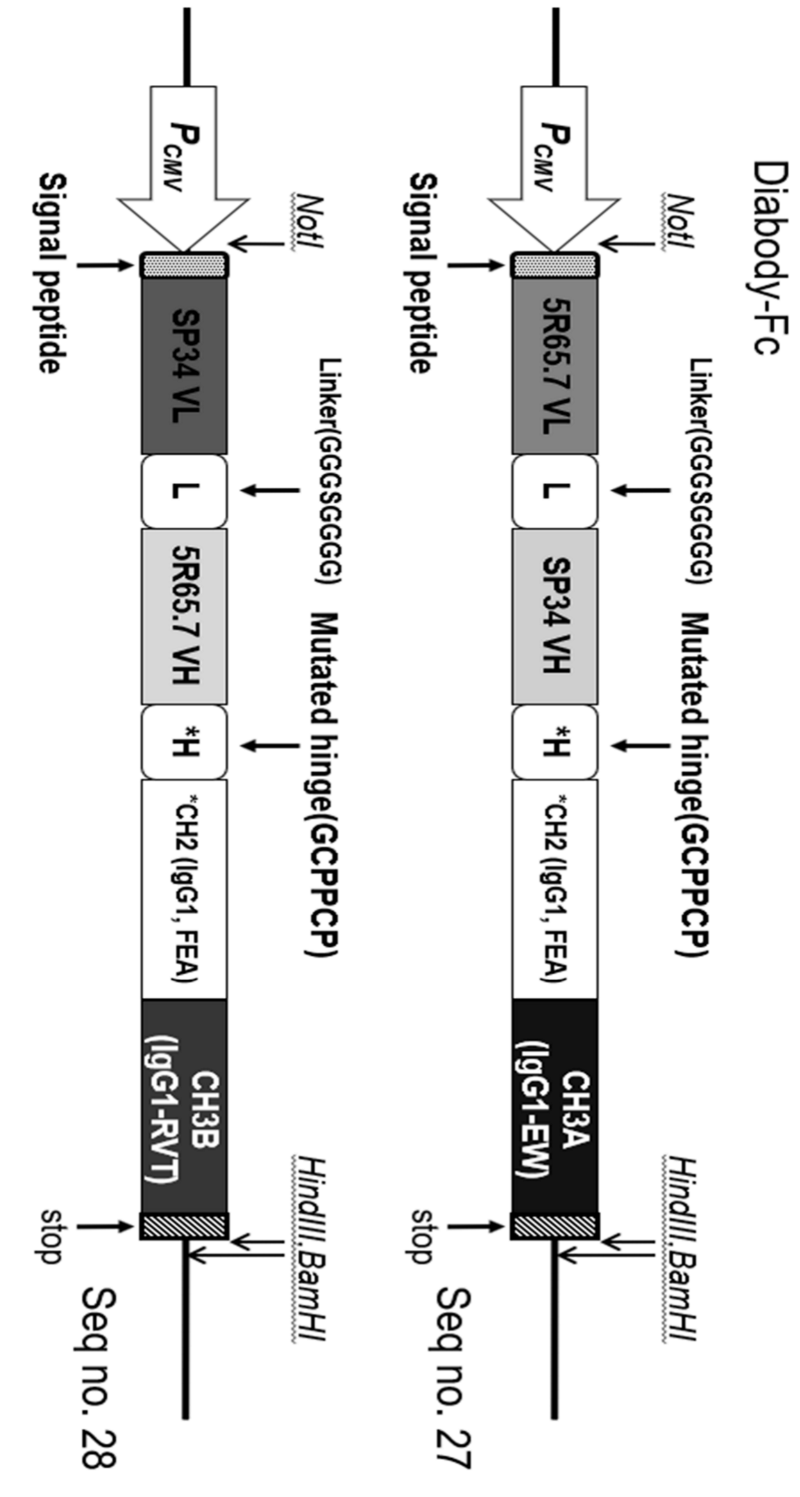
Diabody-Fc
PCMV
NotI
Signal peptide
5R65.7 VL
Linker(GGGSGGGG)
L
SP34 VH
Mutated hinge(GCPPCP)
*H
*CH2 (IgG1, FEA)
CH3A
(IgG1-EW)
HindIII,BamHI
stop
Seq no. 27
PCMV
NotI
Signal peptide
SP34 VL
Linker(GGGSGGGG)
L
5R65.7 VH
Mutated hinge(GCPPCP)
*H
*CH2 (IgG1, FEA)
CH3B
(IgG1-RVT)
HindIII,BamHI
stop
Seq no. 28

【Fig. 2b】

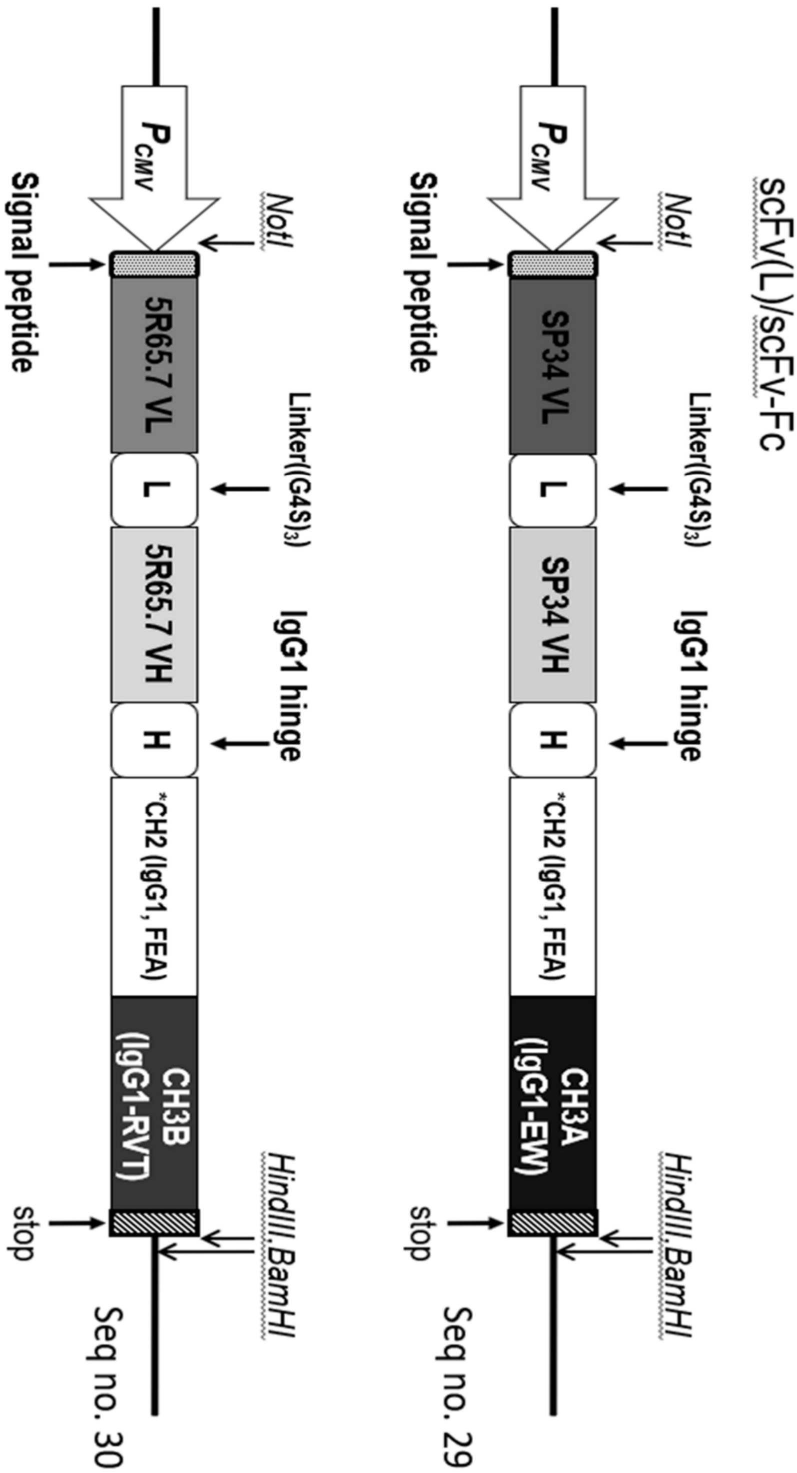
scFv(L)/scFv-Fc
NotI
PCMV
Signal peptide
SP34 VL
L
Linker((G4S)3)
SP34 VH
IgG1 hinge
H
*CH2 (IgG1, FEA)
CH3A
(IgG1-EW)
HindIII,BamHI
stop
Seq no. 29
NotI
PCMV
Signal peptide
5R65.7 VL
L
Linker((G4S)3)
5R65.7 VH
IgG1 hinge
H
*CH2 (IgG1, FEA)
CH3B
(IgG1-RVT)
HindIII,BamHI
stop
Seq no. 30

【Fig. 2c】

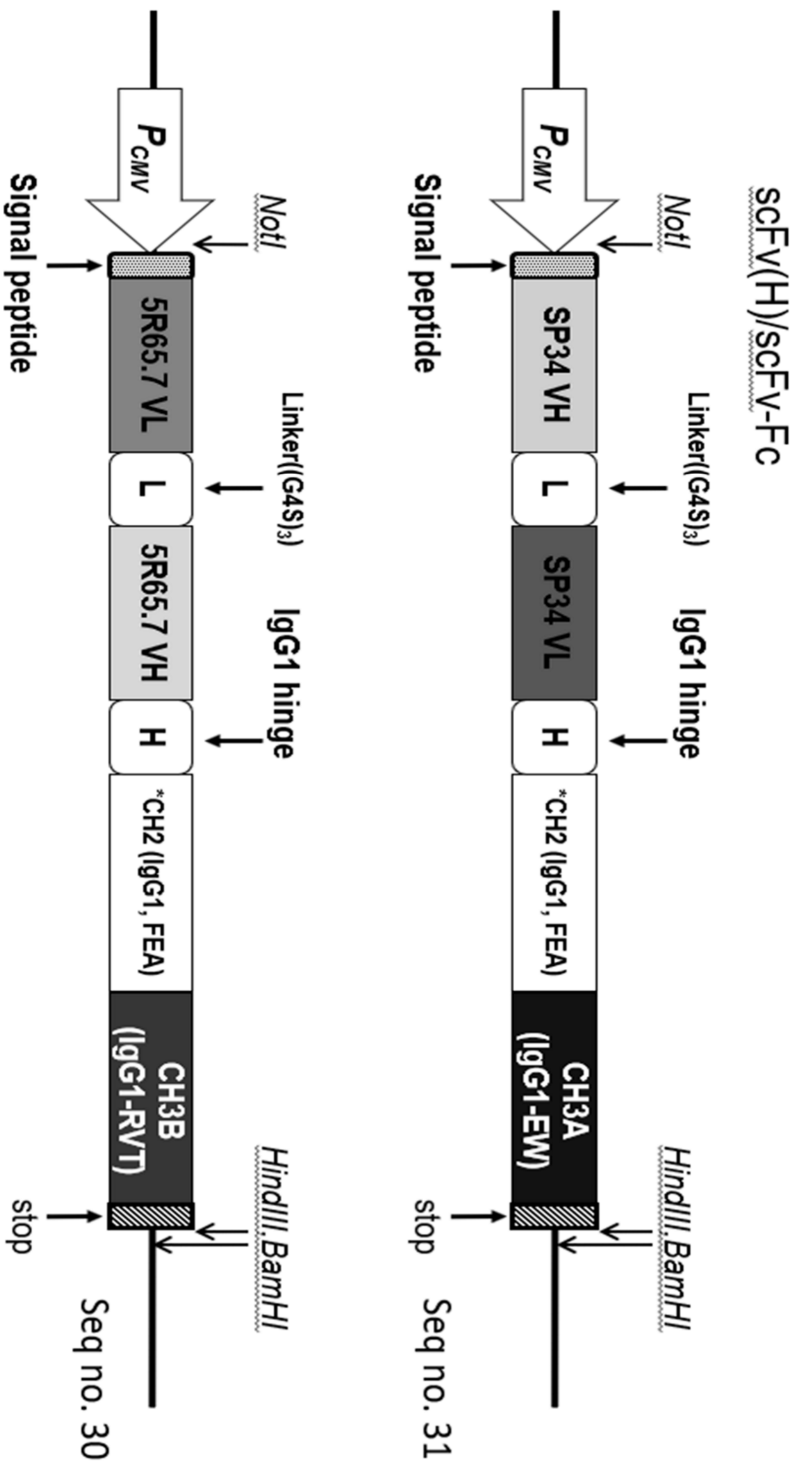
scFv(H)/scFv-Fc
PCMV
NotI
Signal peptide
SP34 VH
Linker((G4S)3)
L
SP34 VL
IgG1 hinge
H
*CH2 (IgG1, FEA)
CH3A (IgG1-EW)
HindIII,BamHI
stop
Seq no. 31
5R65.7 VL
5R65.7 VH
CH3B (IgG1-RVT)
Seq no. 30

【Fig. 2d】

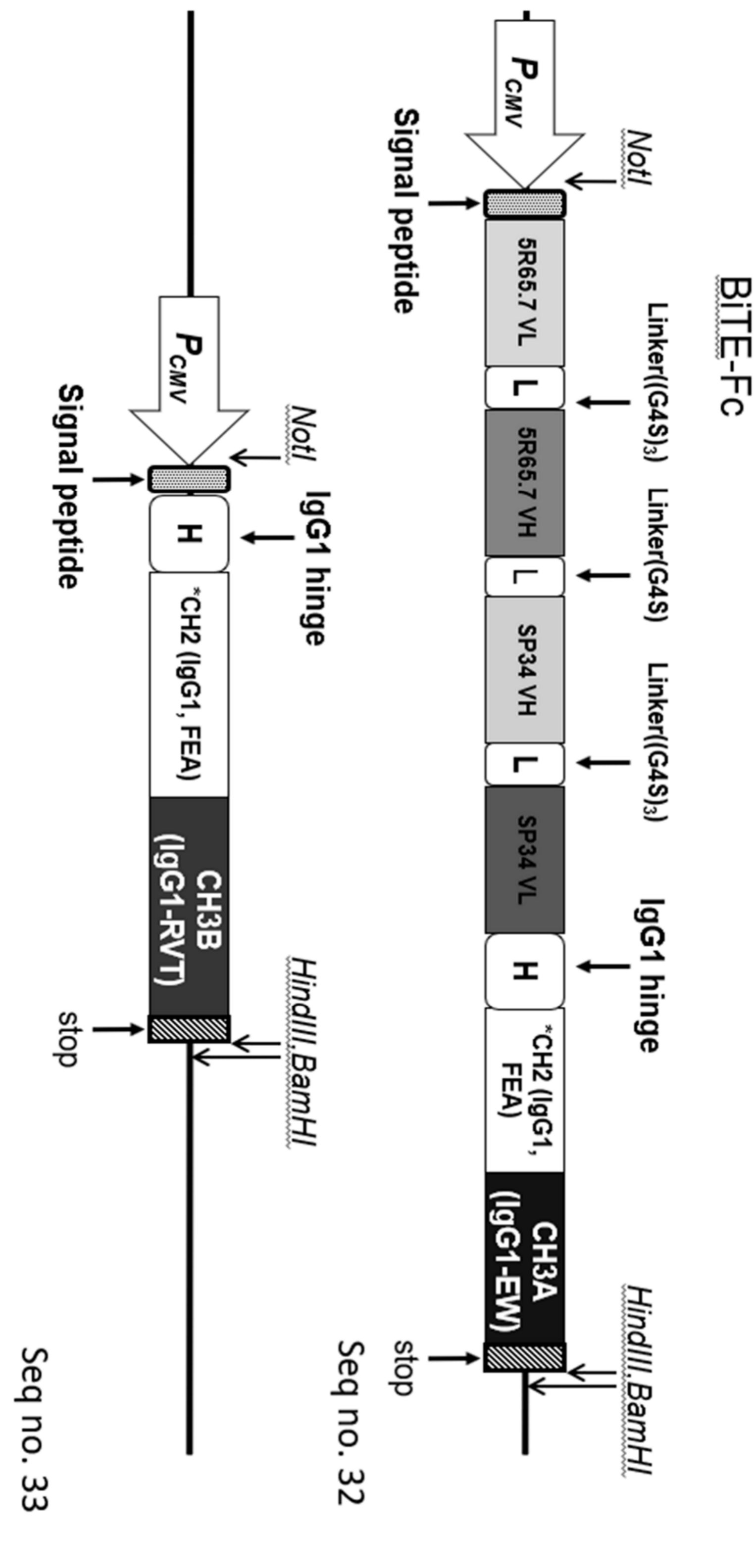
BiTE-Fc
NotI
$P_{CMV}$
Signal peptide
5R65.7 VL
L
Linker((G4S)$_3$)
5R65.7 VH
L
Linker(G4S)
SP34 VH
L
Linker((G4S)$_3$)
SP34 VL
H
IgG1 hinge
*CH2 (IgG1, FEA)
CH3A (IgG1-EW)
stop
HindIII,BamHI
Seq no. 32
$P_{CMV}$
NotI
Signal peptide
H
IgG1 hinge
*CH2 (IgG1, FEA)
CH3B (IgG1-RVT)
stop
HindIII,BamHI
Seq no. 33

【Fig. 2e】

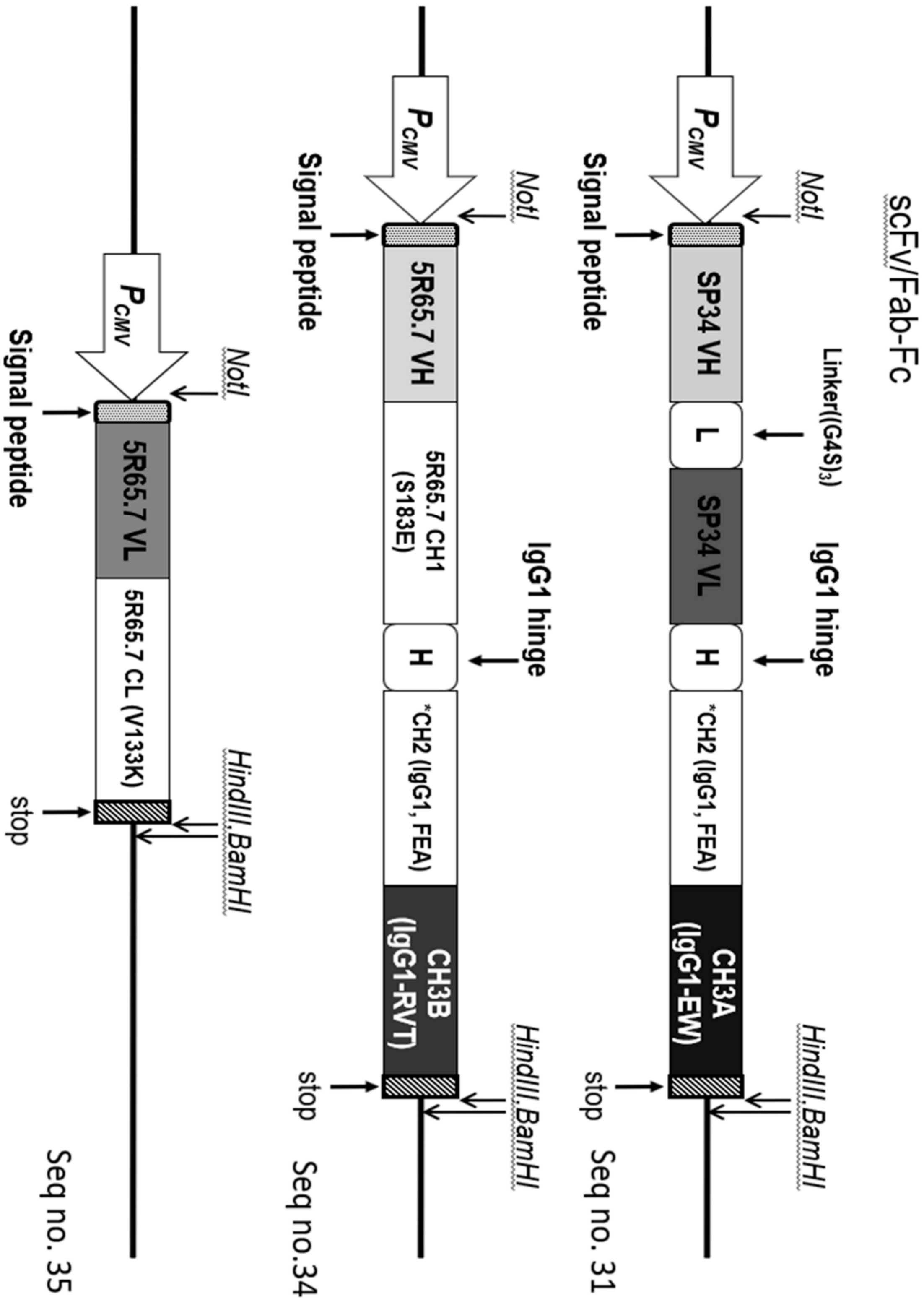
scFv/Fab-Fc
$P_{CMV}$
NotI
Signal peptide
SP34 VH
Linker((G4S)$_3$)
L
SP34 VL
IgG1 hinge
H
*CH2 (IgG1, FEA)
CH3A (IgG1-EW)
stop
HindIII,BamHI
Seq no. 31
$P_{CMV}$
NotI
Signal peptide
5R65.7 VH
5R65.7 CH1 (S183E)
IgG1 hinge
H
*CH2 (IgG1, FEA)
CH3B (IgG1-RVT)
stop
HindIII,BamHI
Seq no.34
$P_{CMV}$
NotI
Signal peptide
5R65.7 VL
5R65.7 CL (V133K)
stop
HindIII,BamHI
Seq no. 35

【Fig. 2f】

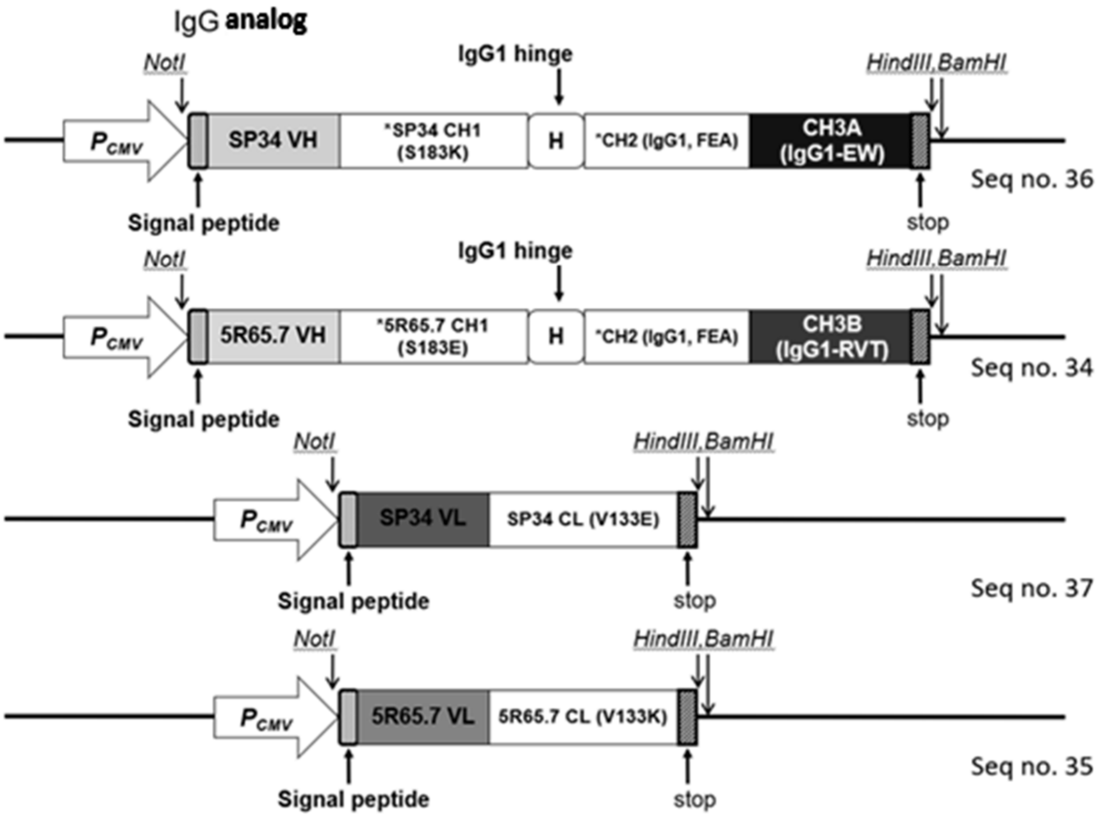
IgG analog
NotI
IgG1 hinge
HindIII,BamHI
PCMV
SP34 VH
*SP34 CH1 (S183K)
H
*CH2 (IgG1, FEA)
CH3A (IgG1-EW)
Signal peptide
stop
Seq no. 36
5R65.7 VH
*5R65.7 CH1 (S183E)
CH3B (IgG1-RVT)
Seq no. 34
SP34 VL
SP34 CL (V133E)
Seq no. 37
5R65.7 VL
5R65.7 CL (V133K)
Seq no. 35

【Fig. 2g】

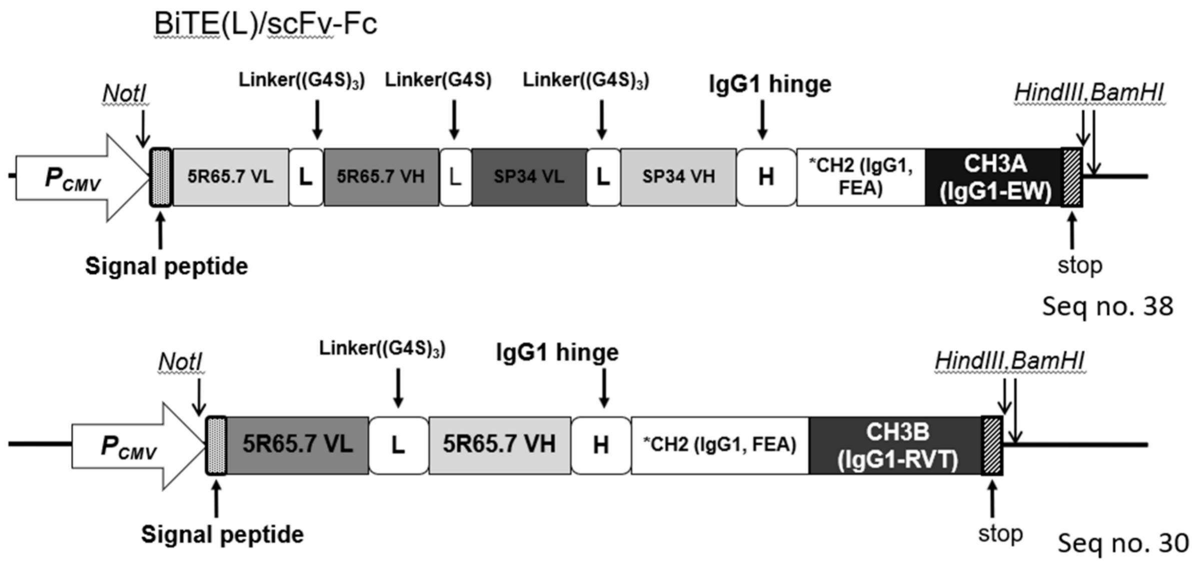
BiTE(L)/scFv-Fc
NotI
Linker((G4S)3)
Linker(G4S)
IgG1 hinge
HindIII,BamHI
PCMV
5R65.7 VL
L
5R65.7 VH
SP34 VL
SP34 VH
H
*CH2 (IgG1, FEA)
CH3A (IgG1-EW)
Signal peptide
stop
Seq no. 38
CH3B (IgG1-RVT)
Seq no. 30

【Fig. 2h】

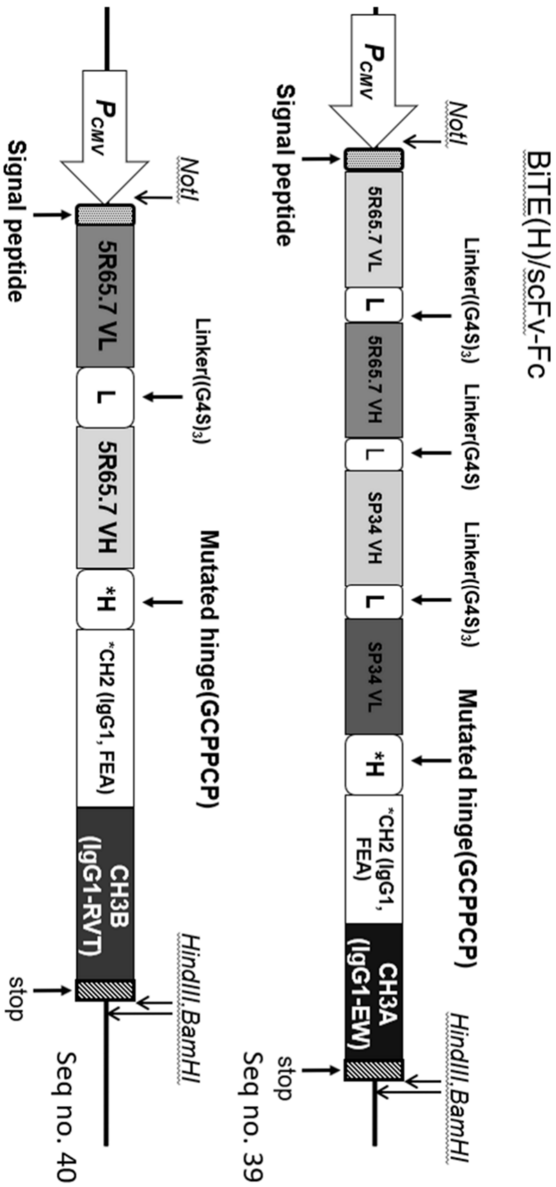
BiTE(H)/scFv-Fc
PCMV
NotI
Signal peptide
5R65.7 VL
Linker((G4S)3)
L
5R65.7 VH
Linker(G4S)
L
SP34 VH
Linker((G4S)3)
L
SP34 VL
Mutated hinge(GCPPCP)
*H
*CH2 (IgG1, FEA)
CH3A (IgG1-EW)
HindIII,BamHI
stop
Seq no. 39
PCMV
NotI
Signal peptide
5R65.7 VL
Linker((G4S)3)
L
5R65.7 VH
Mutated hinge(GCPPCP)
*H
*CH2 (IgG1, FEA)
CH3B (IgG1-RVT)
HindIII,BamHI
stop
Seq no. 40

【Fig. 2i】

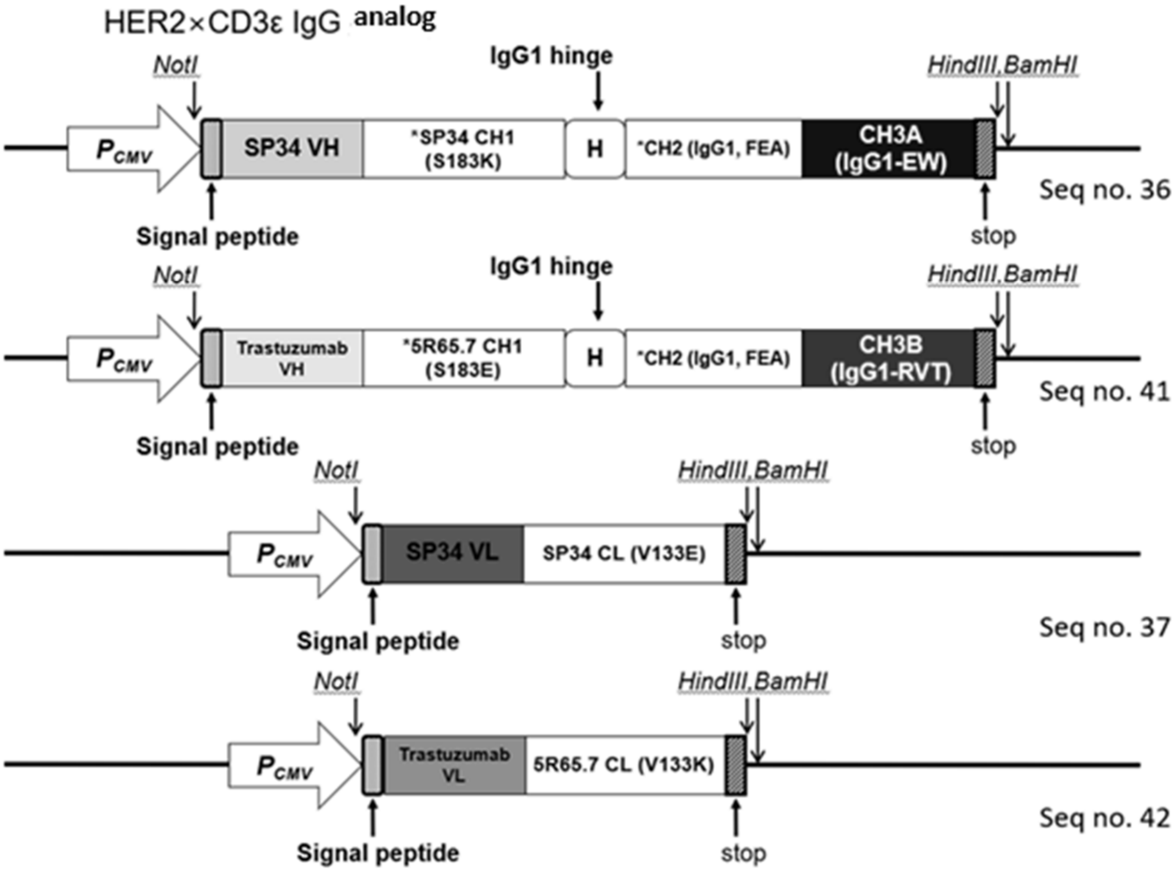
HER2×CD3ε IgG analog
NotI
IgG1 hinge
HindIII,BamHI
PCMV
SP34 VH
*SP34 CH1 (S183K)
H
*CH2 (IgG1, FEA)
CH3A (IgG1-EW)
Seq no. 36
Signal peptide
stop
Trastuzumab VH
*5R65.7 CH1 (S183E)
CH3B (IgG1-RVT)
Seq no. 41
SP34 VL
SP34 CL (V133E)
Seq no. 37
Trastuzumab VL
5R65.7 CL (V133K)
Seq no. 42

【Fig. 3a】

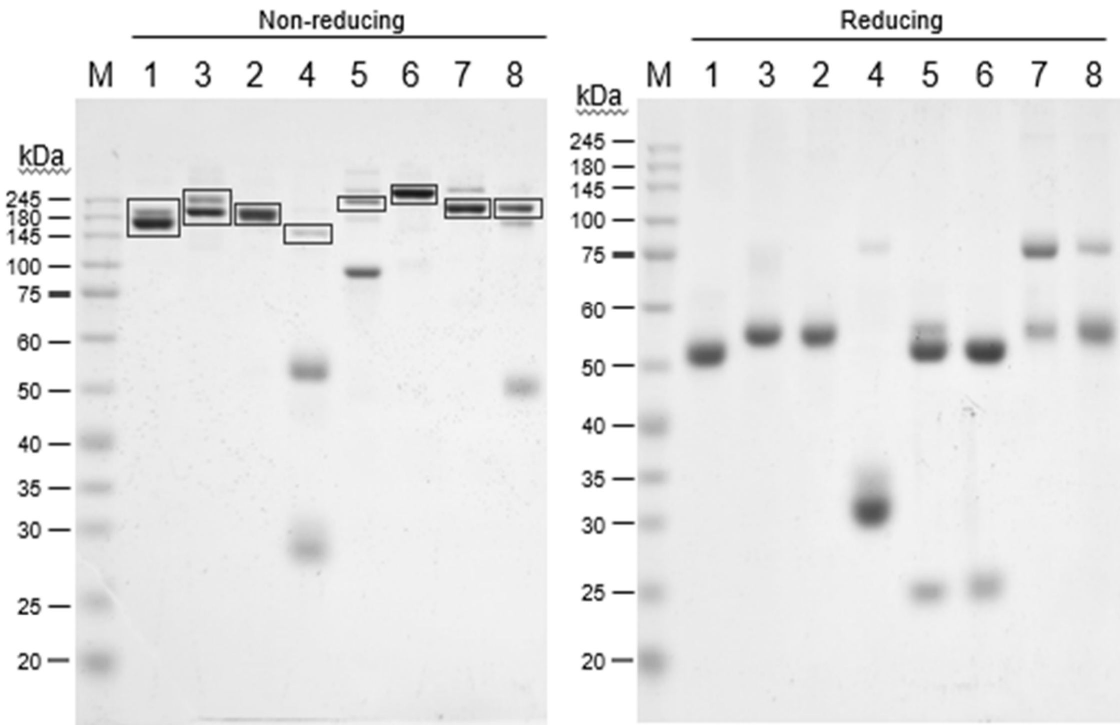


| Lane Number | Format name (IL-5Rα×CD3ε) |
|---|---|
| 1 | Diabody-Fc |
| 2 | scFv(L)/scFv-Fc |
| 3 | scFv(H)/scFv-Fc |
| 4 | BiTE-Fc |
| 5 | Fab/scFv-Fc |
| 6 | IgG like |
| 7 | BiTE(L)/scFv-Fc |
| 8 | BiTE(H)/scFv-Fc |

【Fig. 3b】

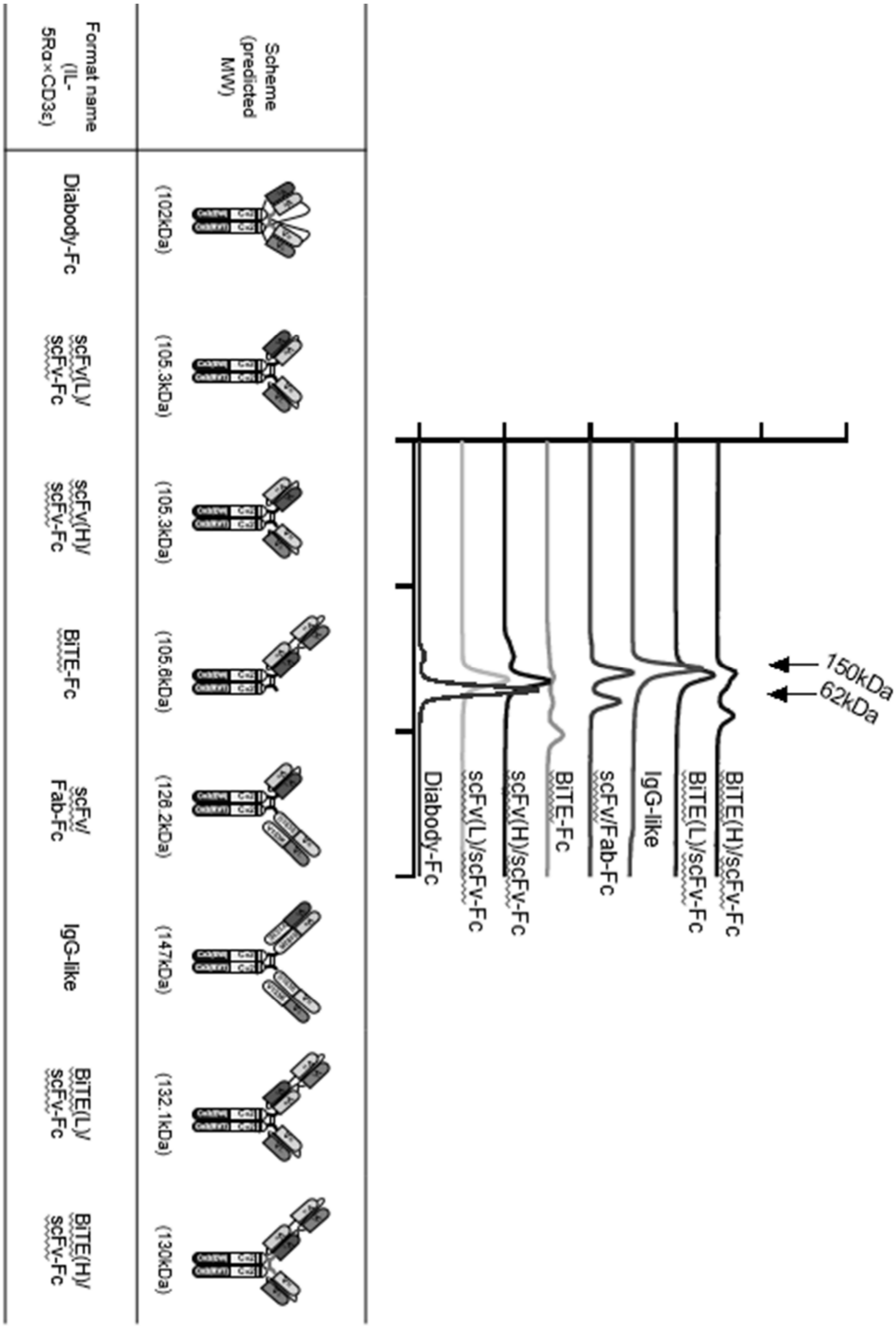
Format name (IL-5Rα×CD3ε)
Scheme (predicted MW)
Diabody-Fc
(102kDa)
scFv(L)/scFv-Fc
(105.3kDa)
scFv(H)/scFv-Fc
(105.3kDa)
BiTE-Fc
(105.6kDa)
scFv/Fab-Fc
(126.2kDa)
IgG-like
(147kDa)
BiTE(L)/scFv-Fc
(132.1kDa)
BiTE(H)/scFv-Fc
(130kDa)
150kDa
62kDa
Diabody-Fc
scFv(L)/scFv-Fc
scFv(H)/scFv-Fc
BiTE-Fc
scFv/Fab-Fc
IgG-like
BiTE(L)/scFv-Fc
BiTE(H)/scFv-Fc

【Fig. 4】

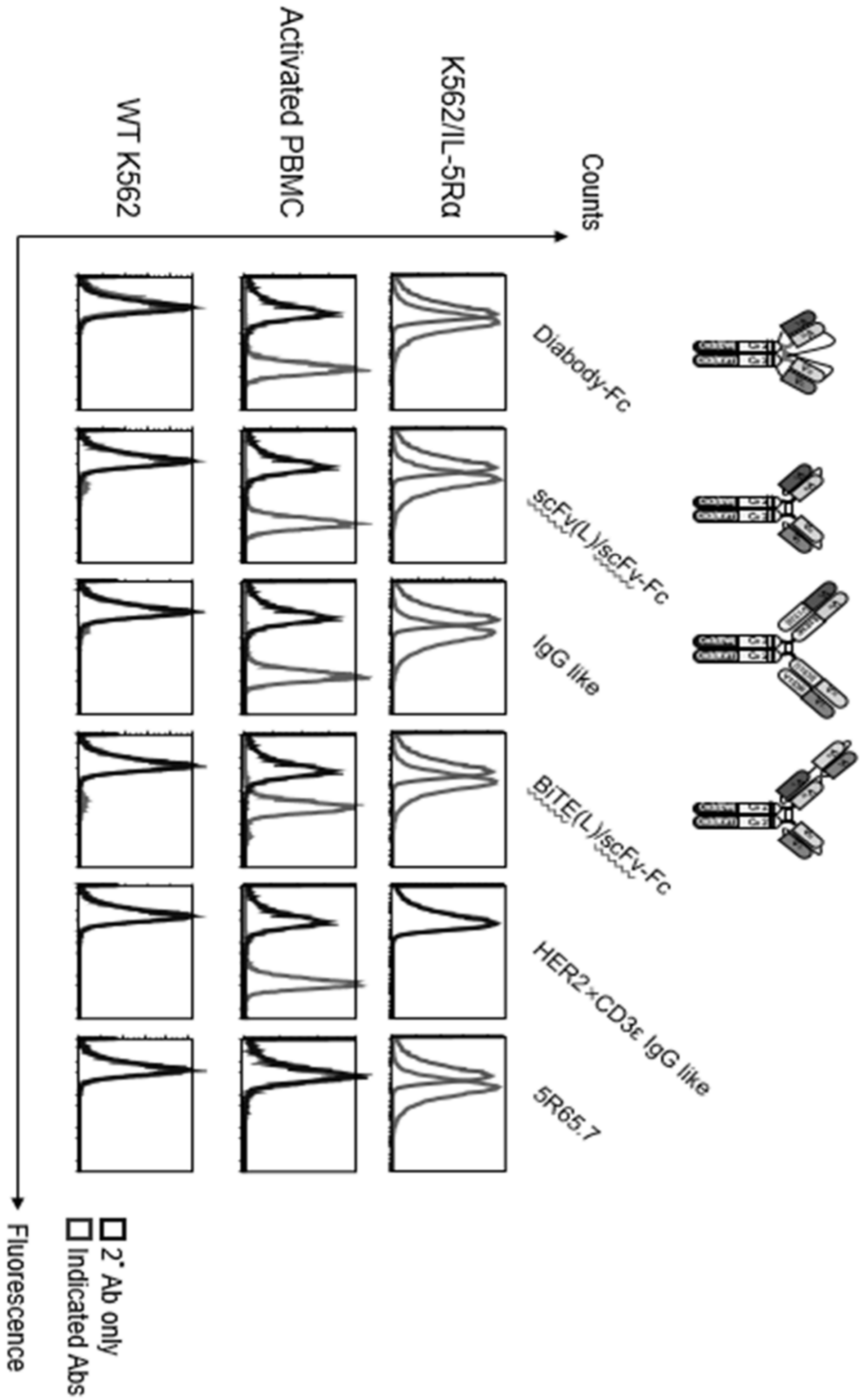
Counts
K562/IL-5Rα
Activated PBMC
WT K562
Diabody-Fc
scFv(L)/scFv-Fc
IgG like
BiTE(L)/scFv-Fc
HER2×CD3ε IgG like
5R65.7
2° Ab only
Indicated Abs
Fluorescence

【Fig. 5a】

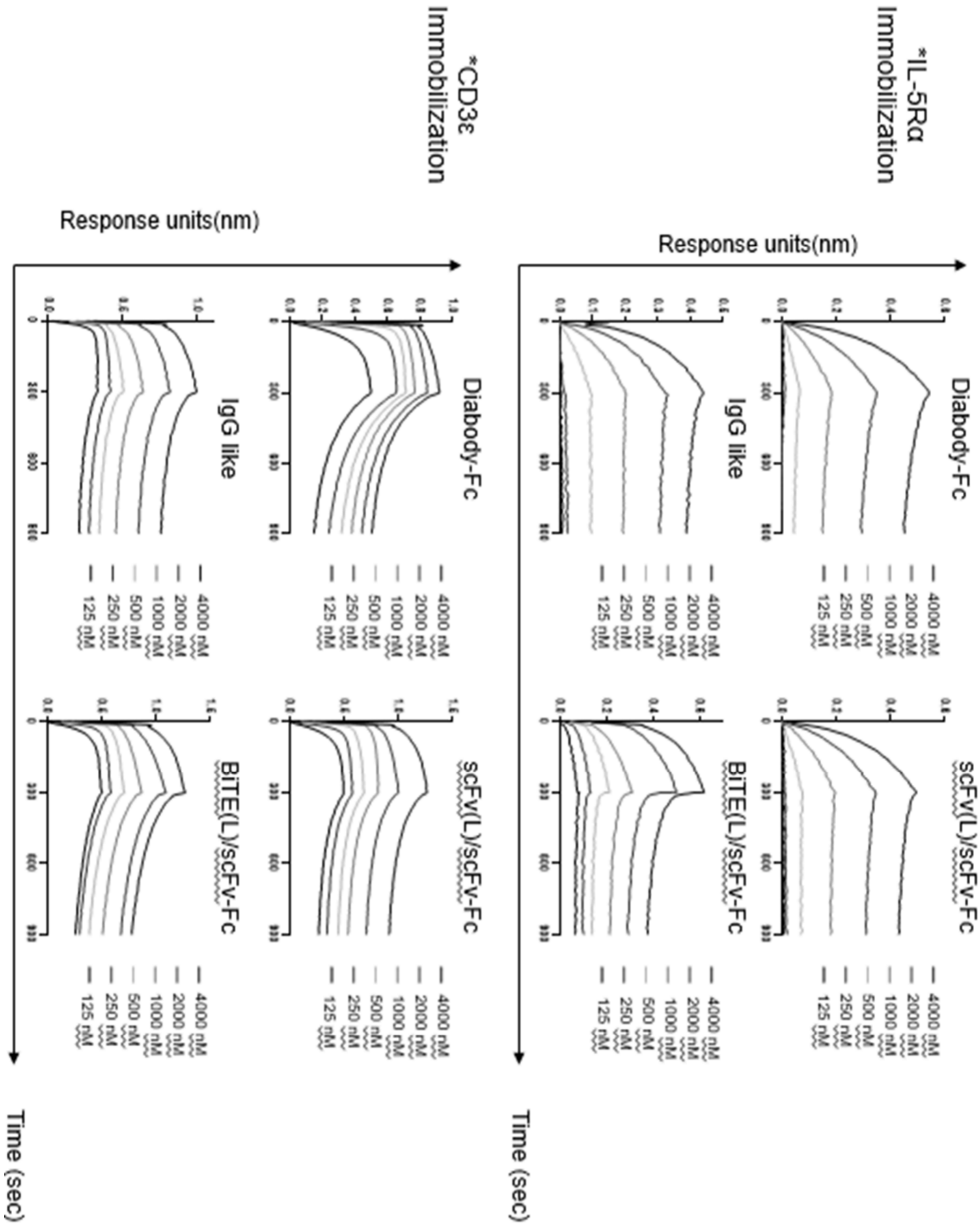
*IL-5Rα Immobilization
*CD3ε Immobilization
Response units(nm)
Time (sec)
Diabody-Fc
IgG like
scFv(L)/scFv-Fc
BiTE(L)/scFv-Fc
4000 nM
2000 nM
1000 nM
500 nM
250 nM
125 nM

【Fig. 5b】

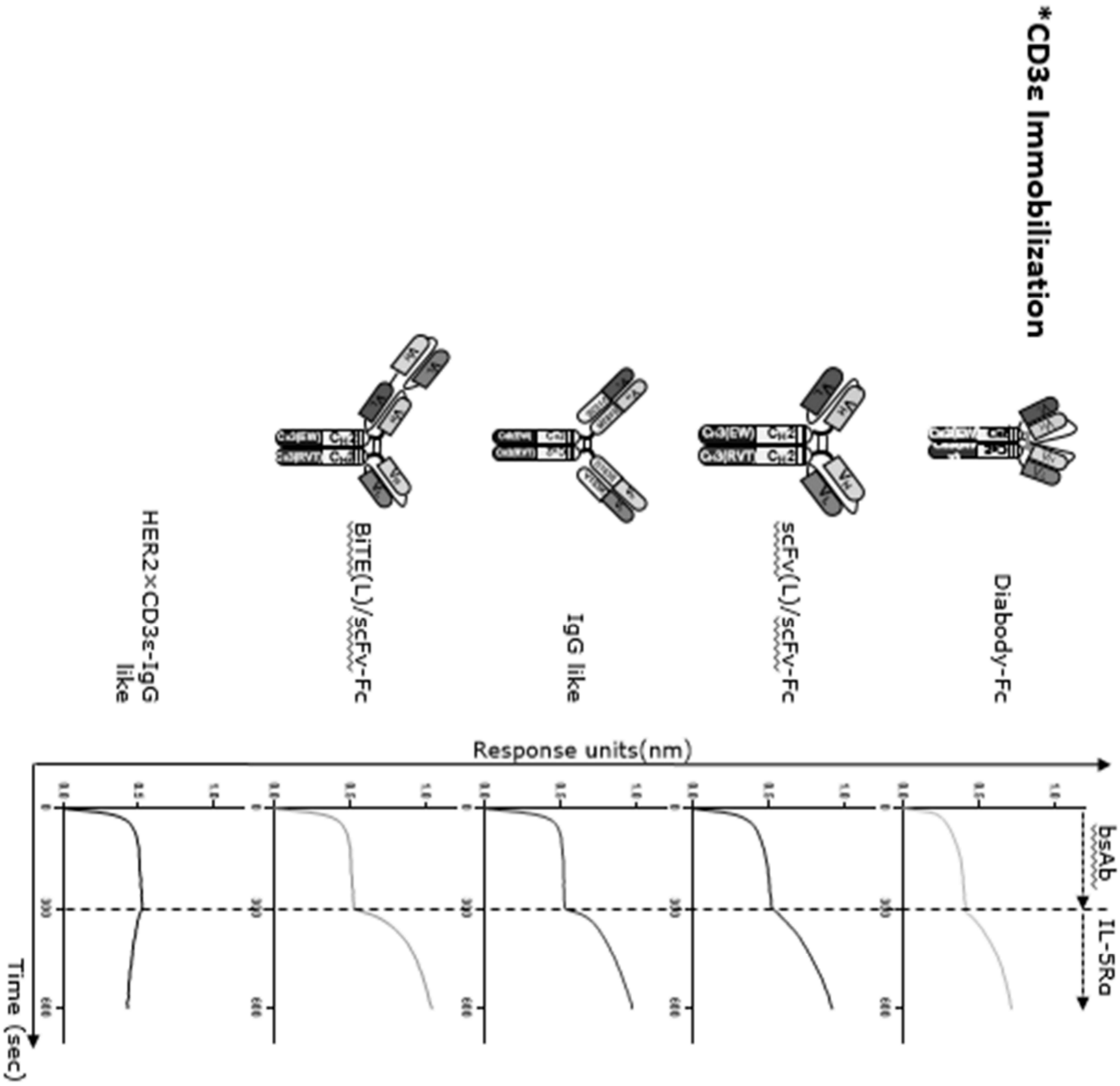
*CD3ε Immobilization
Diabody-Fc
scFv(L)/scFv-Fc
IgG like
BiTE(L)/scFv-Fc
HER2×CD3ε-IgG like
Response units(nm)
bsAb
IL-5Rα
Time (sec)

【Fig. 6a】

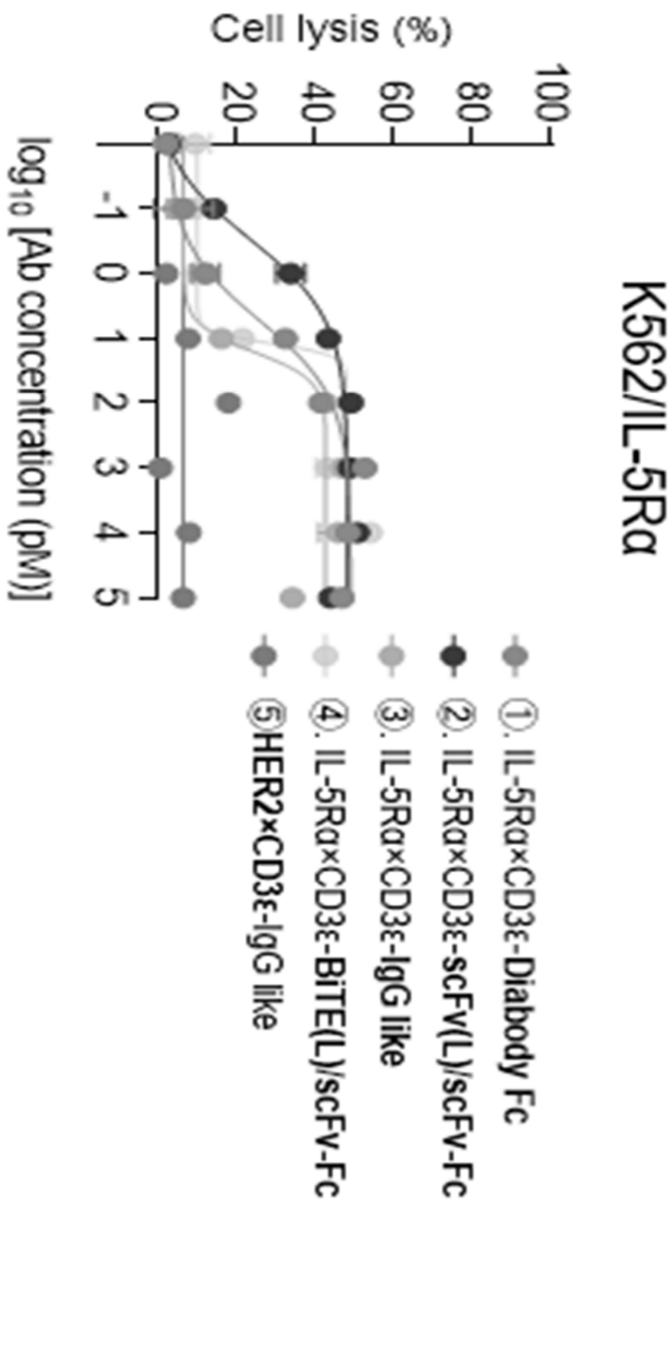
K562/IL-5Rα
Cell lysis (%)
0
20
40
60
80
100
-1
0
1
2
3
4
5
log₁₀ [Ab concentration (pM)]
①. IL-5Rα×CD3ε-Diabody Fc
②. IL-5Rα×CD3ε-scFv(L)/scFv-Fc
③. IL-5Rα×CD3ε-IgG like
④. IL-5Rα×CD3ε-BiTE(L)/scFv-Fc
⑤HER2×CD3ε-IgG like

【Fig. 6b】

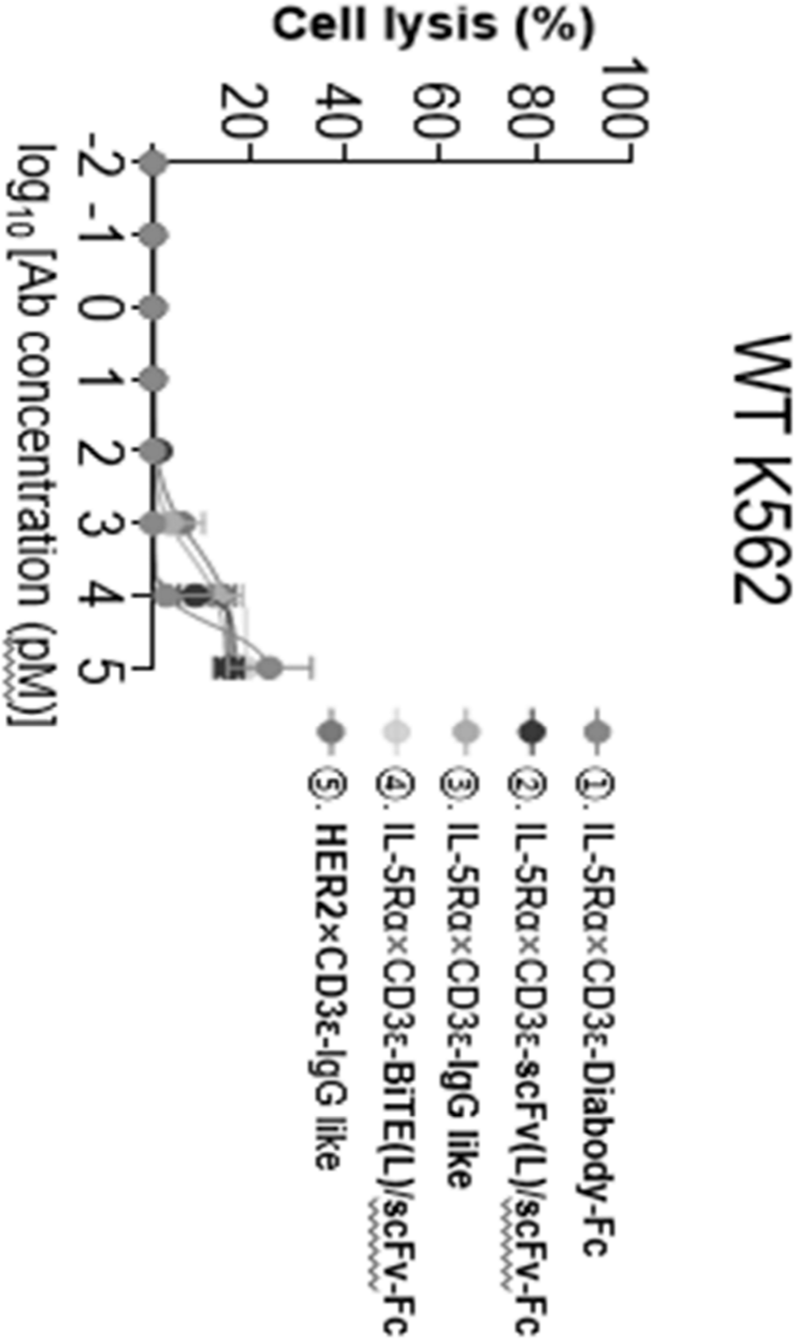
WT K562
Cell lysis (%)
100
80
60
40
20
-2
-1
0
1
2
3
4
5
log10 [Ab concentration (pM)]
①. IL-5Rα×CD3ε-Diabody-Fc
②. IL-5Rα×CD3ε-scFv(L)/scFv-Fc
③. IL-5Rα×CD3ε-IgG like
④. IL-5Rα×CD3ε-BiTE(L)/scFv-Fc
⑤. HER2×CD3ε-IgG like

【Fig. 7】

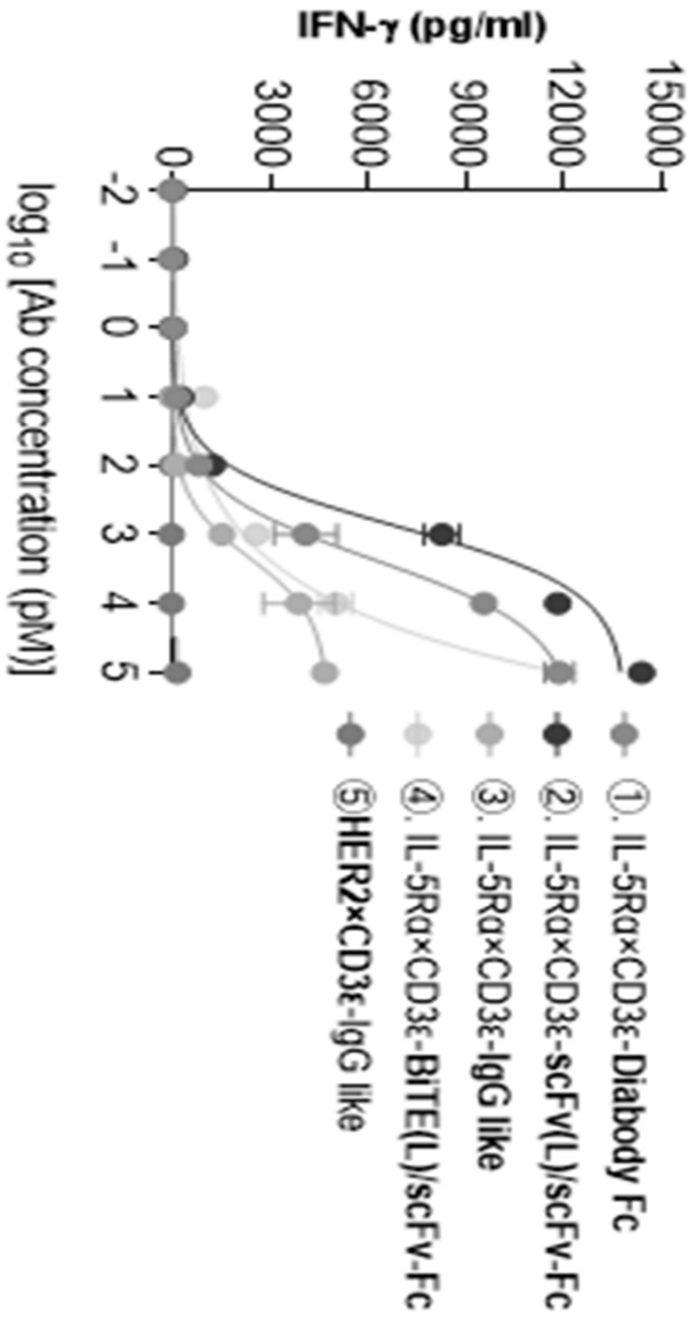
IFN-γ (pg/ml)
15000
12000
9000
6000
3000
0
-2
-1
0
1
2
3
4
5
log10 [Ab concentration (pM)]
①. IL-5Rα×CD3ε-Diabody Fc
②. IL-5Rα×CD3ε-scFv(L)/scFv-Fc
③. IL-5Rα×CD3ε-IgG like
④. IL-5Rα×CD3ε-BiTE(L)/scFv-Fc
⑤HER2×CD3ε-IgG like

【Fig. 8】

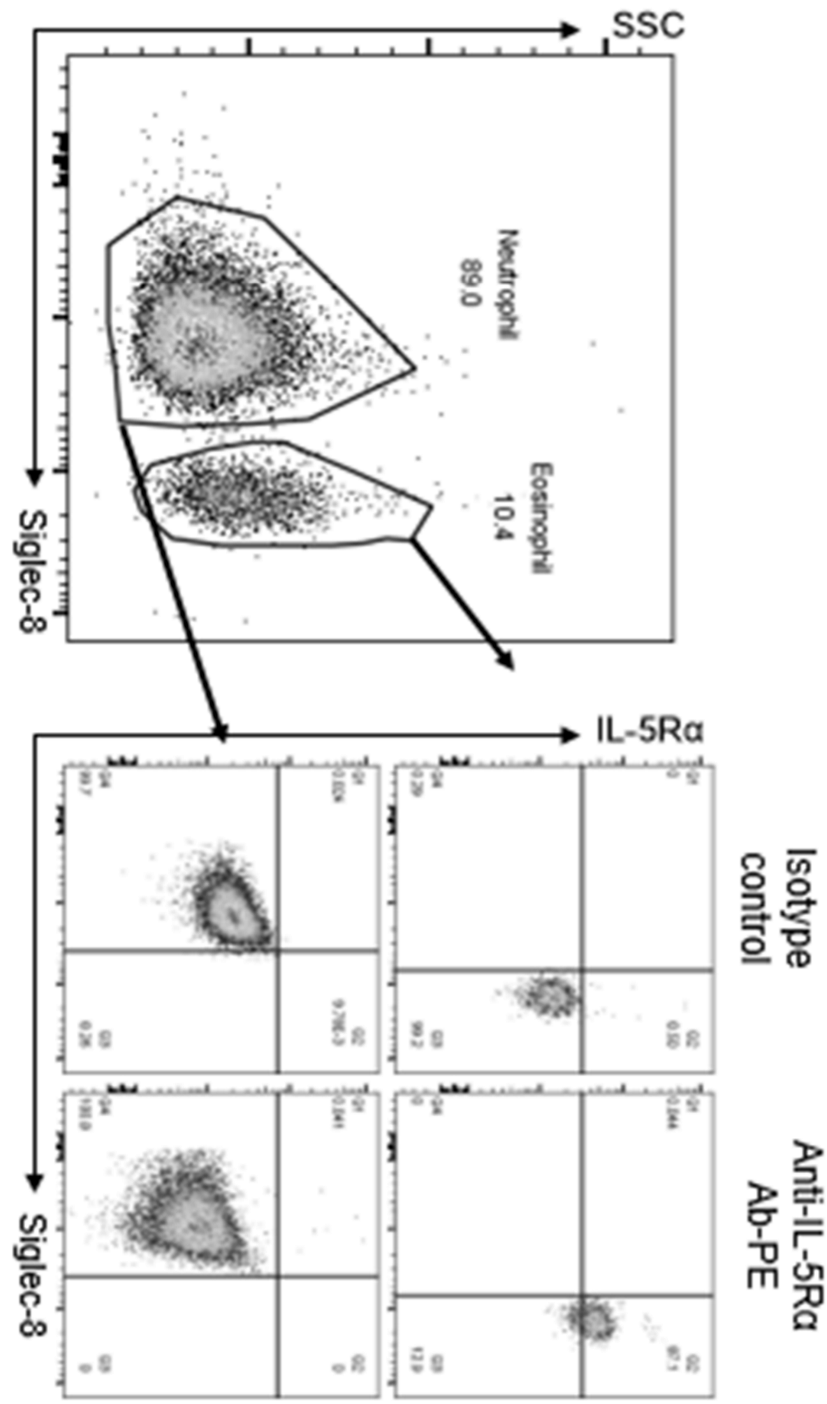
SSC
Siglec-8
Neutrophil
89.0
Eosinophil
10.4
IL-5Rα
Siglec-8
Isotype control
Anti-IL-5Rα Ab-PE

【Fig. 9】

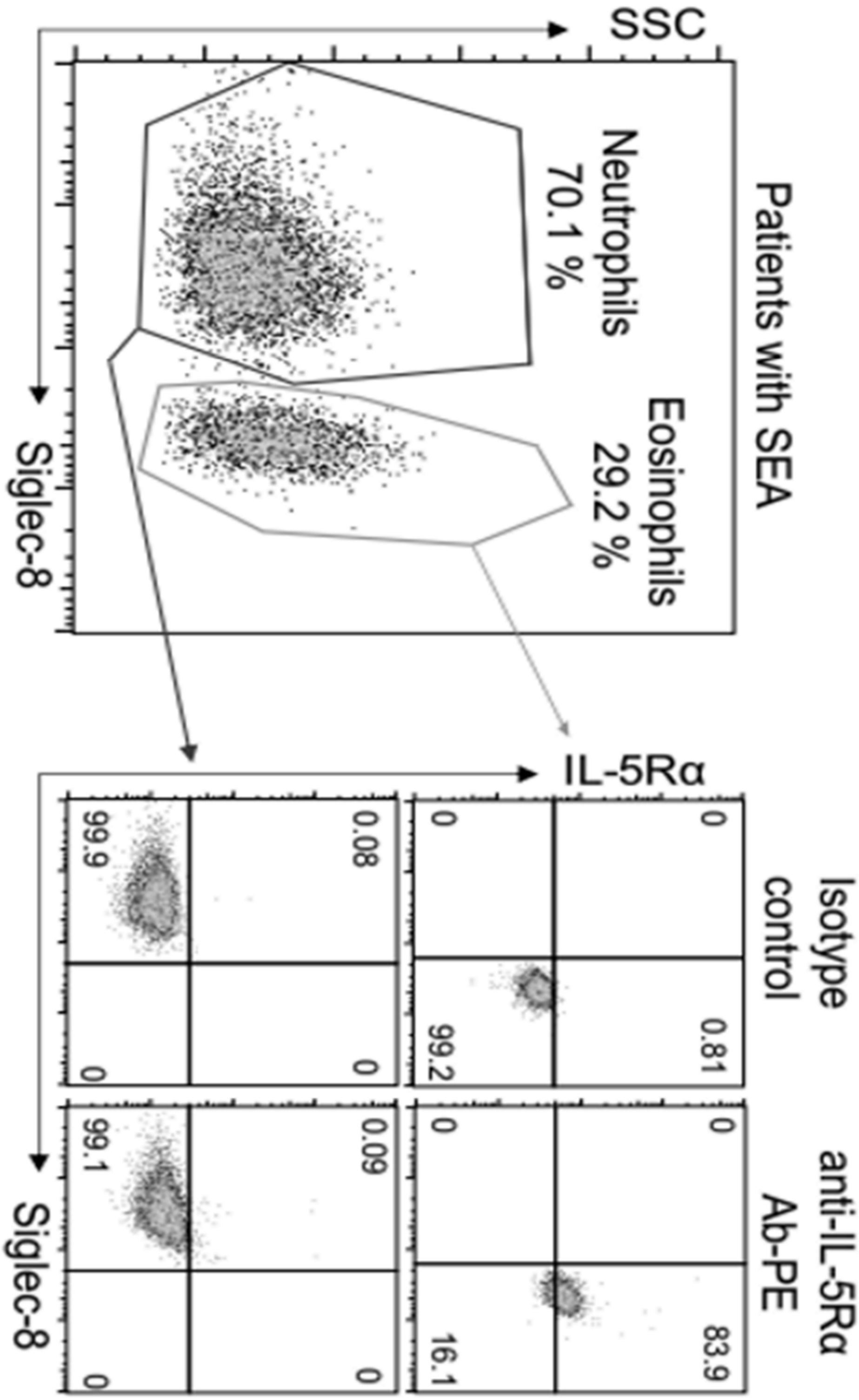
Patients with SEA
SSC
Siglec-8
Neutrophils
70.1 %
Eosinophils
29.2 %
IL-5Rα
Isotype
control
anti-IL-5Rα
Ab-PE
99.9
0.08
0
0
0
0
99.2
0.81
99.1
0.09
0
0
0
0
16.1
83.9
Siglec-8

【Fig. 10】

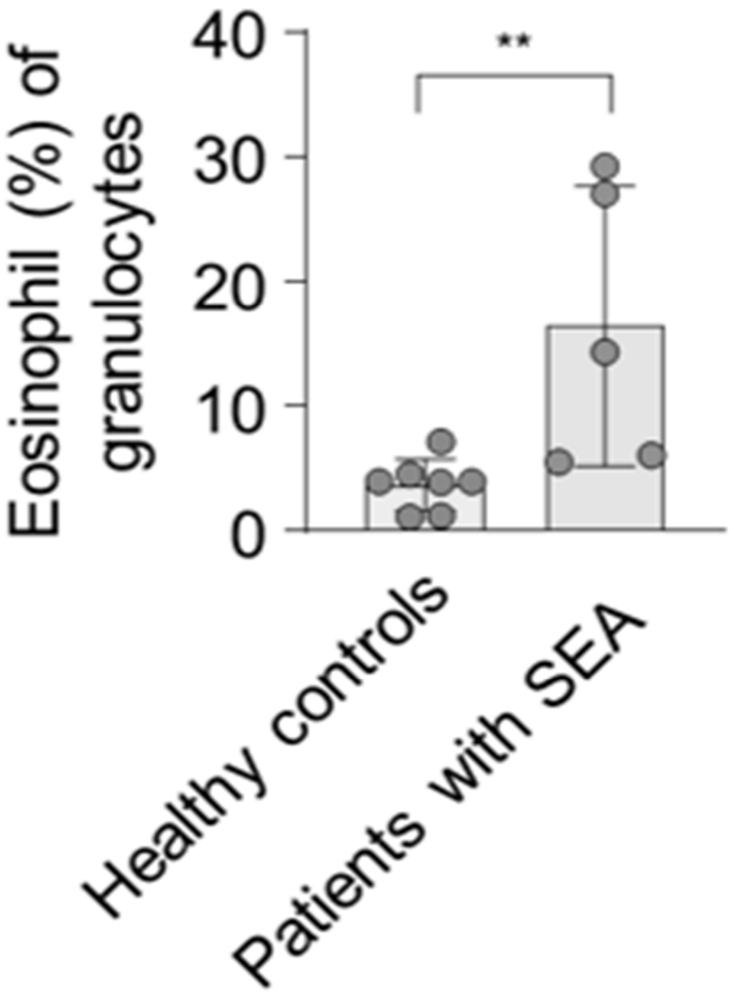
Eosinophil (%) of granulocytes
40
30
20
10
0
**
Healthy controls
Patients with SEA

【Fig. 11a】

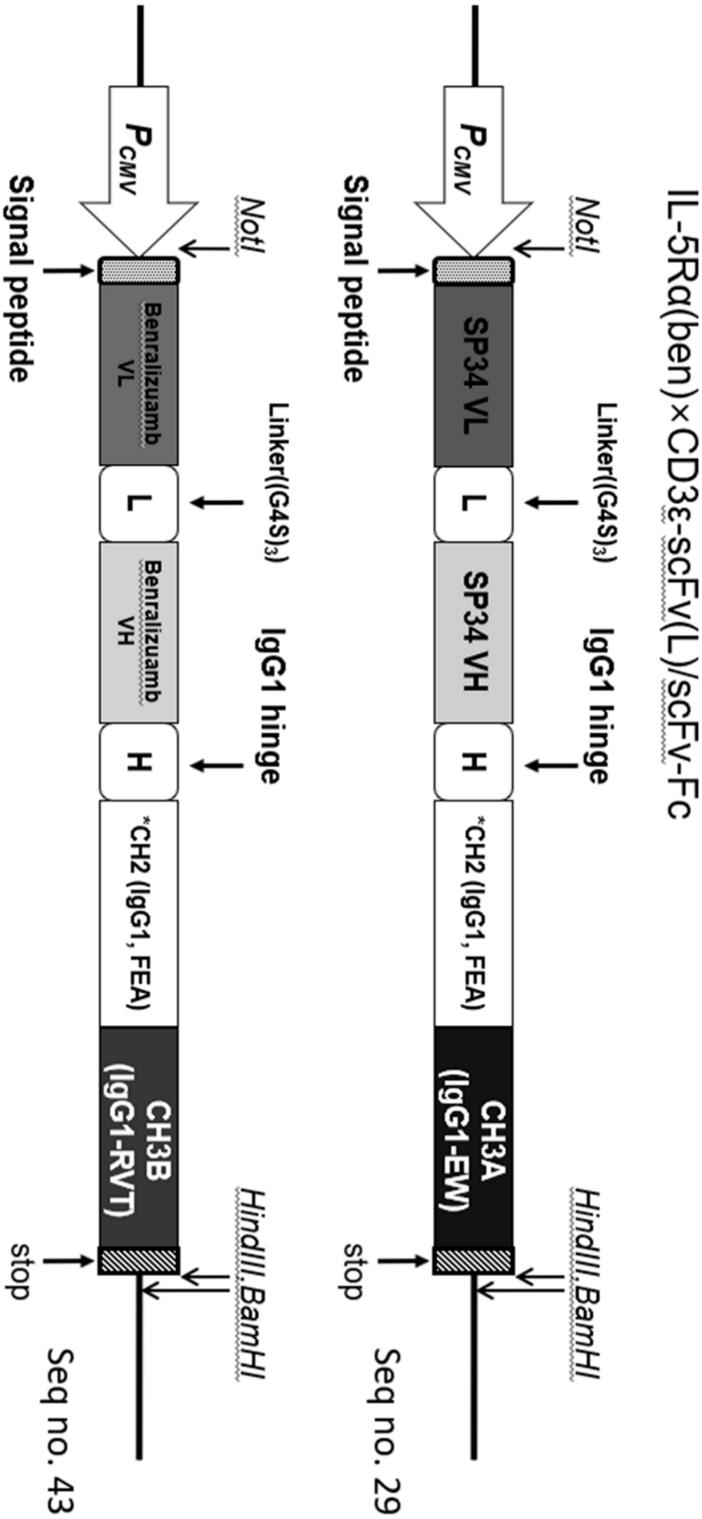
IL-5Rα(ben)×CD3ε-scFv(L)/scFv-Fc
PCMV
NotI
Signal peptide
SP34 VL
Linker((G4S)3)
L
SP34 VH
IgG1 hinge
H
*CH2 (IgG1, FEA)
CH3A (IgG1-EW)
HindIII,BamHI
stop
Seq no. 29
PCMV
NotI
Signal peptide
Benralizuamb VL
Linker((G4S)3)
L
Benralizuamb VH
IgG1 hinge
H
*CH2 (IgG1, FEA)
CH3B (IgG1-RVT)
HindIII,BamHI
stop
Seq no. 43

【Fig. 11b】

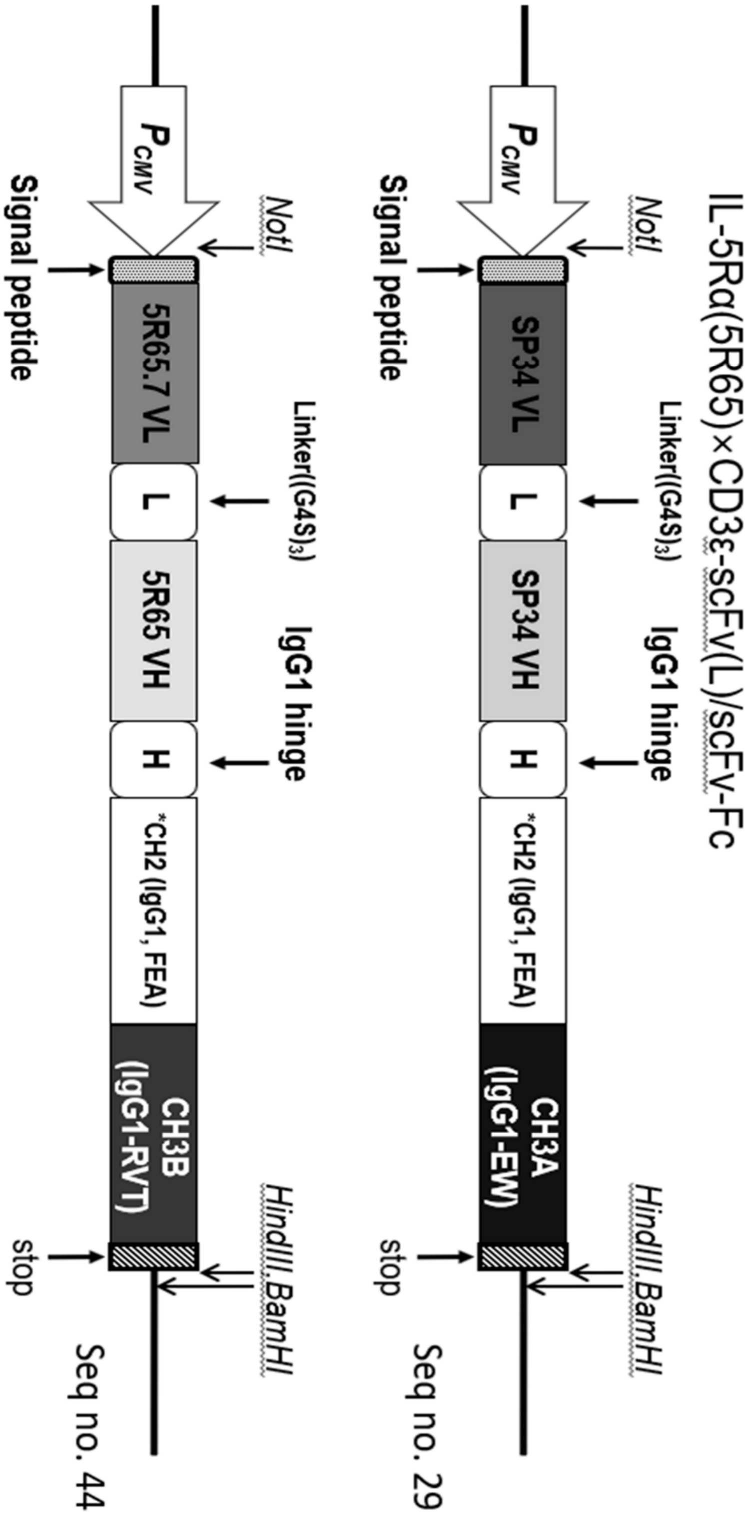
IL-5Rα(5R65)×CD3ε-scFv(L)/scFv-Fc
PCMV
NotI
Signal peptide
SP34 VL
L
Linker((G4S)3)
SP34 VH
IgG1 hinge
H
*CH2 (IgG1, FEA)
CH3A (IgG1-EW)
HindIII,BamHI
stop
Seq no. 29
PCMV
NotI
Signal peptide
5R65.7 VL
L
Linker((G4S)3)
5R65 VH
IgG1 hinge
H
*CH2 (IgG1, FEA)
CH3B (IgG1-RVT)
HindIII,BamHI
stop
Seq no. 44

【Fig. 11c】

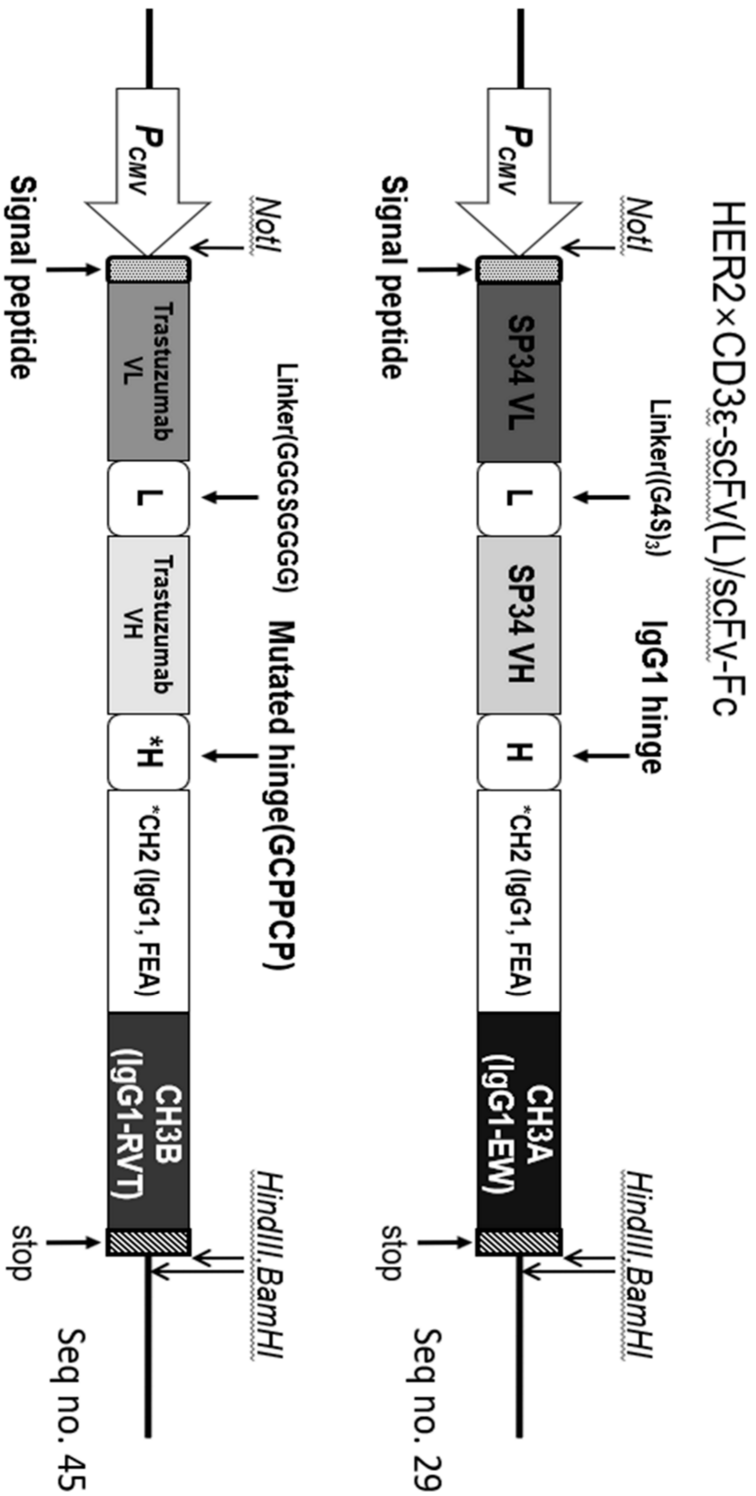
HER2×CD3ε-scFv(L)/scFv-Fc
NotI
$P_{CMV}$
Signal peptide
SP34 VL
Linker((G4S)3)
L
SP34 VH
IgG1 hinge
H
*CH2 (IgG1, FEA)
CH3A (IgG1-EW)
HindIII, BamHI
stop
Seq no. 29
NotI
$P_{CMV}$
Signal peptide
Trastuzumab VL
Linker(GGGSGGGG)
L
Trastuzumab VH
Mutated hinge(GCPPCP)
*H
*CH2 (IgG1, FEA)
CH3B (IgG1-RVT)
HindIII, BamHI
stop
Seq no. 45

【Fig. 12a】

| Scheme (predicted MW) | 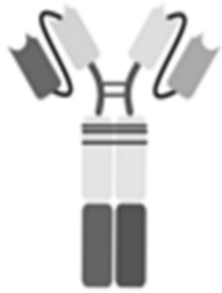 (104.8 kDa) | 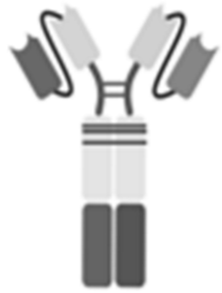 (105.3 kDa) | 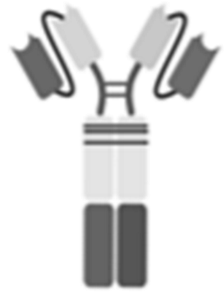 (104.4 kDa) |
|---|---|---|---|
| clone name | IL-5Rα(ben)×CD3ε -scFv(L)/scFv-Fc | IL-5Rα(5R65)×CD3ε -scFv(L)/scFv-Fc | HER2×CD3ε -scFv(L)/scFv-Fc |
| Yield (mg/1L) | 17+4.8 | 18+2.4 | 44+6.6 |

【Fig. 12b】

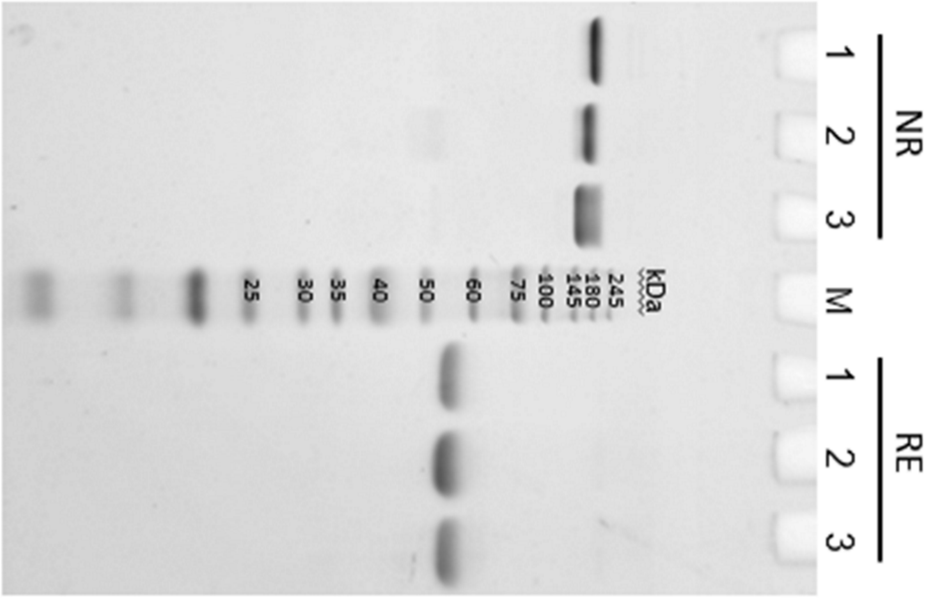


| Lane Number | Format name |
|---|---|
| 1 | IL-5Rα(ben)×CD3ε-scFv(L)/scFv-Fc |
| 2 | IL-5Rα(5R65)×CD3ε-scFv(L)/scFv-Fc |
| 3 | HER2×CD3ε-scFv(L)/scFv-Fc |

【Fig. 12c】

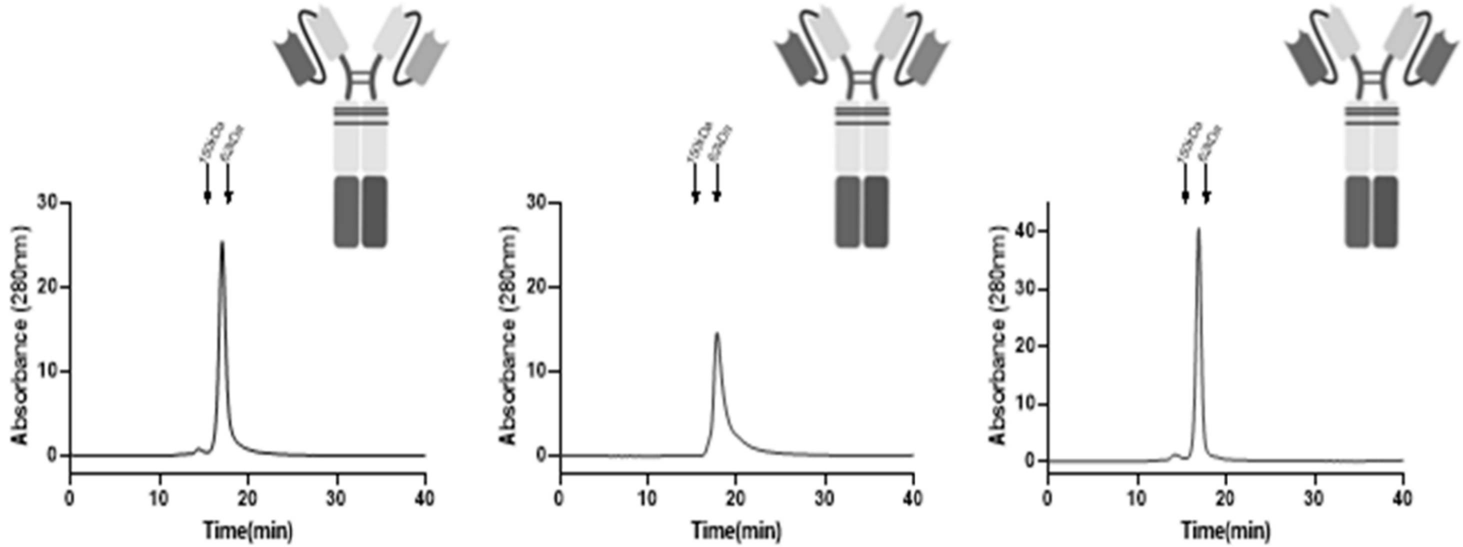


IL-5Rα(ben)×CD3ε-scFv(L)/scFv-Fc

IL-5Rα(5R65)×CD3ε-scFv(L)/scFv-Fc

HER2×CD3ε-scFv(L)/scFv-Fc

| | Clone (scFv(L)/scFv-Fc) | Molecular Weight (kDa) | Naïve(%) | Aggregate(%) | Monomeric(%) |
|---|---|---|---|---|---|
| 1 | IL-5Rα(ben)×CD3ε | 104.8 | 95.9 | 4.1 | - |
| 2 | IL-5Rα(5R65)×CD3ε | 105.3 | 100 | - | - |
| 3 | HER2×CD3ε | 104.4 | 95.2 | 4.8 | - |

【Fig. 13】

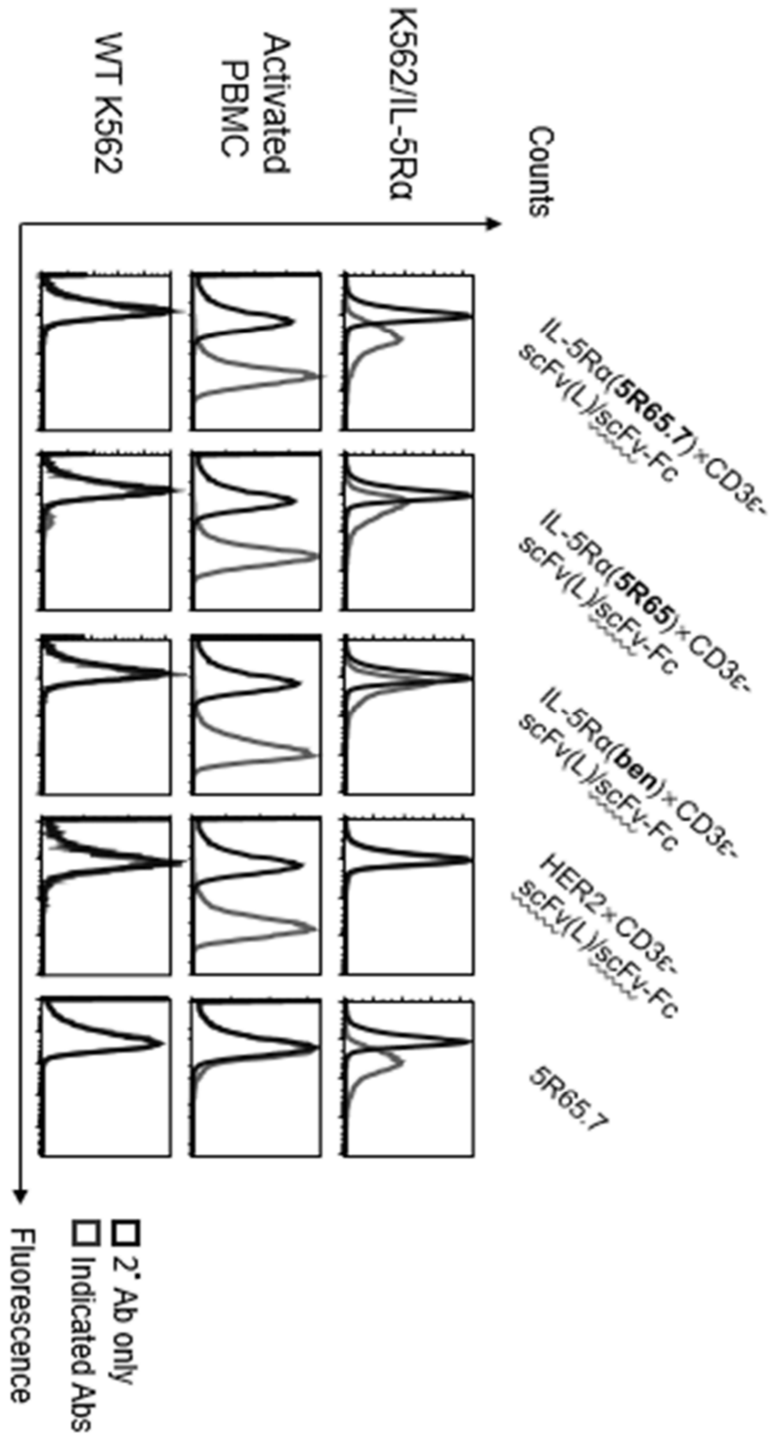
K562/IL-5Rα
Activated PBMC
WT K562
Counts
Fluorescence
IL-5Rα(5R65.7)×CD3ε-scFv(L)/scFv-Fc
IL-5Rα(5R65)×CD3ε-scFv(L)/scFv-Fc
IL-5Rα(ben)×CD3ε-scFv(L)/scFv-Fc
HER2×CD3ε-scFv(L)/scFv-Fc
5R65.7
2° Ab only
Indicated Abs

【Fig. 14a】

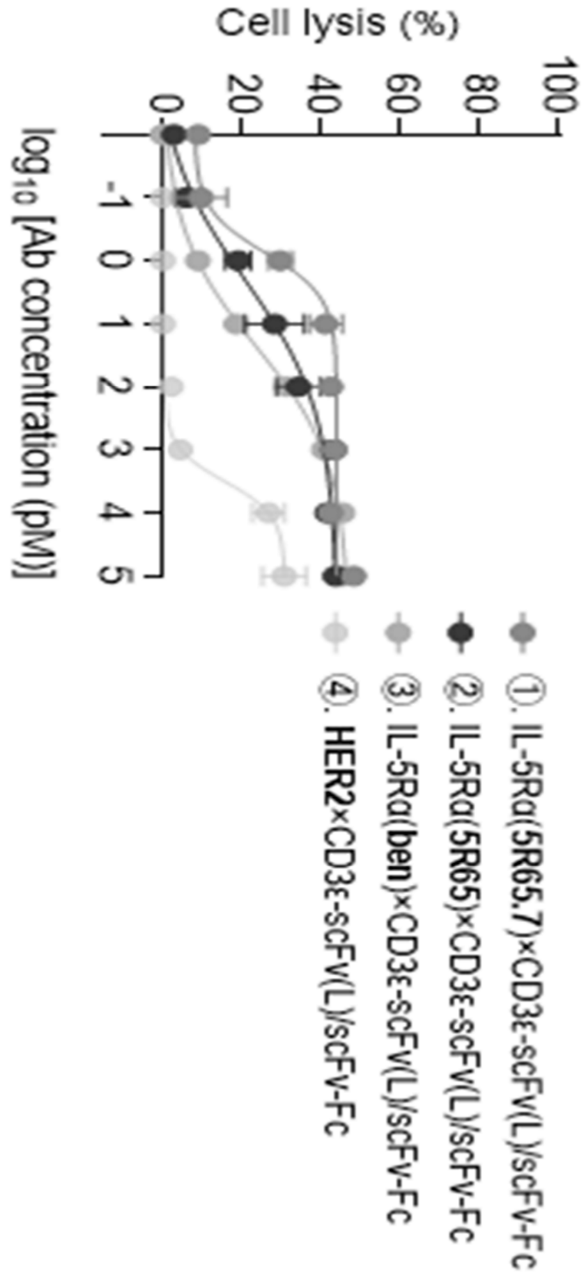
Cell lysis (%)
0
20
40
60
80
100
log10 [Ab concentration (pM)]
-1
0
1
2
3
4
5
①. IL-5Rα(5R65.7)×CD3ε-scFv(L)/scFv-Fc
②. IL-5Rα(5R65)×CD3ε-scFv(L)/scFv-Fc
③. IL-5Rα(ben)×CD3ε-scFv(L)/scFv-Fc
④. HER2×CD3ε-scFv(L)/scFv-Fc

【Fig. 14b】

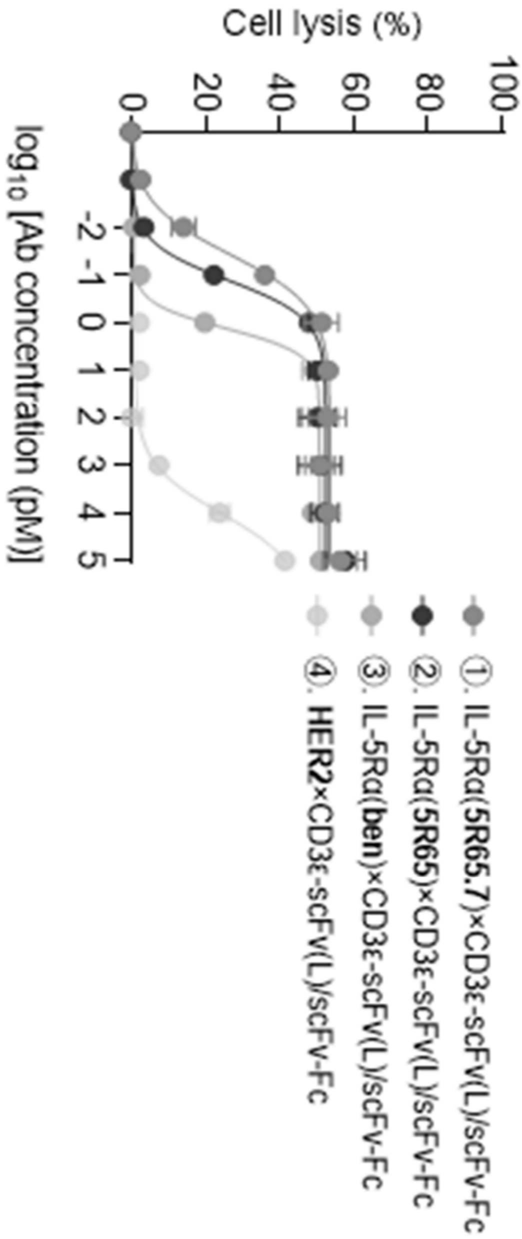
Cell lysis (%)
0 20 40 60 80 100
log10 [Ab concentration (pM)]
-2 -1 0 1 2 3 4 5
①. IL-5Rα(5R65.7)×CD3ε-scFv(L)/scFv-Fc
②. IL-5Rα(5R65)×CD3ε-scFv(L)/scFv-Fc
③. IL-5Rα(ben)×CD3ε-scFv(L)/scFv-Fc
④. HER2×CD3ε-scFv(L)/scFv-Fc

【Fig. 14c】

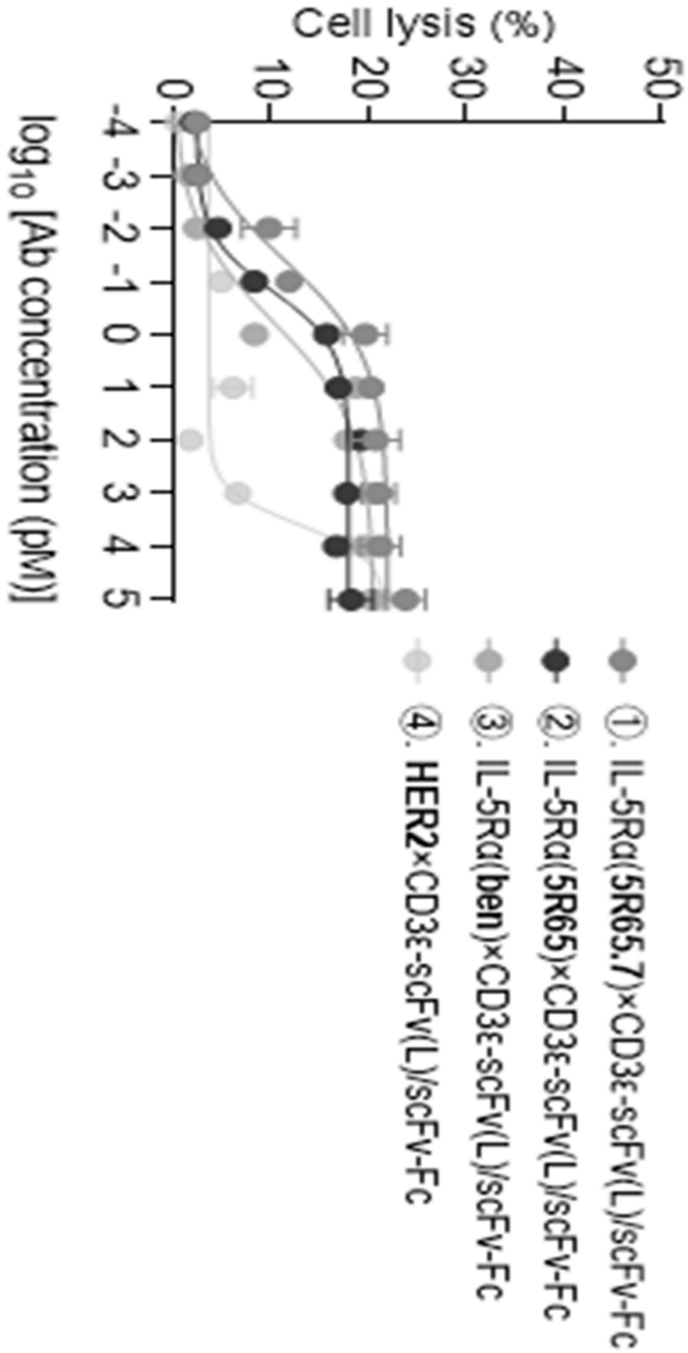
Cell lysis (%)
0
10
20
30
40
50
-4 -3 -2 -1 0 1 2 3 4 5
log10 [Ab concentration (pM)]
①. IL-5Rα(5R65.7)×CD3ε-scFv(L)/scFv-Fc
②. IL-5Rα(5R65)×CD3ε-scFv(L)/scFv-Fc
③. IL-5Rα(ben)×CD3ε-scFv(L)/scFv-Fc
④. HER2×CD3ε-scFv(L)/scFv-Fc

【Fig. 15】

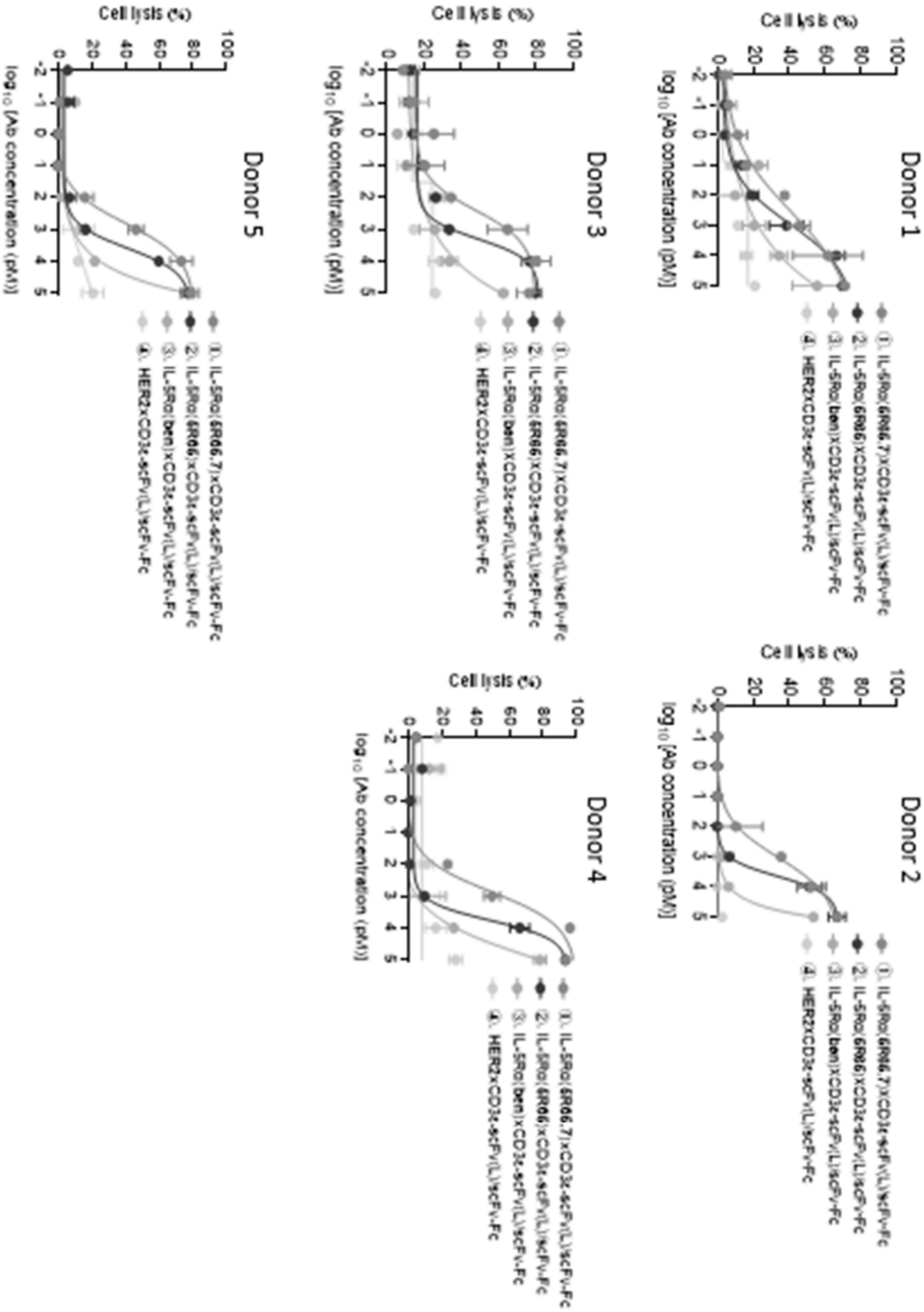
Donor 1
Donor 2
Donor 3
Donor 4
Donor 5
Cell lysis (%)
log10 [Ab concentration (pM)]

【Fig. 16】

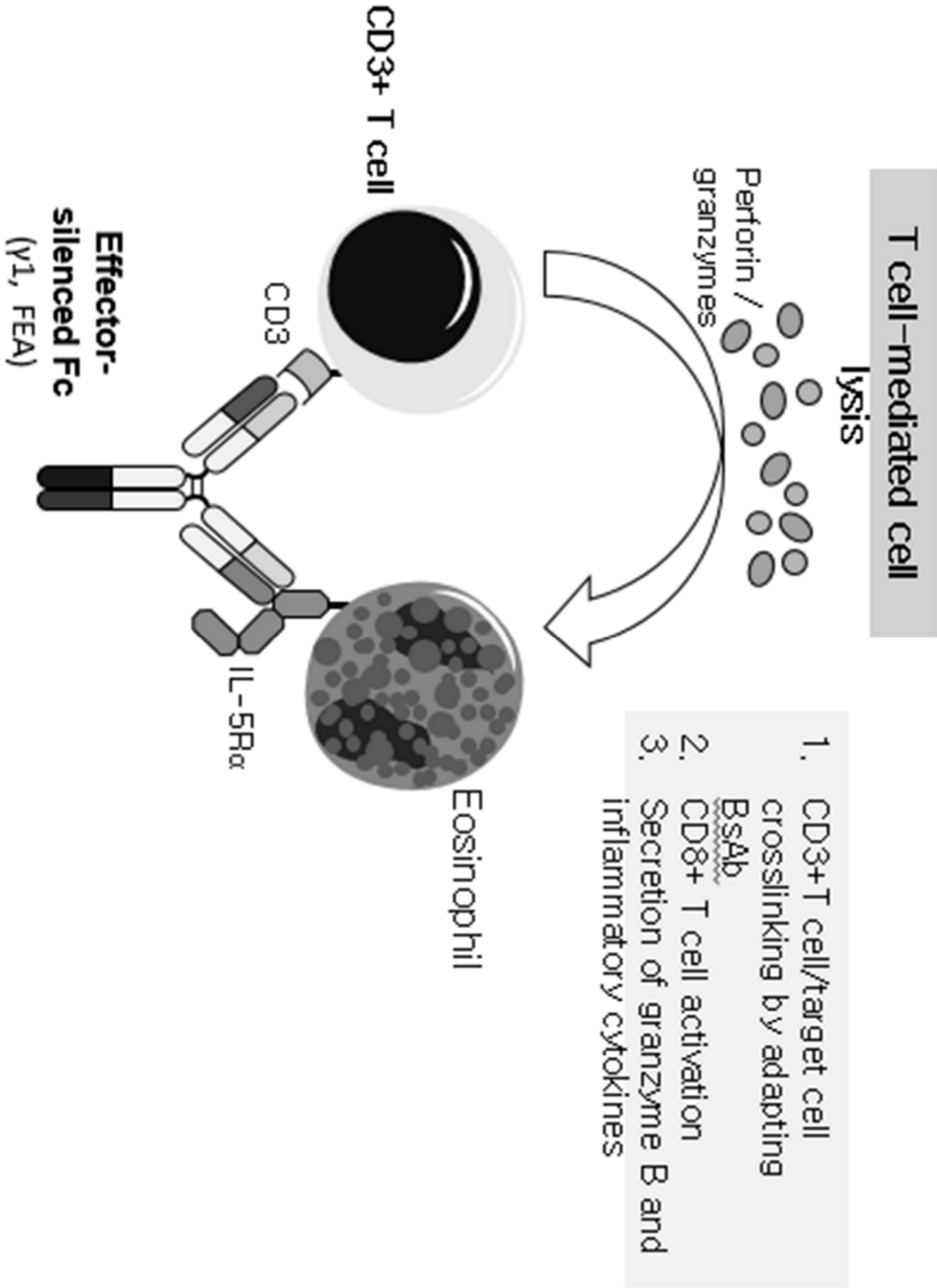
T cell–mediated cell lysis
Perforin / granzymes
CD3+ T cell
CD3
Effector-silenced Fc (γ1, FEA)
IL-5Rα
Eosinophil
1. CD3+T cell/target cell crosslinking by adapting BsAb
2. CD8+ T cell activation
3. Secretion of granzyme B and inflammatory cytokines

**INTERNATIONAL SEARCH REPORT**

International application No.
**PCT/KR2024/005975**

**A. CLASSIFICATION OF SUBJECT MATTER**

**C07K 16/28**(2006.01)i; **A61P 37/00**(2006.01)i; **A61K 39/00**(2006.01)i

According to International Patent Classification (IPC) or to both national classification and IPC

**B. FIELDS SEARCHED**

Minimum documentation searched (classification system followed by classification symbols)

C07K 16/28(2006.01); A61K 39/00(2006.01); C07K 16/30(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 인터루킨-5(interleukin-5, IL-5), CD3, 이중항체(bispecific antibody), 호산구(eosinophil), 염증(inflammation)

**C. DOCUMENTS CONSIDERED TO BE RELEVANT**

| Category* | Citation of document, with indication, where appropriate, of the relevant passages | Relevant to claim No. |
|---|---|---|
| A | KR 10-2022-0035655 A (AJOU UNIVERSITY INDUSTRY-ACADEMIC COOPERATION FOUNDATION) 22 March 2022 (2022-03-22)<br>See entire document. | 1-17 |
| A | WO 2014-167022 A1 (ENGMAB AG) 16 October 2014 (2014-10-16)<br>See entire document. | 1-17 |
| A | CHEN, Sisi et al. Treatment of allergic eosinophilic asthma through engineered IL-5-anchored chimeric antigen receptor T cells. Cell discovery. 2022, vol. 8, article no. 80, inner pp. 1-12 (published online: 16 August 2022).<br>See entire document. | 1-17 |
| A | KR 10-2022-0035656 A (AJOU UNIVERSITY INDUSTRY-ACADEMIC COOPERATION FOUNDATION) 22 March 2022 (2022-03-22)<br>See entire document. | 1-17 |
| A | KR 10-2019-0105112 A (MACROGENICS, INC.) 11 September 2019 (2019-09-11)<br>See entire document. | 1-17 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **19 August 2024** | **20 August 2024** |
| Name and mailing address of the ISA/KR<br>**Korean Intellectual Property Office**<br>**Government Complex-Daejeon Building 4, 189 Cheongsa-ro, Seo-gu, Daejeon 35208**<br>Facsimile No. **+82-42-481-8578** | Authorized officer<br><br>Telephone No. |

Form PCT/ISA/210 (second sheet) (July 2022)

**INTERNATIONAL SEARCH REPORT**

International application No.
**PCT/KR2024/005975**

**C. DOCUMENTS CONSIDERED TO BE RELEVANT**

| Category* | Citation of document, with indication, where appropriate, of the relevant passages | Relevant to claim No. |
|---|---|---|
| PX | KIM, Jun-Ho et al. Engineering bispecific T-cell engagers to deplete eosinophils for the treatment of severe eosinophilic asthma. Clinical immunology. 2023, vol. 255, article no. 109755, inner pp. 1-11 (published online: 09 October 2023). See entire document. | 1-17 |
| PX | KIM, Jun-Ho. Engineering bispecific T-cell engagers to deplete eosinophils for the treatment of severe eosinophilic asthma. 아주대학교 석사학위 논문 (Master's thesis, Ajou University Graduate School). February 2024, pp. 1-54. See entire document. | 1-17 |

Form PCT/ISA/210 (second sheet) (July 2022)

**INTERNATIONAL SEARCH REPORT**

International application No.
**PCT/KR2024/005975**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed.
   b. ☐ furnished subsequent to the international filing date for the purposes of international search (Rule 13*ter*.1(a)),
      ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

**INTERNATIONAL SEARCH REPORT**
**Information on patent family members**

International application No.
**PCT/KR2024/005975**

| Patent document cited in search report | Publication date (day/month/year) | Patent family member(s) | Publication date (day/month/year) |
|---|---|---|---|
| KR 10-2022-0035655 A | 22 March 2022 | EP 4212550 A1 | 19 July 2023 |
| | | US 2023-0357415 A1 | 09 November 2023 |
| | | WO 2022-055299 A1 | 17 March 2022 |
| WO 2014-167022 A1 | 16 October 2014 | EP 2789630 A1 | 15 October 2014 |
| | | EP 2984107 A1 | 17 February 2016 |
| | | EP 2984107 B1 | 19 June 2019 |
| | | US 2016-0297881 A1 | 13 October 2016 |
| | | US 2020-0255521 A1 | 13 August 2020 |
| KR 10-2022-0035656 A | 22 March 2022 | EP 4212551 A1 | 19 July 2023 |
| | | US 2023-0416380 A1 | 28 December 2023 |
| | | WO 2022-055300 A1 | 17 March 2022 |
| KR 10-2019-0105112 A | 11 September 2019 | AU 2012-259161 A1 | 29 November 2012 |
| | | AU 2012-259161 B2 | 06 April 2017 |
| | | AU 2017-204545 A1 | 20 July 2017 |
| | | AU 2017-204545 B2 | 15 November 2018 |
| | | AU 2019-200159 A1 | 31 January 2019 |
| | | AU 2019-200159 B2 | 12 March 2020 |
| | | CA 2836857 A1 | 29 November 2012 |
| | | CA 2836857 C | 03 December 2019 |
| | | CN 103703024 A | 02 April 2014 |
| | | CN 103703024 B | 14 November 2017 |
| | | CN 107722124 A | 23 February 2018 |
| | | CN 107827985 A | 23 March 2018 |
| | | EP 2714733 A2 | 09 April 2014 |
| | | EP 2714733 B1 | 23 January 2019 |
| | | EP 3492494 A1 | 05 June 2019 |
| | | JP 2014-517844 A | 24 July 2014 |
| | | JP 2017-206505 A | 24 November 2017 |
| | | JP 2019-142866 A | 29 August 2019 |
| | | JP 2022-001580 A | 06 January 2022 |
| | | JP 6141831 B2 | 07 June 2017 |
| | | JP 6496349 B2 | 03 April 2019 |
| | | JP 7302990 B2 | 04 July 2023 |
| | | KR 10-2014-0033107 A | 17 March 2014 |
| | | KR 10-2060389 B1 | 31 December 2019 |
| | | US 10150812 B2 | 11 December 2018 |
| | | US 11111299 B2 | 07 September 2021 |
| | | US 2014-0099318 A1 | 10 April 2014 |
| | | US 2017-0137519 A1 | 18 May 2017 |
| | | US 2019-0040135 A1 | 07 February 2019 |
| | | US 2022-0056131 A1 | 24 February 2022 |
| | | US 9587021 B2 | 07 March 2017 |
| | | WO 2012-162067 A2 | 29 November 2012 |
| | | WO 2012-162067 A3 | 31 January 2013 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- KR 1020200117668
- US 9951145 B
- KR 1522954
- KR 101973060

### Non-patent literature cited in the description


- **NAGORSEN, D.** ; **P. A. BAEUERLE**. Immunomodulatory therapy of cancer with T cell-engaging BiTE antibody blinatumomab. *Exp Cell Res*, 2011, vol. 317 (9), 1255-1260
- **DIABODY** ; **HOLLIGER, P. et al.** Specific killing of lymphoma cells by cytotoxic T-cells mediated by a bispecific diabody. *Protein Engineering, Design and Selection*, 1996, vol. 9 (3), 299-305
- **TANDEM DIABODY** ; **KIPRIYANOV, S. M. et al.** Bispecific tandem diabody for tumor therapy with improved antigen binding and pharmacokinetics.. *J Mol Biol*, 1999, vol. 293 (1), 41-56
- **MOORE, P. A. et al.** Application of dual affinity retargeting molecules to achieve optimal redirected T-cell killing of B-cell lymphoma.. *Blood*, 2011, vol. 117 (17), 4542-4551
- **SEIMETZ, D. et al.** Development and approval of the trifunctional antibody catumaxomab (anti-EpCAM×anti-CD3) as a targeted cancer immunotherapy. *Cancer Treatment Reviews*, 2010, vol. 36 (6), 458-467
- **QI, J. et al.** Potent and selective antitumor activity of a T cell-engaging bispecific antibody targeting a membrane-proximal epitope of ROR1.. *Proc Natl Acad Sci U S A*, 2018, vol. 115 (24), E5467-e5476
- **CHOI HJ et al.** *Molecular Cancer Therapeutics*, 2013, vol. 12 (12), 2748-2759